# EUROPEAN PATENT APPLICATION

(11) **EP 3 470 426 A1**
(43) Date of publication of application: **17.04.2019**
(21) Application number: 17195779.8
(22) Date of filing: 10.10.2017
(51) Int. Cl.: C07K 16/18, C07K 16/28

(54) **MULTISPECIFIC ANTIBODY**

(71) Applicant: Numab Therapeutics AG, 8808 Pfäffikon (CH)
(72) Inventor: Urech, David, 8645 Jona (CH); Gunde, Tea, 8005 Zurich (CH); Meyer, Sebastian, 5445 Eggenwil (CH); Brock, Matthias, 8055 Zurich (CH); Hess, Christian, 8045 Zurich (CH); Simonin, Alexandre, 8804 Au ZH (CH); Warmuth, Stefan, 8804 Au ZH (CH)
(74) Representative: Virnekäs, Bernhard

(57) **Abstract**

The present invention relates generally to a multispecific antibody comprising at least one CD137 binding domain and at least one PDL1 binding domain, and pharmaceutical compositions and methods of use thereof, a nucleic acid comprising a nucleotide sequence encoding said multispecific antibody, a vector comprising said nucleic acid, a host cell comprising said nucleic acid or said vector, and a method of producing said multispecific antibody.

## Description

### FIELD OF THE INVENTION

The present invention relates generally to a multispecific antibody comprising at least one CD137 binding domain and at least one PDL1 binding domain, and pharmaceutical compositions and methods of use thereof, a nucleic acid comprising a nucleotide sequence encoding said multispecific antibody, a vector comprising said nucleic acid, a host cell comprising said nucleic acid or said vector, and a method of producing said multispecific antibody.

### BACKGROUND OF THE INVENTION

The tumor necrosis factor receptor superfamily (TNFRSF) is a protein superfamily of receptors characterized by their ability to bind tumor necrosis factors (TNFs) via cysteine-rich pseudorepeats in the extracellular domain (Locksley et al., 2001). At present, 27 TNF family members have been identified. TNFRSF members and their ligands are expressed mostly on immune cells, where they are playing a role of immunomodulators in T-cell-mediated immune responses. TNFRSF members play role in enhancement of dendric cell survival and priming capacity of T cells, optimal generation of effector T cells, optimal antibody responses, and amplification of inflammatory reactions.

CD137 (4-1BB, TNF-receptor superfamily 9, TNFRSF9) is a surface glycoprotein of the TNFR superfamily. It is an inducible costimulatory T cell receptor. CD137 expression is activation-dependent, and encompasses a broad subset of immune cells including activated NK and NKT cells, regulatory T cells, dendritic cells (DC) including follicular DC, stimulated mast cells, differentiating myeloid cells, monocytes, neutrophils, eosinophils (Wang et al, Immunol Rev. 229(1): 192-215 (2009)), and activated B cells (Zhang et al, J Immunol. 184(2):787-795 (2010)). In addition, CD137 expression has also been demonstrated on tumor vasculature (Broil K et al., Am J Clin Pathol. 115(4):543-549 (2001); Seaman et al, Cancer Cell 11(6):539-554 (2007)) and atherosclerotic endothelium (Olofsson et al, Circulation 117(10): 1292 1301 (2008)).

CD137-Ligand (CD137L, 4-1BBL or tnfsf9), a molecule of the TNF family, is an intercellular natural ligand known for CD137 (Alderson, M. R., et al., Eur. J. Immunol. 24:2219-2227 (1994); Pollok K., et al., Eur. J. Immunol. 24:367-374 (1994); Goodwin, R. G., et al., Eur. J. Immunol. 23: 2631-2641 (1993)). The ligand for CD137 forms a homotrimer, and the signaling via CD137 proceeds from ligated molecules at the cell surface, which become cross-linked by trimerized ligand (Won, E. Y., et al., J. Biol. Chem. 285: 9202-9210 (2010)). The higher order clustering of CD137 was thus suggested to be necessary for mediating the signaling. CD137 associates with the adaptors TRAF-2 and TRAF-1 in its cytoplasmic tail, resulting in coimmunoprecipitation, which is enhanced upon CD137 activation in T cells (Saoulli, K., et al., J. Exp. Med. 187: 1849-1862 (1998); Sabbagh, L., et al., J. Immunol. 180: 8093-8101 (2008)). Recruitment of TRAF-1 and TRAF-2 by CD137 results in downstream activation of NFKB and the Mitogen Activated Protein (MAP) Kinase cascade including ERK, JNK, and p38 MAP kinases. NFkB activation leads to upregulation of Bfl-1 and Bcl-XL, pro-survival members of the Bcl-2 family. The pro-apoptotic protein Bim is downregulated in a TRAF-1 and ERK dependent manner (Sabbagh et al., J Immunol. 180(12):8093-8101 (2008)). It has been suggested that the main action of CD137 is to place two or more TRAF-2 molecules in close molecular proximity to each other (Sanchez-Paulete, A. R., et al., Eur. J. Immunology 46(3): 513-522 (2016)). Based on this it was postulated that the major factor driving CD137 signaling is the relative density of TRAF-2-assembled CD137 moieties in micropatches of plasma membrane (Sanchez-Paulete, A. R., et al., Eur. J. Immunology 46(3): 513-522 (2016)). Overall, CD137 signaling is fostered by multimerization, and it was proposed that cross-linking CD137 molecules is the key factor in CD137 co-stimulatory activity.

CD137 co-stimulates T cells to carry out effector functions such as eradication of established tumors, broadening primary CD8⁺T cell responses, and enhancing the memory pool of antigen- specific CD8⁺ T cells, induction of interferon-gamma (IFN-γ) synthesis. The critical role of CD137 stimulation in CD8⁺ T-cell function and survival could be potentially utilized for the treatment of tumors through manipulation of the CD137/CD137L function. In fact, in vivo efficacy studies in mice have demonstrated that treatment with anti-CD 137 antibodies led to tumor regressions in multiple tumor models. For example, agonistic antimouse CD137 antibody were demonstrated to induce an immune response against P815 mastocytoma tumors, and low immunogenic tumor model Ag104 (I. Melero et al., Nat. Med., 3(6):682-5 (1997)). The efficacy of CD137 agonist mAbs in prophylactic and therapeutic settings for both monotherapy and combination therapy and anti-tumor protective T cell memory responses have been reported in several studies (Lynch et al., Immunol Rev. 222:277-286 (2008)). CD137 agonists also inhibit autoimmune reactions in a variety of autoimmunity models (Vinay et al, J Mol Med 84(9):726-736 (2006)).

Two anti-CD 137 antibodies currently in the clinic are urelumab (Bristol-Myers Squibb), a fully humanized IgG4 mAb, and utomilumab (PF-05082566, Pfizer), a fully human IgG2 mAb (Chester C., et al., Cancer Immunol Immunother Oct;65(10):1243-8 (2016)). Although utilization of therapeutic antibodies agonizing CD137 is a very promising treatment strategy, it is coupled to such difficulties as low efficacy of anti-CD 137 agonist antibodies, high toxicities and adverse events.

CD137 agonist antibodies were shown to lead to alterations in immune system and organ function increasing risks of toxicities. High doses of CD137 agonist antibodies in naive and tumor-bearing mice have been reported to induce T-cell infiltration to the liver and elevations of aspartate aminotransferase and alanine aminotransferase consistent with liver inflammation (Niu L, et al. J Immunol 178(7):4194-4213 (2007); Dubrot J, et al., Int J Cancer 128(1):105-118 (2011)). Initial clinical studies into the human therapeutic use of CD137 agonist antibody have also demonstrated elevations of liver enzymes and increased incidence of hepatitis (Sznol M., et al., J Clin Oncol 26(115S):3007 (2008); Ascierto PA, et al., Semin Oncol 37(5):508-516 (2010); Chester C., et al., Cancer Immunol Immunother Oct;65(10):1243-8 (2016)). Potentially fatal hepatitis was observed in a Bristol-Myers Squibb (BMS) phase II anti-CD137 study for previously treated stage III/IV melanoma, National Clinical Trial (NCT) 00612664. This study and several others (NCT00803374, NCT00309023, NCT00461110, NCT00351325) were terminated due to adverse events (Chester C., et al., Cancer Immunol Immunother Oct;65(10):1243-8 (2016)). Such adverse events are most probably due to systemic overstimulation of T-cells.

Further to the above, bivalent CD137 antibodies were shown in vitro to be generally weak in their ability to induce the signaling in the absence of an exogenous clustering. To illustrate, anti-CD 137 antibody utomilumab is only capable to activate CD137 signaling when either cross-linked to anti-human F(ab')2 secondary antibody or immobilized to tissue culture plastic (Fisher at al., Cancer Immunol Immunother 61:1721-1733 (2012)). Studies in rodent agonistic antibodies to CD40 (TNFRSF5), another member of TNFRSF, have suggested that the exogenous clustering can be partially achieved through the interaction with Fcy-receptor (Li F, Ravetch JV, Science 333(6045):1030-10 (2011); White AL, et al., J Immunol 187(4):1754-1763 (2011)). The interaction with Fcy-receptor can however deplete the CD137-expressing cells through effector mechanisms. The current bivalent antibodies targeting CD137 have the limitations that a) they have limited CD137 stimulation capacity in absence of Fcy-receptor interaction, b) such interaction with Fcy-receptor can induce depletion of CD137 expressing cells, which likely affects activity, and c) their activity is not restricted to the target tissue, thereby causing systemic adverse effects

To gain additional cross-linking function and achieve certain levels of TNRSF, in particular CD137, activation, it has been recently suggested to use multivalent and multispecific fusion polypeptides that bind PDL1 and TNRSF members, or folate receptor alpha (FRα) and TNRSF members, wherein the binding to PDL1 or FRα is capable of providing additional crosslinking function (WO2017/123650). Eckelman et al. have demonstrated that bivalent engagement of CD137, as in the case of INBRX-105, a multispecific and multivalent polypeptide having two PDL1 binding domains, two CD137 binding domains and an Fc region, is insufficient to effectively cluster and mediate productive CD 137 signaling in absence of an exogenous clustering event, using an assay isolating the effects of the molecule on a reporter T cell-line. In contrast, engagement for a second cell surface antigen PDL1 in the presence of PDL1-positive cells enables further clustering of CD137 and productive signaling (WO2017/123650).

PDL1 (CD274, B7-H1) is a 40 kDa type I transmembrane protein. PDL1 is a surface glycoprotein ligand for PD1, a key immune checkpoint receptor expressed by activated T and B cells and mediates immunosuppression. PDL1 is implicated in the suppression of immune system responses during chronic infections, pregnancy, tissue allografts, autoimmune diseases, and cancer. PDL1 is found on both antigen-presenting cells and human cancer cells, such as squamous cell carcinoma of the head and neck, melanoma, and brain tumor, thyroid, thymus, esophagus, lung, breast, gastrointestinal tract, colorectum, liver, pancreas, kidney, adrenal cortex, bladder, urothelium, ovary, and skin (Katsuya Y, et al., Lung Cancer.88(2):154-159 (2015); Nakanishi J, et al., Cancer Immunol Immunother. 56(8):1173-1182 (2007); Nomi T, et al., Clin Cancer Res. 13(7):2151-2157 (2007); Fay AP, et al., J Immunother Cancer. 3:3 (2015); Strome SE, et al., Cancer Res. 63(19):6501-6505 (2003); Jacobs JF, et al. Neuro Oncol. 11(4):394-402 (2009); Wilmotte R, et al. Neuroreport. 16(10):1081-1085 (2005)). PDL1 is rarely expressed on normal tissues but inducibly expressed on tumor site (Dong H, et al., Nat Med. 8(8):793-800 (2002); Wang et al., Onco Targets Ther. 9: 5023-5039 (2016)). PDL1 downregulates T cell activation and cytokine secretion by binding to PD-1 (Freeman et al., 2000; Latchman et al, 2001). PD-1, activated by PDL1, potentially provides an immune-tolerant environment for tumor development and growth. PDL1 also negatively regulates T-cell function through interaction with another receptor, B7.1 (B7-1, CD80).

Inhibition of the PDL1/PD-1 interaction allows for potent anti-tumor activity. A number of antibodies that disrupt the PD-1 signaling have entered clinical development. These antibodies belong to the following two main categories: those that target PD-1 (nivolumab, Bristol-Myers Squibb; pembrolizumab, Merck, Whitehouse Station, NJ; pidilizumab, CureTech, Yavne, Israel) and those that target PDL1 (MPDL3280A, Genentech, South San Francisco, CA; MEDI4736, MedImmune/AstraZeneca; BMS-936559, Bristol-Myers Squibb; MSB0010718C, EMD Serono, Rockland, MA) (for review see Postow MA et al., J Clin Oncol. Jun 10;33(17):1974-82 (2015)). Targeting PDL1 versus targeting PD-1 may result in different biologic effects. PD-1 antibodies prevent interaction of PD-1 with both its ligands, PDL1 and PDL2. PDL1 antibodies do not prevent PD-1 from interacting with PDL2, although the effect of this interaction remains unknown. PDL1 antibodies however prevent interaction of PDL1 with not only PD-1, but also B7-1 (Butte MJ, et al., Immunity 27:111-122, (2007)), which is believed to exert negative signals on T cells. Blocking PDL1 has demonstrated promising early data, and currently, four clinical anti-PDLl mAbs are in the testing: atezolizumab and MEDI4736 (both are Fc null variants of human IgG1), MSB001078C (IgG1), and BMS-936559 (IgG4) (Chester C., et al., Cancer Immunol Immunother Oct;65(10):1243-8 (2016)).

The combination of anti-PDLl and anti-CD137 antibodies increased overall survival and enhanced T-cell effector function in the ID-8 ovarian adenocarcinoma model (Duraiswamy J, et al., Cancer Res 73:6900-6912 (2013)). The combination of urelumab (anti-CD137) with nivolumab (anti-PD-1) in both solid tumors and B-cell non-Hodgkin's lymphoma is being tested in a phase I/II trial (NCT02253992), while PF-05082566 (anti-CD137) is being tested in a phase Ib trial with pembrolizumab (anti-PD-1) in patients with solid tumors (NCT02179918) (Chester C., et al., Cancer Immunol Immunother Oct;65(10):1243-8 (2016)).

Recently, the effect of multivalent and multispecific fusion polypeptides that bind PDL1 and CD137 has been evaluated in vitro on T-cell activation and proliferation. Using an autologous in vitro co-culture system implementing immature DC and donor matched T-cells, it has been demonstrated that INBRX-105, a multispecific and multivalent polypeptide having two PDL1 binding domains, two CD137 binding domains and an Fc region, is superior in stimulating interferon-gamma production, when compared to the monospecific PDL1 sd-Ab-Fc fusion protein, the CD137 sdAb-Fc fusion protein, the combination of the two, the anti-PDLl antibody atezolizumab, the anti-CD137 antibody utomilumab (PF-05082566), or the anti-PDL1 antibody prembrolizumab, and combinations thereof, at inducing INFγ or mediating CD8⁺ T-cell proliferation and activation (WO2017/123650).

In spite of numerous treatment options for patients suffering from cancer, there remains a need for effective and safe therapeutic agents and a need for their preferential use in a more targeted manner. Immune-modulating biologics offer promising approaches in treatment of cancers due to their modes of actions, however global immunostimulation and lack of any restriction of this immunomodulation to pathologically relevant cells and sites causes numerous side effects and significant toxicities, which potentially may lead to increased morbidity and mortality of patients. It is therefore an object of the present invention to provide a medicament to improve treatment of a proliferative disease, particularly a cancer. The present invention addresses the need for precision therapeutics for immuno-oncology that target only the disease-co localized T cells for upregulation.

### SUMMARY OF THE INVENTION

It is an object of the present invention to provide a medicament to improve treatment of a proliferative disease, particularly a cancer. The present invention addresses the need for precision therapeutics for immuno-oncology that target only the disease-colocalized T cells for upregulation.

In one aspect, the present invention relates to a multispecific antibody comprising at least one CD137 binding domain and at least one PDL1 binding domain. The present invention further relates to a multispecific antibody comprising at least one CD137 binding domain, at least one PDL1 binding domain, and at least one human serum albumin domain.

In one aspect, the present invention relates to a pharmaceutical composition comprising the multispecific antibody of the invention and a pharmaceutically acceptable carrier.

In a further aspect, the present invention provides the multispecific antibody of the invention or the pharmaceutical composition of the invention for use as a medicament.

In a further aspect, the present invention provides the multispecific antibody of the invention or the pharmaceutical composition of the invention for use in a manufacture of a medicament.

In a further aspect, the present invention provides the multispecific antibody of the invention or the pharmaceutical composition of the invention for use in treatment of cancer in a subject in need thereof.

In one aspect, the present invention provides use of the multispecific antibody of the invention or the pharmaceutical composition of the invention for treating cancer in a subject in need thereof.

In one aspect, the present invention provides use of the multispecific antibody of the invention or the pharmaceutical composition of the invention in the manufacture of a medicament for treatment of a cancer, in a subject in need thereof.

In yet another aspect, the present invention provides a method of treating a cancer in a subject in need thereof comprising administering to the subject a therapeutically effective amount of the multispecific antibody of the invention or the pharmaceutical composition of the invention.

In a further aspect, the present invention provides a nucleic acid comprising a nucleotide sequence encoding the multispecific antibody of the invention. In a further aspect, the present invention provides a vector comprising said nucleic acid. In a further aspect, the present invention provides a host cell comprising said nucleic or said vector.

In yet another aspect, the present invention provides a method of producing the multispecific antibody of the invention or a binding domain thereof or a fragment thereof, the method comprising the step of culturing a host cell expressing a nucleic acid encoding the multispecific antibody of the invention or a binding domain thereof or a fragment thereof.

The aspects, advantageous features and preferred embodiments of the present invention summarized in the following items, respectively alone or in combination, further contribute to solving the object of the invention:
1. A multispecific antibody comprising:
   a) at least one CD137 binding domain (CD137-BD); and
   b) at least one PDL1 binding domain (PDL1-BD).
2. The multispecific antibody of item 1, wherein said antibody is monovalent, bivalent or multivalent for CD137 specificity, preferably monovalent.
3. The multispecific antibody of item 1 or 2, wherein said antibody is monovalent, bivalent or multivalent for PDL1 specificity, preferably monovalent.
4. The multispecific antibody of item 1, wherein said antibody comprises one CD137-BD and one PDL1-BD.
5. The multispecific antibody of item 1, wherein said antibody consists of one CD137-BD and one PDL1-BD.
6. The multispecific antibody of any one of items 1 to 4, wherein said antibody is trispecific.
7. The multispecific antibody of any one of items 1 to 4, wherein said antibody further comprises at least one human serum albumin binding domain, preferably one human serum albumin binding domain.
8. The multispecific antibody of item 7, wherein said antibody comprises one CD137-BD, one PDL1-BD and one HSA-BD, preferably wherein said antibody consists of one CD137-BD, one PDL1-BD and one HSA-BD.
9. The multispecific antibody of any of the preceding items, wherein said antibody does not comprise an immunoglobulin Fc region polypeptide.
10. The multispecific antibody of any of the preceding items, wherein said binding domains are capable of binding to their respective antigen or receptor simultaneously.
11. The multispecific antibody of any of the preceding items, wherein said binding domain, e.g., PDL1-BD, CD137-BD, or HSA-BD, is selected from the group consisting of a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).
12. The multispecific antibody of any of the preceding items, wherein said PDL1-BD and/or said CD137-BD and/or said HSA-BD is/are Fv or scFv.
13. The multispecific antibody of any of the preceding items, wherein said CD137-BD can agonize CD137 upon clustering.
14. The multispecific antibody of item 13, wherein said CD137-BD:
   a) binds to human CD137 with a dissociation constant (KD) of less than 50nM, particularly less than10 nM, particularly less than 5 nM, particularly less than 1nM, particularly less than 500 pM, more particularly less than 100 pM, more particularly less than 50 pM;
   b) binds to human CD137 with a K_{off} rate of 10⁻³ s⁻¹ or less, or 10⁻⁴ s⁻¹ or less, or 10⁻⁵ s⁻¹ or less as measured by SPR;
   c) binds to human CD137 with a Kₒₙ rate of at least 10⁴ M⁻¹s⁻¹ or greater, at least 10⁵ M⁻¹s⁻¹ or greater, at least 10⁶ M⁻¹s⁻¹ or greater, as measured by SPR;
   d) does not cross-compete with urelumab and utolimumab; and/or
   e) is cross-reactive with Macaca fascicularis (Cynomolgus) CD137.
15. The multispecific antibody of item 13 or item 14, wherein said CD137-BD comprises a heavy chain variable region comprising a CDR having the sequence of SEQ ID NO: 1 or SEQ ID NO: 2 for HCDR1, SEQ ID NO: 3 for HCDR2, and SEQ ID NO: 4 for HCDR3, and a light chain variable region comprising a CDR having the sequence SEQ ID NOs: 14 for LCDR1, SEQ ID NOs: 15 for LCDR2, and SEQ ID NO: 16 for LCDR3.
16. The multispecific antibody of item 15, wherein said CD137-BD comprises a heavy chain variable region comprising an amino acid sequence that is at least 90 percent identical to the amino acid sequence selected from the group consisting of SEQ ID NOs: 11, 12 and 13; and a light chain variable region comprising an amino acid sequence that is at least 90 percent identical to the amino acid sequence selected from the group consisting of SEQ ID NOs: 23, 24 and 25.
17. The multispecific antibody of item 16, wherein said CD137-BD comprises: a heavy chain variable region comprising an amino acid sequence selected from any of SEQ ID NOs: 11, 12 and 13; and a light chain variable region comprising an amino acid sequence selected from any of SEQ ID NOs: 23, 24 and 25.
18. The multispecific antibody of item 16, wherein said CD137-BD comprises (a) a VH sequence of SEQ ID NO: 11 and a VL sequence of SEQ ID NO: 23; (b) a VH sequence of SEQ ID NO: 12 and a VL sequence of SEQ ID NO: 24; or (c) a VH sequence of SEQ ID NO: 13 and a VL sequence of SEQ ID NO: 25.
19. The multispecific antibody of item 16, wherein said CD137-BD comprises (a) a HCDR1 comprising the amino acid sequence of SEQ ID NO: 1, a VH sequence of SEQ ID NO: 11 and a VL sequence of SEQ ID NO: 23; (b) a HCDR1 comprising the amino acid sequence of SEQ ID NO: 1, a VH sequence of SEQ ID NO: 12 and a VL sequence of SEQ ID NO: 24; or (c) a HCDR1 comprising the amino acid sequence of SEQ ID NO: 2, a VH sequence of SEQ ID NO: 13 and a VL sequence of SEQ ID NO: 25.
20. The multispecific antibody of item of any of the preceding items, wherein said PDL1-BD is a blocker of PDL1.
21. The multispecific antibody of item 20, wherein said PDL1-BD:
   a) binds to human PDL1 with a dissociation constant (KD) of less than 50nM, particularly less than 10nV, particularly less than 5nM, particularly less than 1nM, particularly less than 500pM, more particularly less than 100pM, preferably less than 10pM, more preferably 5pM;
   b) binds to human PDL1 with a K_{off} rate of 10⁻³ s⁻¹ or less, or 10⁻⁴ s⁻¹ or less, or 10⁻⁵ s⁻¹ or less as measured by SPR;
   c) binds to human PDL1 with a Kₒₙ rate of at least 10³ M⁻¹s⁻¹ or greater, at least 10⁴ M⁻¹s⁻¹ or greater, at least 10⁵ M⁻¹s⁻¹ or greater, at least 10⁶ M⁻¹s⁻¹ or greater as measured by SPR;
   d) is cross-reactive with Macaca fascicularis (Cynomolgus) PDL1; and/or
   e) is non-cross reactive to Mus musculus PDL1.
22. The multispecific antibody of item 20 or item 21, wherein said PDL1-BD comprises:
   (a) a HCDR1 comprising the amino acid sequence of SEQ ID NO: 52 or SEQ ID NO: 53; (b) a HCDR2 comprising the amino acid sequence of SEQ ID NO: 54; (c) a HCDR3 comprising the amino acid sequence of SEQ ID NO: 55; (d) a LCDR1 comprising the amino acid sequence of SEQ ID NOs: 64; (e) a LCDR2 comprising the amino acid sequence of SEQ ID NOs: 65; and (f) a LCDR3 comprising the amino acid sequence of SEQ ID NO: 66.
23. The multispecific antibody of item 20 or item 22, wherein said PDL1-BD comprises:
   (a) a HCDR1 comprising the amino acid sequence of SEQ ID NO: 77 or SEQ ID NO: 78; (b) a HCDR2 comprising the amino acid sequence of SEQ ID NO: 79; (c) a HCDR3 comprising the amino acid sequence of SEQ ID NO: 80; (d) a LCDR1 comprising the amino acid sequence of SEQ ID NOs: 89; (e) a LCDR2 comprising the amino acid sequence of SEQ ID NOs: 90; and (f) a LCDR3 comprising the amino acid sequence of SEQ ID NO: 91.
24. The multispecific antibody of any one of items 20 to 23, wherein said PDL1-BD comprises: a heavy chain variable region comprising an amino acid sequence that is at least 90 percent identical to the amino acid sequence selected from the group consisting of SEQ ID NOs: 62, 63, 87 and 88; and a light chain variable region comprising an amino acid sequence that is at least 90 percent identical to the amino acid sequence selected from the group consisting of SEQ ID NOs: 73, 98 and 99.
25. The multispecific antibody of any one of items 20 to 23, wherein said PDL1-BD comprises: a heavy chain variable region comprising an amino acid sequence selected from any of SEQ ID NOs: 62, 63, 87 and 88; and a light chain variable region comprising an amino acid sequence selected from any of SEQ ID NOs: 73, 98 and 99.
26. The multispecific antibody of any one of items 20 to 23, wherein said PDL1-BD comprises: (a) a VH sequence of SEQ ID NO: 62 and a VL sequence of SEQ ID NO: 73; (b) a VH sequence of SEQ ID NO: 63 and a VL sequence of SEQ ID NO: 73; (c) a VH sequence of SEQ ID NO: 87 and a VL sequence of SEQ ID NO: 98; or (d) a VH sequence of SEQ ID NO: 88 and a VL sequence of SEQ ID NO: 99.
27. The multispecific antibody of item 22, wherein said PDL1-BD comprises: (a) a HCDR1 comprising the amino acid sequence of SEQ ID NO: 52, a VH sequence of SEQ ID NO: 62 and a VL sequence of SEQ ID NO: 73; or (b) a HCDR1 comprising the amino acid sequence of SEQ ID NO: 53, a VH sequence of SEQ ID NO: 63 and a VL sequence of SEQ ID NO: 73.
28. The multispecific antibody of item 23, wherein said PDL1-BD comprises: (a) a HCDR1 comprising the amino acid sequence of SEQ ID NO: 77, a VH sequence of SEQ ID NO: 87 and a VL sequence of SEQ ID NO: 98; or (b) a HCDR1 comprising the amino acid sequence of SEQ ID NO: 78, a VH sequence of SEQ ID NO: 88 and a VL sequence of SEQ ID NO: 99.
29. The multispecific antibody of any of the preceding items, wherein the affinity of said CD137-BD(s) relative to the affinity of said PDL1-BD(s) is at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times weaker (lower) and/or (ii) said CD137-BD(s) has the avidity of at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times weaker (lower) relative to the avidity of said PDL1-BD(s).
30. The multispecific antibody of claim 29, wherein said CD137-BD binds to human CD137 with a dissociation constant (KD) between 10nM and 10pM, preferably between 10nM and 0.1nM, more preferably between 5nM and 1nM.
31. The multispecific antibody of any one of items 7 to 30, wherein said HSA-BD comprises: (a) a heavy chain variable region CDR1 comprising an amino acid sequence selected from any one of SEQ ID NOs: 103 and 125; (b) a heavy chain variable region CDR2 comprising an amino acid sequence selected from any of SEQ ID NOs: 104 and 126; (c) a heavy chain variable region CDR3 comprising an amino acid sequence selected from any of SEQ ID NOs: 105 and 127; (d) a light chain variable region CDR1 comprising an amino acid sequence selected from any of SEQ ID NOs: 113 and 135; (e) a light chain variable region CDR2 comprising an amino acid sequence selected from any of SEQ ID NOs: 114 and 136; and (f) a light chain variable region CDR3 comprising an amino acid sequence selected from any of SEQ ID NOs: 115 and 137.
32. The multispecific antibody of item 31, wherein said HSA-BD comprises: a heavy chain variable region comprising an amino acid sequence that is at least 90 percent identical to the amino acid sequence selected from the group consisting of SEQ ID NOs: 112, and 134; and a light chain variable region comprising an amino acid sequence that is at least 90 percent identical to the amino acid sequence selected from the group consisting of SEQ ID NOs: 122 and 144.
33. The multispecific antibody of item 31, wherein said HSA-BD comprises: a heavy chain variable region comprising an amino acid sequence selected from any of SEQ ID NOs: 112, and 134; and a light chain variable region comprising an amino acid sequence selected from any of SEQ ID NOs: 122 and 144.
34. The multispecific antibody of item 31, wherein said HSA-BD comprises: (a) a VH sequence of SEQ ID NO: 112 and a VL sequence of SEQ ID NO: 122; or (b) a VH sequence of SEQ ID NO: 134 and a VL sequence of SEQ ID NO: 144.
35. The multispecific antibody of item 31, wherein said HSA-BD comprises: (a) HCDR1, HCDR2, and HCDR3 sequences of SEQ ID NOs: 103, 104, and 195, respectively, and LCDR1, LCDR2, and LCDR3 sequences of SEQ ID NOs: 113, 114, and 115, respectively, and a heavy chain variable region comprising an amino acid sequence that is at least 90 percent identical to the amino acid sequence SEQ ID NO: 112, and a light chain variable region comprising an amino acid sequence that is at least 90 percent identical to the amino acid sequence SEQ ID NO: 122; or (b) HCDR1, HCDR2, and HCDR3 sequences of SEQ ID NOs: 125, 126, and 127, respectively, and LCDR1, LCDR2, and LCDR3 sequences of SEQ ID NOs: 135, 136, and 137, respectively, and a heavy chain variable region comprising an amino acid sequence that is at least 90 percent identical to the amino acid sequence SEQ ID NO: 134; and a light chain variable region comprising an amino acid sequence that is at least 90 percent identical to the amino acid sequence SEQ ID NO: 144.
36. The multispecific antibody of item 1, wherein said antibody is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab,, Fab-Fv2, Morrison (IgG CH₃-scFv fusion (Morrison L) or IgG CL-scFv fusion (Morrison H)), triabody (scDb-scFv), bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH and DuoBodies.
37. The multispecific antibody of item 1, wherein said antibody is a scDb comprising an amino acid sequence selected from any of SEQ ID NOs: 156, 157, 158, 159, 160, 161, and 162.
38. The multispecific antibody of item 1, wherein said antibody is a scTriabody comprising an amino acid sequence selected from any of SEQ ID NOs: 163, 164, 165, and 166.
39. A pharmaceutical composition comprising the multispecific antibody of anyone of the preceding items and a pharmaceutically acceptable carrier.
40. The multispecific antibody of anyone of items 1 to 38 or the pharmaceutical composition of item 39 for use as a medicament.
41. The multispecific antibody of anyone of items 1 to 38 or the pharmaceutical composition of item 39 for use in a manufacture of a medicament.
42. The multispecific antibody of anyone of items 1 to 38 or the pharmaceutical composition of item 39 for use in treatment of cancer in a subject in need thereof.
43. Use of the multispecific antibody of anyone of items 1 to 38 or the pharmaceutical composition of item 39 for treating cancer in a subject in need thereof.
44. Use of the multispecific antibody of anyone of items 1 to 38 or the pharmaceutical composition of item 39 in the manufacture of a medicament for treatment of a cancer, in a subject in need thereof.
45. A method of treating a cancer in a subject in need thereof comprising administering to the subject a therapeutically effective amount of the multispecific antibody of items 1 to 38 or the pharmaceutical composition of item 39.
46. The multispecific antibody of anyone of items 40 to 41 or the use of anyone of items 43 to 44 or the method of item 45, wherein said cancer is PDL1-positive cancer, preferably said wherein cancer expresses high levels of PDL1 in comparison to a healthy tissue.
47. A nucleic acid comprising a nucleotide sequence encoding the multispecific antibody according to anyone of items 1 to 38 or a binding domain thereof or a fragment thereof.
48. A vector comprising the nucleic acid of item 47.
49. A host cell comprising the nucleic acid of item 47 or the vector of item 48.
50. A method of producing the multispecific antibody according to anyone of items 1 to 38 or a binding domain thereof or a fragment thereof, the method comprising the step of culturing a host cell expressing a nucleic acid encoding the multispecific antibody according to items 1 to 38 or a binding domain thereof or a fragment thereof.

### BRIEF DESCRIPTION OF THE DRAWINGS

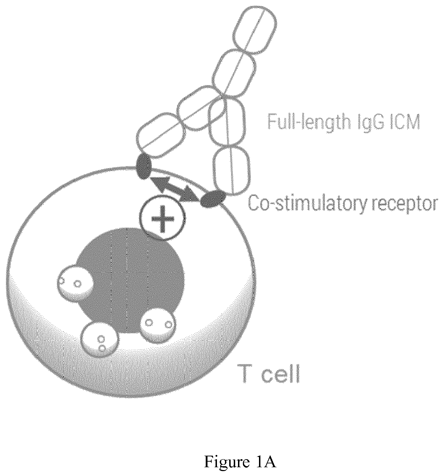

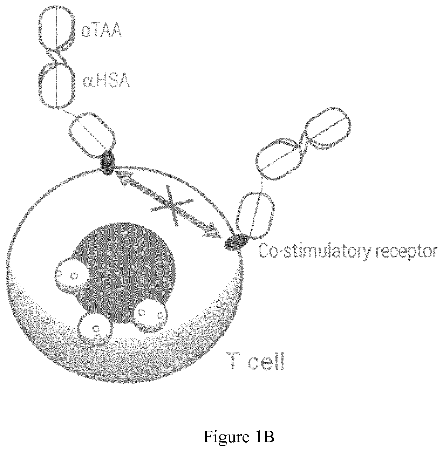

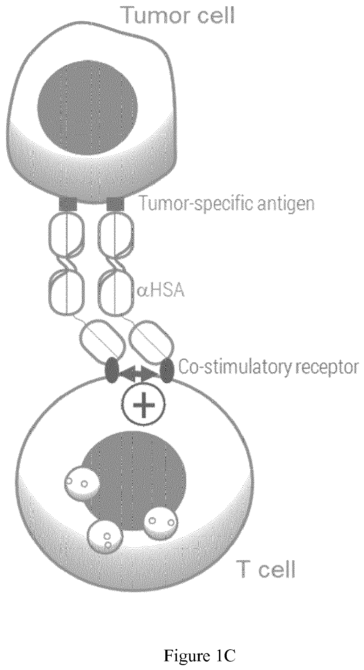

**FIG. 1** Cross-linking of T cell co-stimulatory receptors by, for example, a full-length bivalent IgG supports global stimulation of T cells, resulting in dose-limiting toxicities (Figure 1A). The stable bi-/trispecific monovalent molecules of the present invention cannot cross-link (or, by extension, agonize) co-stimulatory receptors on T cells in the absence of the cell-type being targeted for depletion (Figure 1B). The stable bi-/trispecific monovalent molecules of the present invention cross-link (or, by extension, agonize) co-stimulatory receptors on T cells in the presence of the cell-type being targeted for depletion (Figure 1C).

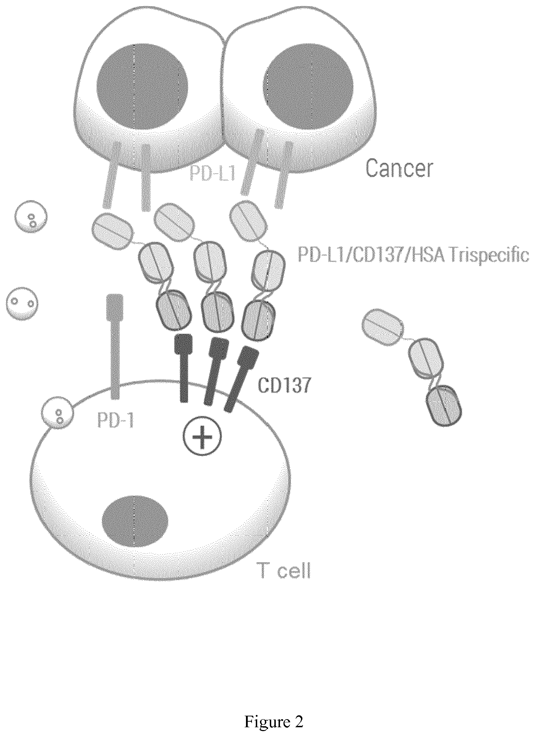

**FIG. 2** Concomitant binding to PDL1 and CD137 triggers selective activation of tumor-reactive T cells and simultaneously blocks PD-1 signaling.

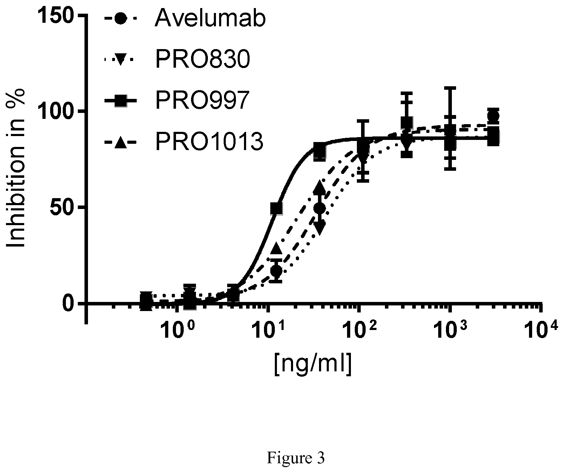

**FIG. 3** Effect of CDR set and framework selection on neutralization of the PDL1/PD-1 interaction in the NFAT-Luciferase reporter gene assay. % inhibition proportional to the luminescence signal obtained in the assay is represented in function of the molecules concentrations in ng/ml. Avelumab was used as reference.

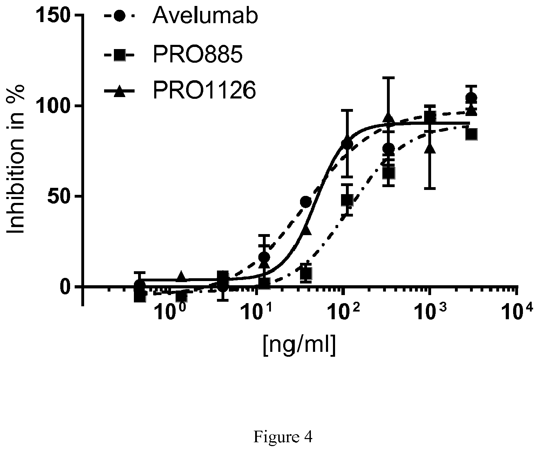

**FIG. 4** Effect of domain optimization on neutralization potency of the PDL1/PD-1 interaction in the NFAT-Luciferase reporter gene assay. % inhibition proportional to the luminescence signal obtained in the assay is represented in function of the scFvs concentrations in ng/ml. Avelumab was used as reference.

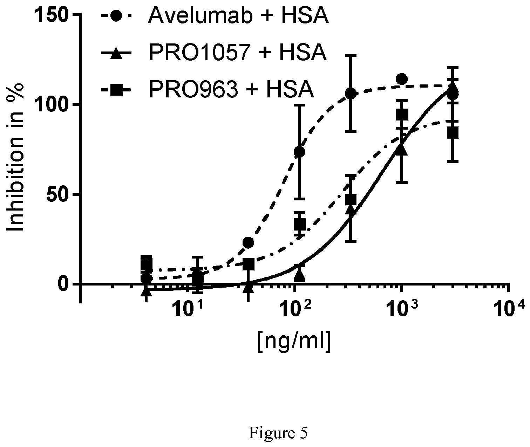

**FIG. 5** Neutralization potency of the PDL1/PD-1 interaction in the reporter gene assay by triabodies in presence of recombinant human serum albumin. % inhibition proportional to the luminescence signal obtained in the assay is represented in function of the scFvs concentrations in ng/ml. Avelumab was used as reference.

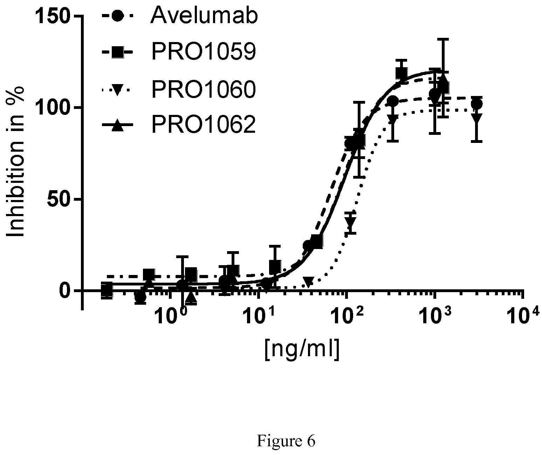

**FIG. 6** Potency of bivalent molecule and influence of LC or HC scFv fusion in Morrison formats on neutralization potency of the PDL1/PD-1 interaction in the NFAT-Luciferase reporter gene assay. % inhibition proportional to the luminescence signal obtained in the assay is represented in function of the molecules concentrations in ng/ml. Avelumab was used as reference.

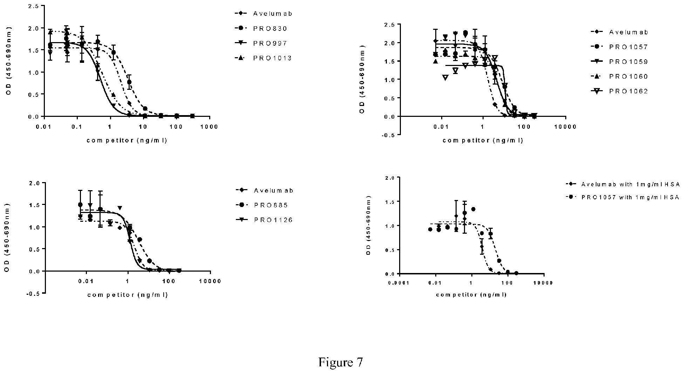

**FIG. 7** PD-1/PD-L1 competition ELISA. All molecules potently blocked the interaction between PD1 and PD-L1, with similar or smaller IC50 values than the reference IgG Avelumab.

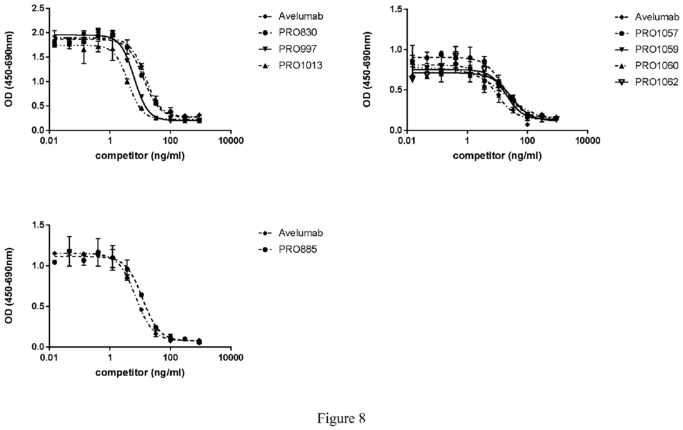

**FIG. 8** B7.1/PD-L1 competition ELISA. Similarly to Avelumab, all molecules potently blocked the interaction between B7.1 and PD-L1.

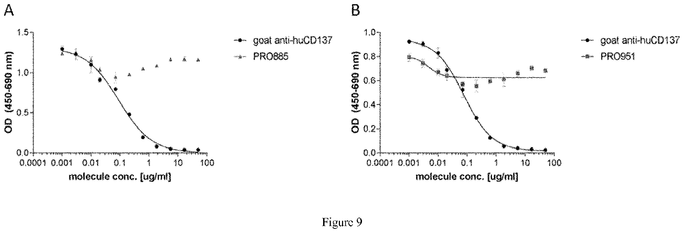

**FIG. 9** No inhibition of CD137 binding to CD137L in competition ELISA. The absorbance measured in the competitive ELISA assessing the binding of CD137L to CD137 are represented in function of increasing concentrations of PRO885 (A) or PRO951 (B) respectively. The inhibitory antibody goat anti-human CD137 served as a reference.

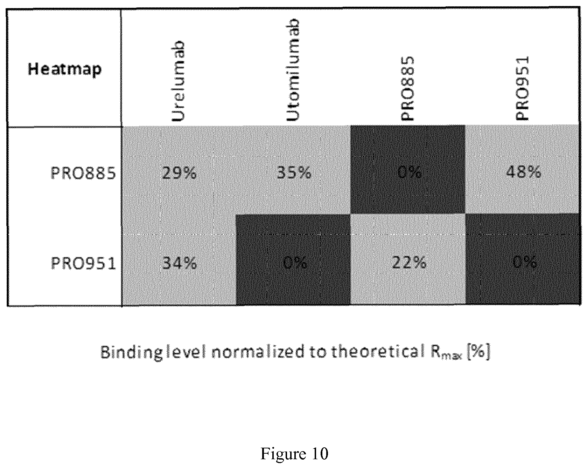

**FIG. 10** Heatmap of epitope binning results of PRO885 and PRO951 and Urelumab and Utomilumab. Binding level normalized to theoretical Rmax in percent (%) of analyte molecules (column) to immobilized molecules (row). No binding (dark grey) means same epitope, bright grey means the secondary molecule (analyte) can bind and has another epitope than the immobilized molecule.

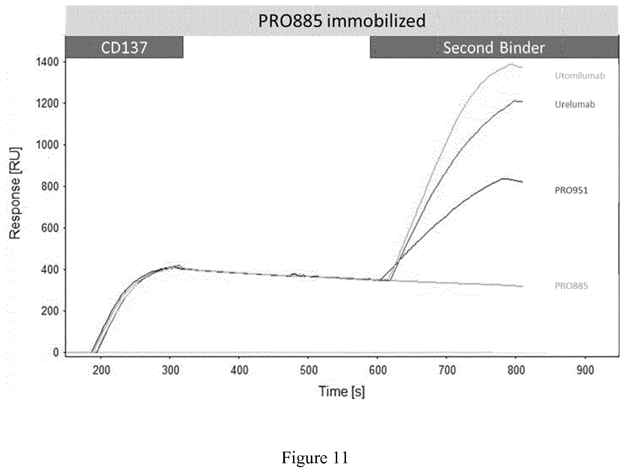

**FIG. 11** Epitope binning sensorgram of PRO885. PRO885 was immobilized on sensor chip and CD137 was captured by PRO885 in a first step (left hand side) followed by injections of the 4 different antibodies (right hand side). PRO951 as well as competitors were able to bind to captured CD137 whereas an injection of PRO885 did not show any binding.

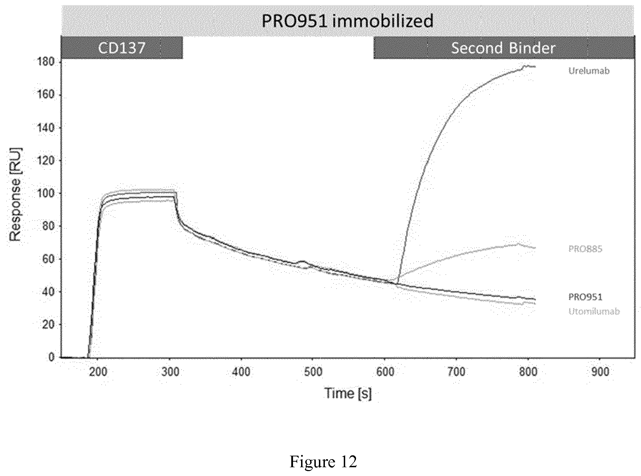

**FIG. 12** Epitope binning sensorgram of PRO951. PRO951 was immobilized on sensor chip and CD137 was captured by PRO951 in a first step (left hand side) followed by injections of the 4 different antibodies (right hand side). PRO885 as well as Urelumab was able to bind to captured CD137 whereas an injection of Utomilumab and PRO951 did not show further binding.

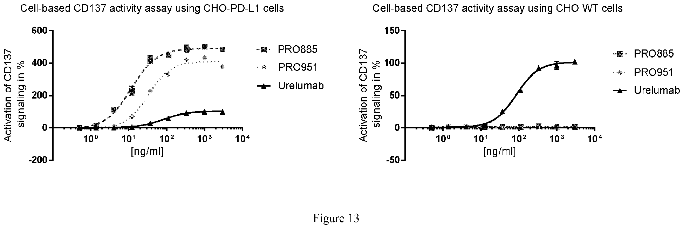

**FIG. 13** CD137 activation by PRO885 and PRO951 as assessed in the NFkB-Luciferase reporter gene assay. In the presence of PD-L1 expressing cells, PRO885 and PRO951 activated CD137 signaling in Jurkat cells whereas no activation was observed when CHO WT cells were tested. Urelumab activated CD137 signaling independently of PD-L1 expression. Luminescence was read 6h after addition of Jurkat reporter cells and data were fitted using sigmoidal 4PL fit (GraphPad Prism).

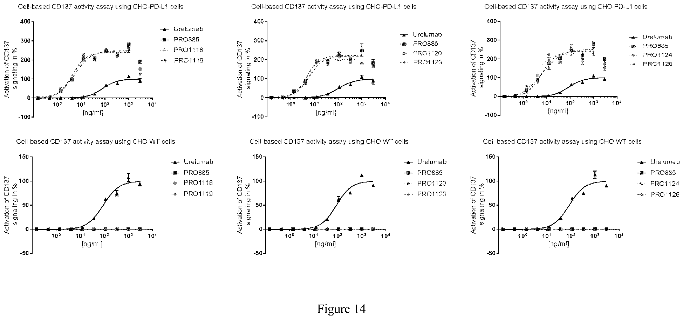

**FIG. 14** CD137 activation in the NFkB-Luciferase reporter gene assay by scDb with different affinities to PDL1 and CD137. In the presence of PD-L1 expressing CHO cells, all scDb activated CD137 signaling in Jurkat cells whereas no activation was observed when CHO WT cells were tested. Urelumab activated CD137 signaling independently of PD-L1 expression. Luminescence was read 6h after addition of Jurkat reporter cells and data were fitted using sigmoidal 4PL fit (GraphPad Prism).

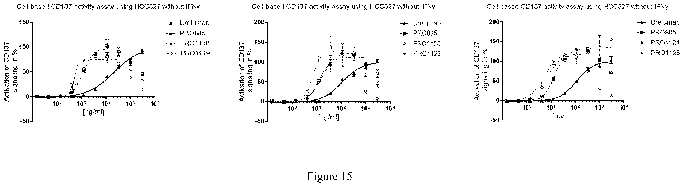

**FIG. 15** CD137 activation in the NFkB-Luciferase reporter gene assay by scDb with different affinities to PDL1 and CD137. In the presence of PD-L1 expressing HCC827 cells, all scDb activated CD137 signaling in Jurkat cells. Urelumab served as reference molecule to assess the relative activation of CD137 signaling. Potency increased slightly with increasing affinity to CD137 and PDL1. A signal decrease at high concentrations (bell-shaped curve) was more pronounced with increasing affinity to CD137, while increased affinity to PDL1 did not contribute to this effect. Luminescence was read 6h after addition of Jurkat reporter cells and data were fitted using sigmoidal 4PL fit (GraphPad Prism).

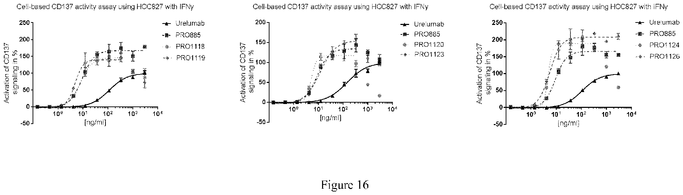

**FIG. 16** CD137 activation in the NFkB-Luciferase reporter gene assay by scDb with different affinities to PDL1 and CD137. In the presence of PD-L1 expressing HCC827 cells stimulated with IFNy for 24h at 10 ng/ml, STR grafted scDb activated CD137 signaling in Jurkat cells. Urelumab served as reference molecule to assess the relative activation of CD137 signaling. Potency increased slightly with increasing affinity to CD137 and PDL1. A signal decrease at high concentrations (bell-shaped curve) was more pronounced with increasing affinity to CD137, while increased affinity to PDL1 did not contribute to this effect. Luminescence was read 6h after addition of Jurkat reporter cells and data were fitted using sigmoidal 4PL fit (GraphPad Prism).

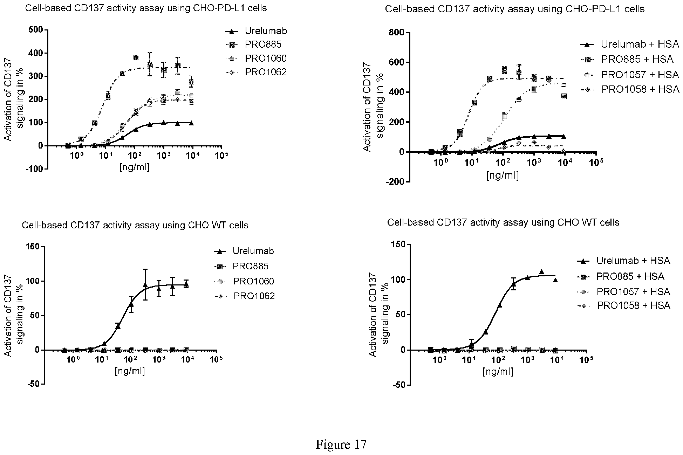

**FIG. 17** CD137 activation by molecules with prolonged serum half-life in the NFkB-Luciferase reporter gene assay after 6h. In the presence of PD-L1 expressing CHO cells, long half-life molecules activated CD137 signaling in Jurkat cells whereas no activation was observed when CHO WT cells were tested. Urelumab activated CD137 signaling independently of PD-L1 expression. Interestingly, despite similar affinities to both targets, PRO1057 showed a much higher maximal signal than PRO1058. And further, the monovalent scDb-scFv PRO1057 showed stronger activation than the respective bivalent Morrison format PRO 1060. Luminescence was read 6h after addition of Jurkat reporter cells and data were fitted using sigmoidal 4PL fit (GraphPad Prism).

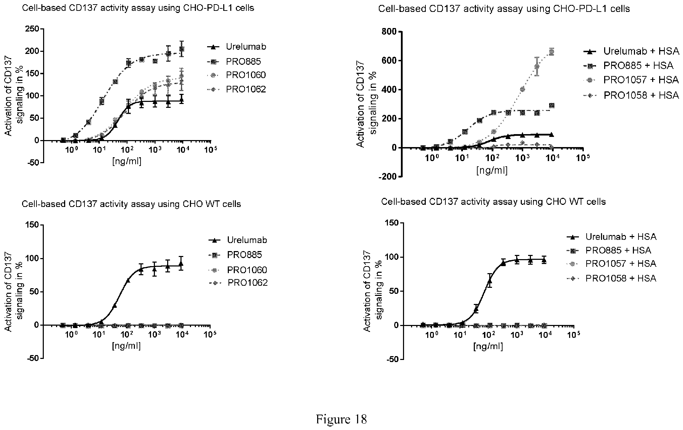

**FIG. 18** CD137 activation by molecules with prolonged serum half-life in the NFkB-Luciferase reporter gene assay after 24h. In the presence of PD-L1 expressing CHO cells, long half-life molecules activated CD137 signaling in Jurkat cells whereas no activation was observed when CHO WT cells were tested. Urelumab activated CD137 signaling independently of PD-L1 expression. Interestingly, despite similar affinities to both targets, PRO1057 showed a much higher maximal signal than PRO1058, which after 24 hours exceeded even the activity of the scDb Pro885. And further, the monovalent scDb-scFv PRO1057 showed much stronger activation than the respective bivalent Morrison format PRO1060. Luminscence was read 24h after addition of Jurkat reporter cells and data were fitted using sigmoidal 4PL fit (GraphPad Prism).

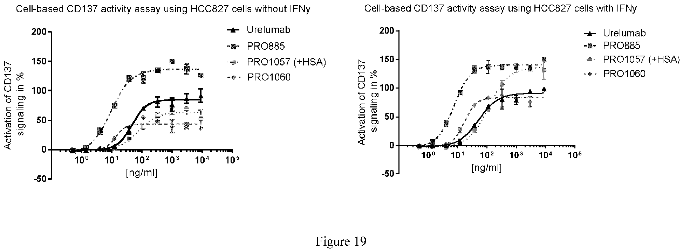

**FIG. 19** CD137 activation by molecules with prolonged half-life, after 24h. In the presence of HCC827 cells either unstimulated or stimulated with IFNy at 10 ng/ml for 24h, long half-life molecules activated CD137 signaling in Jurkat cells. Urelumab served as reference molecule to assess the relative activation of CD137 signaling. The monovalent scDb-scFv PRO1057 showed higher maximal activation than the respective bivalent Morrison format PRO1060. Luminescence was read 24h after addition of Jurkat reporter cells and data were fitted using sigmoidal 4PL fit (GraphPad Prism).

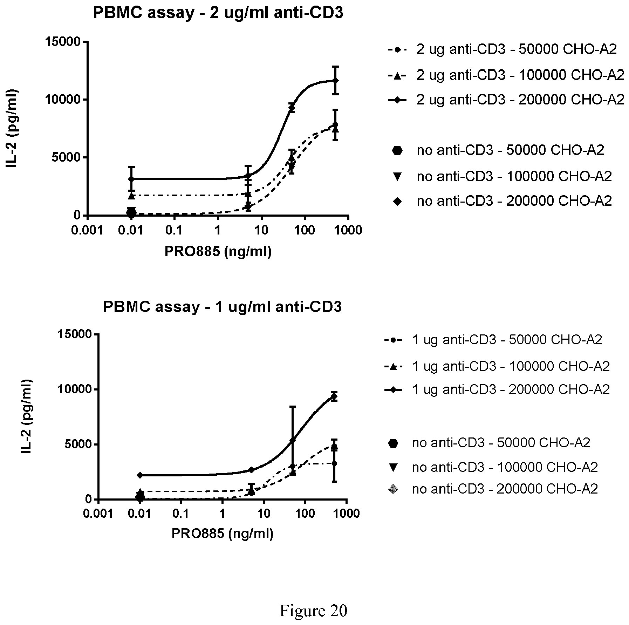

**FIG. 20** Ex vivo T cell activation. The costimulatory engagement of PD-L1 and CD137 by PRO885 is shown, leading to IL-2 production clearly above background IL-2 levels. CHO-A2 cells are transgenic CHO cells expressing PD-L1.

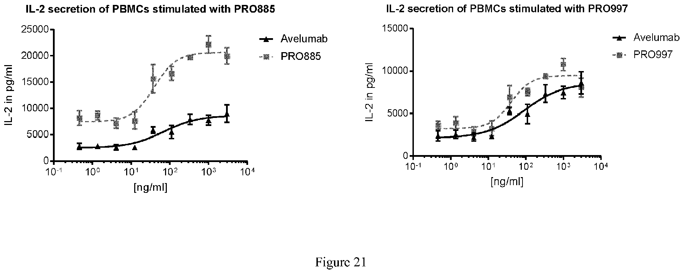

**FIG. 21** Ex vivo T cell activation assay. PBMC were stimulated with 10 ng/ml SEA and treated with serial dilutions of the scFv PRO997 or the scDb PRO885 for 96h. Activation of T-cells was assessed by quantification of IL-2 in harvested supernatants by ELISA. Treatment with PRO885 and PRO997 resulted in pronounced IL-2 secretion. PRO997 showed higher potency than Avelumab. PRO885 showed much increased effect size when compared to Avelumab. Data were fitted using sigmoidal 4PL fit (GraphPad Prism).

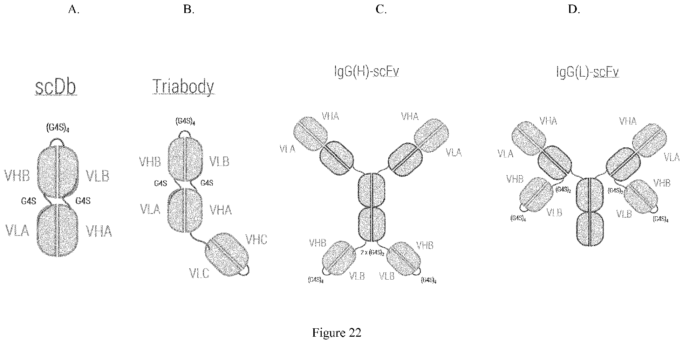

**FIG. 22** Schematic representation of the exemplary formats of the multispecific antibodies of the disclosure: single chain diabodies (scDb) (A), Single chain Triabodies (scTb) (B), IgG-scFv molecules (C and D).

### DETAILED DESCRIPTION OF THE INVENTION

Even though utilization of therapeutic antibodies agonizing CD137 is a very promising treatment strategy, it is coupled to such difficulties as low efficacy of anti-CD137 agonist antibodies, and their high toxicities and adverse events. Cross-linking of T cell co-stimulatory receptors by, for example, a full-length bivalent IgG, as in the case of urelumab, supports global stimulation of T cells, resulting in dose-limiting toxicities (Figure 1A). There is thus a need in the medical field for novel anti-CD 137 agonist antibodies, which are capable of potently inducing CD137 signaling without systemic overstimulation of T-cells, and thus which have lower rate of dose-limiting toxicities and adverse events than the currently available antibodies.

The present invention provides a multispecific antibody comprising: (a) at least one CD137 binding domain (CD137-BD), and (b) at least one PDL1 binding domain (PDL1-BD). The multispecific antibody of the present disclosure are capable of agonizing CD137 signaling in a targeted manner, e.g. at a site of interest, namely in PDL1-positive tumor microenviroment. The multispecific antibody of the present invention is capable of mediating, e.g. agonizing, potent CD137 signaling without any need for as cross-linkage by anti-human F(ab')2 secondary antibody or immobilization to tissue culture plastic as in the case of PF-05082566 (Fisher at al., Cancer Immunol Immunother 61:1721-1733 (2012)), or Fcy-receptor interaction. Thus, the multispecific antibody of the present invention due to its ability to mediate, e.g. agonize, potent CD137 signaling without interacting with Fcy-receptor, does not lead to depletion of CD137-expressing cells. Further, it was surprisingly found that the multispecific antibody of the present disclosure, even when monovalent for CD137, in particular when comprising the novel CD137 binding domain of the present disclosure, is able to cluster and to agonize CD137, however solely in the presence of PDL1-positive cells, thus avoiding systemic activation of CD137. In addition, it has been surprisingly found that, the multispecific antibody of the present disclosure comprising (a) at least one CD137 binding domain (CD137-BD), (b) at least one PDL1 binding domain (PDL1-BD), and (c) at least one human serum albumin binding domain (HSA-BD) demonstrated further beneficial properties such as (i) enhanced clustering of CD137 compared to non-cross-linked bivalent antibodies, (ii) increased half-life of the antibodies while retaining the ability to block PDL1 and to agonize CD137, and (iii) beneficial kinetics (e.g., higher levels of CD137 activation). The multispecific antibodies of the present invention thus provide distinct therapeutic advantages over conventional compositions and therapies.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of'.

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In a first aspect, the present invention relates to a multispecific antibody comprising: (a) at least one CD137 binding domain (CD137-BD), and (b) at least one PDL1 binding domain (PDL1-BD).

The term "antibody" and the like, as used herein, include whole antibodies and any antigen-binding fragment (i.e., "antigen-binding portion") or single chains thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "binding domain", "antigen-binding fragment thereof", "antigen binding portion" of an antibody, and the like, as used herein, refer to one or more fragments of an intact antibody that retain the ability to specifically bind to a given antigen (e.g., CD137, PDL1, HSA). Antigen binding functions of an antibody can be performed by fragments of an intact antibody. In some embodiments, a binding domain of a multispecific antibody of the present invention is selected from the group consisting of a Fab fragment, a monovalent fragment consisting of the VL, VH, CL and CHI domains; a F (ab)2 fragment, a bivalent fragment comprising two Fab fragments linked by a disulfide bridge at the hinge region; an Fd fragment consisting of the VH and CHI domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology). Suitably, a binding domain of the present invention is a single-chain Fv fragment (scFv) or single antibody variable domains. In a preferred embodiment, a binding domain of the present invention is a single-chain Fv fragment (scFv).

The term "Complementarity Determining Regions" ("CDRs") are amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273, 927-948 ("Chothia" numbering scheme), ImMunoGenTics (IMGT) numbering (Lefranc, M.-P., The Immunologist, 7, 132-136 (1999); Lefranc, M.-P. et al., Dev. Comp. Immunol., 27, 55-77 (2003) ("IMGT" numbering scheme) and numbering scheme described in Honegger & Plückthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 2624-32 34 (LCDR1), 50-52 56 (LCDR2), and 9189-96 97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align. In the context of the present invention, the numbering system suggested by Honegger & Plückthun ("AHo) is used (Honegger & Plückthun, J. Mol. Biol. 309 (2001) 657-670), unless specifically mentioned otherwise. Furthermore, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H26H27-H42; HCDR2 (also referred to as CDR-H2): H57-H76; HCDR3 (also referred to as CDR-H3): H108-H138.

The term "binding specificity" as used herein refers to the ability of an individual antibody to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets.

Suitably, the antibody of the invention is an isolated antibody. The term "isolated antibody", as used herein, refers to an antibody that is substantially free of other antibodies having different antigenic specificities (e.g., an isolated antibody that specifically binds CD137 and PDL1 is substantially free of antibodies that specifically bind antigens other than CD137 and PDL1, e.g., an isolated antibody that specifically binds CD137, PDL1 and human serum albumin is substantially free of antibodies that specifically bind antigens other than CD137, PDL1 and human serum albumin). Moreover, an isolated antibody may be substantially free of other cellular material and/or chemicals.

Suitably, the antibody of the invention is a monoclonal antibody. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies of the invention include, but are not limited to, the chimeric, human and humanized.

The term "chimeric antibody" (or antigen-binding fragment thereof) is an antibody molecule (or antigen-binding fragment thereof) in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, e.g., human germline sequences, or mutated versions of human germline sequences. The human antibodies and antigen-binding fragments thereof of the invention may include amino acid residues not encoded by human sequences (e.g., mutations introduced by random or site-specific mutagenesis in vitro or by somatic mutation in vivo). This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1991); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(1):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology). See also, for example, Li et al, Proc. Natl. Acad. Sci. USA, 103:3557- 3562 (2006) regarding human antibodies generated via a human B-cell hybridoma technology.

A "humanized" antibody (or antigen-binding fragment thereof), as used herein, is an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (i.e., the constant region as well as the framework portions of the variable region). Additional framework region modifications may be made within the human framework sequences as well as within the CDR sequences derived from the germline of another mammalian species. The humanized antibodies of the invention may include amino acid residues not encoded by human sequences (e.g., mutations introduced by random or site-specific mutagenesis in vitro or by somatic mutation in vivo, or a conservative substitution to promote stability or manufacturing). See, e.g., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include, but is not limited to Xoma technology disclosed in U.S. Pat. No. 5,766,886. The term "recombinant humanized antibody" as used herein, includes all human antibodies that are prepared, expressed, created or isolated by recombinant means, such as antibodies isolated from a host cell transformed to express the humanized antibody, e.g., from a transfectoma, and antibodies prepared, expressed, created or isolated by any other means that involve splicing of all or a portion of a human immunoglobulin gene, sequences to other DNA sequences.

The term "recombinant humanized antibody" as used herein, includes all human antibodies that are prepared, expressed, created or isolated by recombinant means, such as antibodies isolated from a host cell transformed to express the humanized antibody, e.g., from a transfectoma, and antibodies prepared, expressed, created or isolated by any other means that involve splicing of all or a portion of a human immunoglobulin gene, sequences to other DNA sequences.

The term "multispecific antibody" as used herein, refers to an antibody that binds to two or more different epitopes on at least two or more different targets (e.g., CD137 and PDL1). The term "multispecific antibody" includes bispecific, trispecific, tetraspecific, pentaspecific and hexaspecific. The term "bispecific antibody" as used herein, refers to an antibody that binds to two different epitopes on at least two different targets (e.g., CD137 and PDL1). The term "trispecific antibody" as used herein, refers to an antibody that binds to three different epitopes on at least three different targets (e.g., CD137, PDL1 and HSA).

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein, and show a significantly reduced rate of HD exchange due to Fab binding. The conformation epitope contains, but is not limited to, the functional epitope.

The term "linear epitope" refers to an epitope with all of the points of interaction between the protein and the interacting molecule (such as an antibody) occurring linearly along the primary amino acid sequence of the protein (continuous).

The term "recognize" as used herein refers to an antibody antigen-binding fragment thereof that finds and interacts (e.g., binds) with its conformational epitope.

Suitably, the multispecific antibody of the present invention is monovalent, bivalent or multivalent for CD137 specificity. In one embodiment, the multispecific antibody of the present invention is bivalent for CD137 specificity. In a preferred embodiment, the multispecific antibody of the present invention is monovalent for CD137 specificity.

Suitably, the multispecific antibody of the present invention is monovalent, bivalent or multivalent for PDL1 specificity. In one embodiment, the multispecific antibody of the present invention is bivalent for PDL1 specificity. In a preferred embodiment, the multispecific antibody of the present invention is monovalent for PDL1 specificity.

The term "multivalent antibody" refers to a single binding molecule with more than one valency, where "valency" is described as the number of antigen-binding moieties that binds to epitopes on identical target molecules. As such, the single binding molecule can bind to more than one binding site on a target molecule. Examples of multivalent antibodies include, but are not limited to bivalent antibodies, trivalent antibodies, tetravalent antibodies, pentavalent antibodies, and the like.

The term "monovalent antibody", as used herein, refers to an antibody that binds to a single epitope on a target molecule, such as CD137. Also, the term "binding domain" or "monovalent binding domain", as used herein, refers to a binding domain that binds to a single epitope on a target molecule such as CD137.

The term "bivalent antibody" as used herein, refers to an antibody that binds to two epitopes on at least two identical target molecules, such as CD137 target molecules.

Recently, in order to gain additional cross-linking function and achieve certain levels of CD137 activation, use of multivalent and multispecific fusion polypeptides that bind PDL1 and CD137 was proposed. Eckelman et al. have demonstrated that while bivalent engagement of CD137 in the case of INBRX-105 (a multispecific and multivalent polypeptide having two PDL1 binding domains, two CD137 binding domains and an Fc region) is insufficient to effectively cluster and mediate productive CD137 signaling, engagement of a second cell surface antigen PDL1 in the presence of PDL1-positive cells enables further clustering of CD137 and productive signaling (WO2017/123650).

The inventors of the present invention have now surprisingly found that a multispecific antibody comprising (a) only one CD137 binding domain (CD137-BD); and (b) at least one PDL1 binding domain (PDL1-BD) is able to effectively activate CD137 signaling in a targeted manner. The stable multispecific (e.g., bi-/trispecific) monovalent for CD137 molecules of the present invention are shown to be not capable of agonizing CD137 on T cells in the absence of another cell-type, which is recognized by PDL1 binding domain (Figure 1B). The effective activation of CD137 takes place only in the presence of PDL1-positive cells due to binding of anti-PDL1 domains of the multispecific antibodies of the invention to PDL1 molecules exposed on the surface of PDL1-positive cells (Figure 1C and Figure 2). This leads to increased density of the multispecific antibodies of the present invention in a specific location, and thus increased density of CD137 binding domains. The CD137-BDs thus can effectively cluster and agonize CD137. This concomitant binding to PDL1 and CD137 triggers selective activation of tumor-reactive T cells and simultaneously blocks PD-1 signaling (Figure 2). Due to high overexpression of PDL1 on tumor cells, CD137 signaling is activated only locally in the presence of said tumor cells, which leads to reduced systemic toxicity. The antibody of the invention thus is expected to have several beneficial effects in comparison to current treatment options. The antibody of the invention is predicted to have (i) lower rate of immune-related adverse events, and (ii) lower rate of dose-limiting toxicities.

In a preferred embodiment, the multispecific antibody of the present invention is monovalent for CD137 specificity. Importantly, the monovalent for CD137 specificity multispecific antibody of the present invention is not capable of inducing CD137 signaling systemically due to a lack of CD137 activation in the absence of clustering, which is caused by binding of PDL1-BD to its antigen. In one embodiment, the present invention relates to a multispecific antibody comprising (a) one CD137-BD; and (b) at least one PDL1-BD, preferably one or two PDL1-BDs, more preferably one PDL1-BD. Thus, the multispecific antibody of the invention is monovalent, bivalent or multivalent for PDL1 specificity, preferably monovalent for PDL1 specificity. In one embodiment, the multispecific antibody of the present invention comprises one CD137-BD and one PDL1-BD. In one embodiment, the multispecific antibody of the present invention consist of one CD137-BD and one PDL1-BD.

The term "CD137" refers in particular to human CD137 with UniProt ID number Q07011, or a variant thereof, reproduced herein as SEQ ID NO: 147. Suitably, the CD137-BD of the present invention targets CD137, in particular human CD137 as shown in UniProt ID number Q07011, or a variant thereof, reproduced herein as SEQ ID NO: 147. Suitably, the multispecific antibody of the invention comprising a CD137-BD targets human and cynomoglous (Macaca fascicularis) CD137. Preferably, the multispecific antibody of the invention comprising a CD137-BD does not block CD137/CD137L interaction.

The CD137-BD of the invention specifically binds CD137.

Suitably, the CD137-BD of the invention is CD137 agonist. An "activator" or "activating antibody" or "agonist" or "agonist antibody" or "agonist binding domains" or "activating binding domain" is one that enhances or initiates signaling by the antigen to which it binds. In the context of the present invention, the term "CD137 agonist" encompasses the CD137 binding domains of the invention that are capable to activate CD137 signaling upon their clustering, e.g., wherein binding of at least two of said CD137-BDs allow for multimerization of the bound CD137 molecules and their activation. In some embodiments, agonist antibodies activate signaling without the presence of the natural ligand.

In some embodiments, the CD137-BD of the invention is derived from a monoclonal antibody or antibody fragment.

Suitable CD137-BDs for use in the multispecific antibody of the present invention are novel binding domains provided in the present disclosure. The novel CD137-BDs of the invention include, but are not limited to, the humanized monoclonal antibodies isolated as described herein, including in the Examples. Examples of such CD137-BDs are antibodies or binding domains thereof whose sequences are listed in Table 1. Additional details regarding the generation and characterization of the antibodies and binding domains described herein are provided in the Examples. The novel CD137-BDs of the present invention are particularly suitable for the purposes of the present invention. The multispecific antibodies of the present invention comprising at least one said CD137-BD, e.g. monovalent for CD137 binding specificity, are capable of activating CD137 in the presence of PDL1 positive cells.

Suitably, the CD137-BD specifically binds to CD137 and is characterized by one or more of the following parameters:
(i) binds to human CD137 with a dissociation constant (KD) of less than 50nM, particularly less than10 nM, particularly less than 5 nM, particularly less than 1nM, particularly less than 500 pM, more particularly less than 100 pM, more particularly less than 50 pM;
(ii) binds to human CD137 with a K_{off} rate of 10⁻³ s⁻¹ or less, or 10⁻⁴ s⁻¹ or less, or 10⁻⁵ s⁻¹ or less as measured by SPR;
(iii) binds to human CD137 with a Kₒₙ rate of at least 10⁴ M⁻¹s⁻¹ or greater, at least 10⁵ M⁻¹s⁻¹ or greater, at least 10⁶ M⁻¹s⁻¹ or greater, as measured by SPR;
(iv) does not cross-compete with urelumab and utolimumab; and/or
(v) is cross-reactive with Macaca fascicularis (Cynomolgus) CD137;

The term "avidity" refers to an informative measure of the overall stability or strength of the antibody-antigen complex. It is controlled by three major factors: antibody epitope affinity; the valency of both the antigen and antibody; and the structural arrangement of the interacting parts. Ultimately these factors define the specificity of the antibody, that is, the likelihood that the particular antibody is binding to a precise antigen epitope.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the sum total of non-covalent interactions between a single binding site of a molecule (e.g., of an antibody) and its binding partner (e.g., an antigen). Unless indicated otherwise, as used herein, "binding affinity", "bind to", "binds to" or "binding to" refers to intrinsic binding affinity that reflects a 1:1 interaction between members of a binding pair (e.g., an antibody fragment and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, i.e. binding strength are described in the following.

The term "K_{assoc}", "Ka" or "Kₒₙ", as used herein, is intended to refer to the association rate of a particular antibody-antigen interaction, whereas the term "K_{dis}", "Kd" or "K_{off}", as used herein, is intended to refer to the dissociation rate of a particular antibody- antigen interaction. In one embodiment, the term "KD", as used herein, is intended to refer to the dissociation constant, which is obtained from the ratio of Kd to Ka (i.e. Kd/Ka) and is expressed as a molar concentration (M). The "KD" or "KD value" or "K_{D}" or "K_{D} value" according to this invention is in one embodiment measured by using surface-plasmon resonance assays using a MASS-1 SPR instrument (Sierra Sensors). To measure affinity, an antibody specific for the Fc region of rabbit IgGs (Bethyl Laboratories, Cat. No. A120-111A) is immobilized on a sensor chip (SPR-2 Affinity Sensor, High Capacity Amine, Sierra Sensors) using a standard amine-coupling procedure. Rabbit monoclonal antibodies in B-cell supernatants are captured by the immobilized anti-rabbit IgG antibody. A minimal IgG concentration in the B-cell supernatants is required to allow sufficient capture. After capturing of the monoclonal antibodies, human CD137 ECD (Peprotech, cat. 310-15-1MG) or, as in the case of PDL1-BD, human PDL1 (Peprotech) is injected into the flow cells for 3 min at a concentration of 90 nM, and dissocia-tion of the protein from the IgG captured on the sensor chip was allowed to proceed for 5 min. After each injection cycle, surfaces are regenerated with two injections of 10 mM Glycine-HCl. The apparent dissociation (kd) and association (ka) rate constants and the apparent dissociation equilibrium constant (KD) are calculated with the MASS-1 analysis software (Analyzer, Sierra Sensors) using one-to-one Langmuir binding model and quality of the fits is monitored based on relative Chi² (Chi² normalized to the extrapolated maximal binding level of the analyte), which is a measure for the quality of the curve fitting. The smaller the value for the Chi² the more accurate is the fitting to the one-to-one Langmuir binding model. Results are deemed valid if the response units (RU) for ligand binding are at least 2% of the RUs for antibody capturing. Samples with RUs for ligand binding with less than 2% of the RUs for antibody capturing are considered to show no specific binding of CD137 or PDL1 to the captured antibody. The equilibrium dissociation constant (K_{D}) is calculated as the ratio k_{off}/kₒₙ. See, e.g., Chen et al, J. Mol. Biol. 293:865-881 (1999).

Suitably, the affinity of the multispecific antibody of the invention to CD137 may be comparable to or higher than the affinity of CD137L to CD137. Suitably, the affinity of the multispecific antibody of the invention to CD137 may be comparable to or higher than the affinity of urelumab to CD137. It will be appreciated that the higher affinity of the CD137 binding domain may be particularly suitable for use in the multispecific antibody of the invention, wherein said antibody is monovalent for CD137. The binding affinity of an antibody or binding fragment thereof, may be determined, for example, by the dissociation constant (KD). A stronger affinity is represented by a lower KD, while a weaker affinity is represented by a higher KD.

Thus, in a suitable embodiment, the multispecific antibody of the invention or the CD137-BD of the invention binds to human CD137 with a KD of between 5 to 50000 pM, 5 to 40000 pM, 5 to 30000 pM, 5 to 20000 pM, 5 to 10000 pM, 5 to 5000 pM, 5 to 2500 pM, 5 to 1000 pM, 5 to 750 pM, 5 to 500 pM, 5 to 250 pM, 5 to 100 pM, 5 to 75 pM, 5 to 50 pM, 5 to 30 pM. In a further embodiment, the multispecific antibody of the invention or the CD137-BD of the invention binds to human CD137 with a KD of between 10nM and 10pM, preferably between 10nM and 0.1nM, more preferably between 5nM and 1nM.

In a suitable embodiment, the multispecific antibody of the invention or the CD137-BD of the invention binds to human CD137 with a KD of less than approximately 50nM, less than approximately 45nM, less than approximately 40nM, less than approximately 35nM, less than approximately 30nM, less than approximately 25nM, less than 20nM, less than approximately 15nM, less than approximately 10nM, less than approximately 9nM, less than approximately 8nM, less than approximately 7nM, less than approximately 6nM, less than approximately 5nM, less than approximately 4nM, less than approximately 3nM, less than 2nM, less than 1nM, less than 0.5nM, less than 0.25nM, or less than 0.1nM, less than 50pM, less than 40pM, less than 30pM, less than 20pM. Suitably, the multispecific antibody of the invention or the CD137-BD of the invention binds to human CD137 with a KD of less than 10nM. Preferably, the multispecific antibody of the invention or the CD137-BD of the invention binds to human CD137 with a KD of less than 5nM. Suitably, the multispecific antibody of the invention or the CD137-BD of the invention binds to human CD137 with a KD of less than 1nM. Suitably, the multispecific antibody of the invention or the CD137-BD of the invention binds to human CD137 with a KD of less than 50pM.

Suitably, the multispecific antibody of the invention or the CD137-BD of the invention binds to human CD137 with a Kₒₙ rate of at least 10³ M⁻¹s⁻¹ or greater, at least 10⁴ M⁻¹s⁻¹ or greater, at least 5x10⁴ M⁻¹s⁻¹ or greater, at least 10⁵ M⁻¹s⁻¹ or greater, at least 5x10⁵ M⁻¹s⁻¹ or greater, at least 10⁶ M⁻¹s⁻¹ or greater, at least 5x10⁶ M⁻¹s⁻¹ or greater, at least 10⁷ M⁻¹s⁻¹ or greater, at least 5x10⁷ M-¹s-¹ or greater as measured by surface plasmon resonance (SPR). Suitably, the multispecific antibody of the invention or the CD137-BD of the invention binds to human CD137 with a Kₒₙ rate of at least 10⁴ M⁻¹s⁻¹ or greater, in particular at least 10⁵ M-¹s-¹ or greater, as measured by SPR.

Suitably, the multispecific antibody of the invention or the CD137-BD of the invention binds to human CD137 with a K_{off} rate of 10⁻³ s⁻¹ or less, 3x10⁻³ s⁻¹ or less, 5x10⁻³ s⁻¹ or less, 10⁻⁴ s⁻¹ or less, 5x10⁻⁴ s⁻¹ or less, 10⁻⁵ s⁻¹ or less, 5x10⁻⁵ s⁻¹ or less, 10⁻⁶ s⁻¹ or less, or 10⁻⁷ s⁻¹ or less as measured by surface plasmon resonance (SPR). Suitably, the multispecific antibody of the invention or the CD137-BD of the invention binds to human CD137 with a K_{off} rate of 10⁻⁴ s⁻¹ or less, in particular 10⁻⁵ s⁻¹ or less as measured by SPR.

Suitably the multispecific antibody of the invention or the CD137-BD of the invention has a binding to human CD137 of at least 60% or greater, at least 70% or greater, at least 75% or greater, at least 80% of greater, at least 85% or greater, at least 90% or greater, at least 95% or greater, as measured with SPR and normalized to binding levels obtained for Urelumab.

In one embodiment, the CD137-BD of the invention does not cross-compete for binding with urelumab. The present invention thus provides the CD137-BD that binds to a different epitope than urelumab. Urelumab, also referred to as BMS-663513, is a fully humanized IgG4 mAb from Bristol-Myers Squibb, and is described in WO04/010947, US 6,887,673 and US 7,214,493, which are hereby incorporated into the present application by reference in their entirety.

In one embodiment, the CD137-BD of the invention does not cross-compete for binding with utomilumab. The present invention thus provides the CD137-BD that binds to a different epitope than utomilumab. Utomilumab, also referred to as PF-05082566, is a fully human IgG2 mAb from Pfizer, and is described in WO12/032433 and US 8,821,867, which is hereby incorporated into the present application by reference in its entirety.

In a further embodiment, the CD137-BD of the invention does not cross-compete for binding neither with urelumab nor with utomilumab. The present invention thus provides the CD137-BD of the invention that binds to a different epitope than urelumab and utomilumab.

The terms "compete" or "cross-compete" and related terms are used interchangeably herein to mean the ability of an antibody or other binding agent to interfere with the binding of other antibodies or binding agents to CD137 in a standard competitive binding assay.

The ability or extent to which an antibody or other binding agent is able to interfere with the binding of another antibody or binding molecule to a target of interest, e.g., CD137, PDL1, and therefore whether it can be said to cross-compete according to the invention, can be determined using standard competition binding assays. Suitable quantitative cross-competition assay uses a FACS- or an AlphaScreen-based approach to measure competition between the labelled (e.g. His tagged, biotinylated or radioactive labelled) an antibody or fragment thereof and the other an antibody or fragment thereof in terms of their binding to the target. In general, a cross-competing antibody or fragment thereof is for example one which will bind to the target in the cross-competition assay such that, during the assay and in the presence of a second antibody or fragment thereof, the recorded displacement of the immunoglobulin single variable domain or polypeptide according to the invention is up to 100% (e.g. in FACS based competition assay) of the maximum theoretical displacement (e.g. displacement by cold (e.g. unlabeled) antibody or fragment thereof that needs to be cross-blocked) by the to be tested potentially cross-blocking antibody or fragment thereof that is present in a given amount. Preferably, cross-competing antibodies or fragments thereof have a recorded displacement that is between 10% and 100%, more preferred between 50% to 100%. For the purposes of this invention, a competition ELISA was utilized, the corresponding protocol is described in details in the"Examples" section of the present disclosure.

The CD137-BD of the invention does not inhibit the binding of urelumab or utomiulumab to CD137 protein, which demonstrates that the CD137-BD of the invention cannot compete with urelumab or utomiulumab for binding to CD137; such CD137-BD or an antibody comprising said domain may, according to non-limiting theory, bind to a different (e.g., a structurally different or a spatially remote) epitope on CD137 as urelumab or utomiulumab.

The present invention also provides binding domains that bind to the same epitope as do the CD137-BD listed in Table 1. Additional binding domains can therefore be identified based on their ability to cross-compete (e.g., to competitively inhibit the binding of, in a statistically significant manner) with other antibodies and antigen-binding fragments thereof of the invention in CD137 binding assays.

The ability of a test binding domain to inhibit the binding of the CD137-BD of the invention to CD137 protein demonstrates that the test binding domain can compete with that CD137-BD for binding to CD137; such binding domain may, according to non-limiting theory, bind to the same or a related (e.g., a structurally similar or spatially proximal) epitope on CD137 as the CD137-BD with which it competes. In a certain embodiment, the binding domain that binds to the same epitope on CD137 as the CD137-BD of the present invention is a human or humanized monoclonal antibody. Such human or humanized monoclonal antibodies can be prepared and isolated as described herein.

Once a desired epitope on an antigen is determined, it is possible to generate antibodies to that epitope, e.g., using the techniques described in the present invention. Alternatively, during the discovery process, the generation and characterization of antibodies may elucidate information about desirable epitopes. From this information, it is then possible to competitively screen antibodies for binding to the same epitope. An approach to achieve this is to conduct cross-competition studies to find antibodies that competitively bind with one another, e.g., the antibodies compete for binding to the antigen. A high throughput process for "binning" antibodies based upon their cross-competition is described in International Patent Application No. WO 2003/48731. As will be appreciated by one of skill in the art, practically anything to which an antibody can specifically bind could be an epitope. An epitope can comprise those residues to which the antibody binds.

Regions of a given polypeptide that include an epitope can be identified using any number of epitope mapping techniques, well known in the art. See, e.g., Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al., (1984) Proc. Natl. Acad. Sci. USA 8:3998-4002; Geysen et al., (1985) Proc. Natl. Acad. Sci. USA 82:78-182; Geysen et al., (1986) Mol. Immunol. 23:709-715. Similarly, conformational epitopes are readily identified by determining spatial conformation of amino acids CD137 such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., (1981) Proc. Natl. Acad. Sci USA 78:3824-3828; for determining antigenicity profiles, and the Kyte-Doolittle technique, Kyte et al., (1982) J.MoI. Biol. 157: 105-132; for hydropathy plots.

The present invention provides the CD137-BDs that specifically bind to CD137 protein, said binding domains comprising a VH CDR having an amino acid sequence of any one of the VH CDRs listed in Table 1. In particular, the invention provides CD137-BDs that specifically bind to CD137 protein, said CD137-BDs comprising (or alternatively, consisting of) one, two, three, or more VH CDRs having an amino acid sequence of any of the VH CDRs listed in Table 1.

The present invention also provides CD137-BDs that specifically bind to CD137 protein, said CD137-BDs comprising a VL CDR having an amino acid sequence of any one of the VL CDRs listed in Table 1. In particular, the invention provides CD137-BDs that specifically bind to CD137 protein, said CD137-BDs comprising (or alternatively, consisting of) one, two, three or more VL CDRs having an amino acid sequence of any of the VL CDRs listed in Table 1.

Other CD137-BDs of the invention include amino acids that have been mutated, yet have at least 60, 70, 80, 90, 91, 92, 93, 94, 95, 96, 97, 98 or 99 percent identity in the CDR regions with the CDR regions depicted in the sequences described in Table 1. In one aspect, other CD137-BDs of the invention include mutant amino acid sequences wherein no more than 1, 2, 3, 4 or 5 amino acids have been mutated in the CDR regions when compared with the CDR regions depicted in the sequence described in Table 1.

The terms "identical" or percent "identity", in the context of two or more nucleic acids or polypeptide sequences, refer to two or more sequences or subsequences that are the same. "Percent (%) amino acid sequence identity" and "homology" with respect to nucleic acid, a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2 or ALIGN software. Those skilled in the art can determine appropriate parameters for measuring alignment, including any algorithms needed to achieve maximal alignment over the full length of the sequences being compared.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman (1970) Adv. Appl. Math. 2:482c, by the homology alignment algorithm of Needleman and Wunsch, J. Mol. Biol. 48:443, 1970, by the search for similarity method of Pearson and Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Brent et al., Current Protocols in Molecular Biology, John Wiley and Sons, Inc. (ringbou ed., 2003)).

Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (N) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sci. USA 89: 10915, 1989) alignments (B) of 50, expectation (E) of 10, M=5, N=-4, and a comparison of both strands.

The BLAST algorithm also performs a statistical analysis of the similarity between two sequences (see, e.g., Karlin and Altschul, Proc. Natl. Acad. Sci. USA 90:5873- 5787, 1993). One measure of similarity provided by the BLAST algorithm is the smallest sum probability (P (N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci., 4: 11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, gamma-carboxyglutamate, and O-phosphoserine. The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

Suitably, the CD137-BD of the multispecific antibody of the present invention comprises: (a) a heavy chain variable region CDR1 comprising an amino acid sequence selected from any one of SEQ ID NOs: 1, 2, 5, 8, 30, 33 and 36; (b) a heavy chain variable region CDR2 comprising an amino acid sequence selected from any of SEQ ID NOs: 3, 6, 9, 31, 34 and 37; (c) a heavy chain variable region CDR3 comprising an amino acid sequence selected from any of SEQ ID NOs: 4, 7, 10, 32, 35 and 38; (d) a light chain variable region CDR1 comprising an amino acid sequence selected from any of SEQ ID NOs: 14, 17, 20, 40, 43, and 46; (e) a light chain variable region CDR2 comprising an amino acid sequence selected from any of SEQ ID NOs: 15, 18, 21, 41, 44, and 47; and (f) a light chain variable region CDR3 comprising an amino acid sequence selected from any of SEQ ID NOs: 16, 19, 22, 42, 45 and 48. Suitably, the CD137-BD of the multispecific antibody of the present invention comprises: (a) a heavy chain variable region CDR1 comprising an amino acid sequence having at least 60, 70, 80, 90, 91, 92, 93, 94, 95, 96, 97, 98 or 99 percent identity to any one of SEQ ID NOs: 1, 2, 5, 8, 30, 33 and 36; (b) a heavy chain variable region CDR2 having at least 60, 70, 80, 90, 91, 92, 93, 94, 95, 96, 97, 98 or 99 percent identity to any of SEQ ID NOs: 3, 6, 9, 31, 34 and 37; (c) a heavy chain variable region CDR3 having at least 60, 70, 80, 90, 91, 92, 93, 94, 95, 96, 97, 98 or 99 percent identity to any of SEQ ID NOs: 4, 7, 10, 32, 35 and 38; (d) a light chain variable region CDR1 having at least 60, 70, 80, 90, 91, 92, 93, 94, 95, 96, 97, 98 or 99 percent identity to any of SEQ ID NOs: 14, 17, 20, 40, 43, and 46; (e) a light chain variable region CDR2 having at least 60, 70, 80, 90, 91, 92, 93, 94, 95, 96, 97, 98 or 99 percent identity to any of SEQ ID NOs: 15, 18, 21, 41, 44, and 47; and (f) a light chain variable region CDR3 having at least 60, 70, 80, 90, 91, 92, 93, 94, 95, 96, 97, 98 or 99 percent identity to any of SEQ ID NOs: 16, 19, 22, 42, 45 and 48.

In one embodiment, the CD137-BD of the multispecific antibody of the present invention comprises: (a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively; (b) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively; (c) HCDR1, HCDR2, and HCDR3 sequences of SEQ ID NOs: 5, 6, and 7, respectively, and LCDR1, LCDR2, and LCDR3 sequences of SEQ ID NOs: 17, 18, and 19, respectively; (d) HCDR1, HCDR2, and HCDR3 sequences of SEQ ID NOs: 8, 9, and 10, respectively, and LCDR1, LCDR2, and LCDR3 sequences of SEQ ID NOs: 20, 21, and 22, respectively; (e) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively; (f) HCDR1, HCDR2, and HCDR3 sequences of SEQ ID NOs: 33, 34, and 35, respectively, and LCDR1, LCDR2, and LCDR3 sequences of SEQ ID NOs: 43, 44, and 45, respectively; (g) HCDR1, HCDR2, and HCDR3 sequences of SEQ ID NOs: 36, 37, and 38, respectively, and LCDR1, LCDR2, and LCDR3 sequences of SEQ ID NOs: 46, 47, and 48, respectively.

Suitably, the CD137-BD of the multispecific antibody of the present invention comprises: (a) HCDR1, HCDR2, and HCDR3 sequences having at least 60, 70, 80, 90, 91, 92, 93, 94, 95, 96, 97, 98 or 99 percent identity to SEQ ID NOs: 1, 3, and 4, respectively, and LCDR1, LCDR2, and LCDR3 sequences having at least 60, 70, 80, 90, 91, 92, 93, 94, 95, 96, 97, 98 or 99 percent identity to SEQ ID NOs: 14, 15, and 16, respectively; (b) HCDR1, HCDR2, and HCDR3 sequences having at least 60, 70, 80, 90, 91, 92, 93, 94, 95, 96, 97, 98 or 99 percent identity to SEQ ID NOs: 2, 3, and 4, respectively, and LCDR1, LCDR2, and LCDR3 sequences having at least 60, 70, 80, 90, 91, 92, 93, 94, 95, 96, 97, 98 or 99 percent identity to SEQ ID NOs: 14, 15, and 16, respectively; (c) HCDR1, HCDR2, and HCDR3 sequences having at least 60, 70, 80, 90, 91, 92, 93, 94, 95, 96, 97, 98 or 99 percent identity to SEQ ID NOs: 5, 6, and 7, respectively, and LCDR1, LCDR2, and LCDR3 sequences having at least 60, 70, 80, 90, 91, 92, 93, 94, 95, 96, 97, 98 or 99 percent identity to SEQ ID NOs: 17, 18, and 19, respectively; (d) HCDR1, HCDR2, and HCDR3 sequences having at least 60, 70, 80, 90, 91, 92, 93, 94, 95, 96, 97, 98 or 99 percent identity to SEQ ID NOs: 8, 9, and 10, respectively, and LCDR1, LCDR2, and LCDR3 sequences having at least 60, 70, 80, 90, 91, 92, 93, 94, 95, 96, 97, 98 or 99 percent identity to SEQ ID NOs: 20, 21, and 22, respectively; (e) HCDR1, HCDR2, and HCDR3 sequences having at least 60, 70, 80, 90, 91, 92, 93, 94, 95, 96, 97, 98 or 99 percent identity to SEQ ID NOs: 30, 31, and 32, respectively, and LCDR1, LCDR2, and LCDR3 sequences having at least 60, 70, 80, 90, 91, 92, 93, 94, 95, 96, 97, 98 or 99 percent identity to SEQ ID NOs: 40, 41, and 42, respectively; (f) HCDR1, HCDR2, and HCDR3 sequences having at least 60, 70, 80, 90, 91, 92, 93, 94, 95, 96, 97, 98 or 99 percent identity to SEQ ID NOs: 33, 34, and 35, respectively, and LCDR1, LCDR2, and LCDR3 sequences having at least 60, 70, 80, 90, 91, 92, 93, 94, 95, 96, 97, 98 or 99 percent identity to SEQ ID NOs: 43, 44, and 45, respectively; (g) HCDR1, HCDR2, and HCDR3 sequences having at least 60, 70, 80, 90, 91, 92, 93, 94, 95, 96, 97, 98 or 99 percent identity to SEQ ID NOs: 36, 37, and 38, respectively, and LCDR1, LCDR2, and LCDR3 sequences having at least 60, 70, 80, 90, 91, 92, 93, 94, 95, 96, 97, 98 or 99 percent identity to SEQ ID NOs: 46, 47, and 48, respectively.

In a further embodiment, the CD137-BD of the multispecific antibody of the present invention comprises: (a) a HCDR1 comprising the amino acid sequence of SEQ ID NO: 1 or SEQ ID NO: 2; (b) a HCDR2 comprising the amino acid sequence of SEQ ID NO: 3; (c) a HCDR3 comprising the amino acid sequence of SEQ ID NO: 4; (d) a LCDR1 comprising the amino acid sequence of SEQ ID NOs: 14; (e) a LCDR2 comprising the amino acid sequence of SEQ ID NOs: 15; and (f) a LCDR3 comprising the amino acid sequence of SEQ ID NO: 16.

Suitably, the CD137-BD of the multispecific antibody of the present invention comprises: (a) a HCDR1 comprising the amino acid sequence having at least 60, 70, 80, 90, 91, 92, 93, 94, 95, 96, 97, 98 or 99 percent identity to SEQ ID NO: 1 or SEQ ID NO: 2; (b) a HCDR2 comprising the amino acid sequence having at least 60, 70, 80, 90, 91, 92, 93, 94, 95, 96, 97, 98 or 99 percent identity to SEQ ID NO: 3; (c) a HCDR3 comprising the amino acid sequence having at least 60, 70, 80, 90, 91, 92, 93, 94, 95, 96, 97, 98 or 99 percent identity to SEQ ID NO: 4; (d) a LCDR1 comprising the amino acid sequence having at least 60, 70, 80, 90, 91, 92, 93, 94, 95, 96, 97, 98 or 99 percent identity to SEQ ID NOs: 14; (e) a LCDR2 comprising the amino acid sequence having at least 60, 70, 80, 90, 91, 92, 93, 94, 95, 96, 97, 98 or 99 percent identity to SEQ ID NOs: 15; and (f) a LCDR3 comprising the amino acid sequence of SEQ ID NO: 16.

In a further embodiment, the CD137-BD of the multispecific antibody of the present invention comprises: (a) a HCDR1 comprising the amino acid sequence of SEQ ID NO: 30; (b) a HCDR2 comprising the amino acid sequence of SEQ ID NO: 31; (c) a HCDR3 comprising the amino acid sequence of SEQ ID NO: 32; (d) a LCDR1 comprising the amino acid sequence of SEQ ID NOs: 40; (e) a LCDR2 comprising the amino acid sequence of SEQ ID NOs: 41; and (f) a LCDR3 comprising the amino acid sequence of SEQ ID NO: 42.

Suitably, the CD137-BD of the multispecific antibody of the present invention comprises: (a) a HCDR1 comprising the amino acid sequence having at least 60, 70, 80, 90, 91, 92, 93, 94, 95, 96, 97, 98 or 99 percent identity to SEQ ID NO: 30; (b) a HCDR2 comprising the amino acid sequence having at least 60, 70, 80, 90, 91, 92, 93, 94, 95, 96, 97, 98 or 99 percent identity to SEQ ID NO: 31; (c) a HCDR3 comprising the amino acid sequence having at least 60, 70, 80, 90, 91, 92, 93, 94, 95, 96, 97, 98 or 99 percent identity to SEQ ID NO: 32; (d) a LCDR1 comprising the amino acid sequence having at least 60, 70, 80, 90, 91, 92, 93, 94, 95, 96, 97, 98 or 99 percent identity to SEQ ID NOs: 40; (e) a LCDR2 comprising the amino acid sequence having at least 60, 70, 80, 90, 91, 92, 93, 94, 95, 96, 97, 98 or 99 percent identity to SEQ ID NOs: 41; and (f) a LCDR3 comprising the amino acid sequence having at least 60, 70, 80, 90, 91, 92, 93, 94, 95, 96, 97, 98 or 99 percent identity to SEQ ID NO: 42.

In a further embodiment, the CD137-BD of the multispecific antibody of the present invention comprises a VH domain and a VL domain. In the context of the present invention the terms "VH" (variable heavy chain), "VL" (variable light chain), "Vκ" and "Vλ" refer to families of antibody heavy and light chain sequences that are grouped according to sequence identity and homology. Methods for the determination of sequence homologies, for example by using a homology search matrix such as BLOSUM (Henikoff, S. & Henikoff, J. G., Proc. Natl. Acad. Sci. USA 89 (1992) 10915-10919), and methods for the grouping of sequences according to homologies are well known to one of ordinary skill in the art. For VH, Vκ and Vλ different subfamilies can be identified, as shown, for example, in Knappik et al., J. Mol. Biol. 296 (2000) 57-86, which groups VH in VH1A, VH1B and VH2 to VH6, Vκ in Vκ1 to Vκ4 and Vλ in Vλ1 to Vλ3. In vivo, antibody Vκ chains, Vλ chains, and VH chains are the result of the random rearrangement of germline κ chain V and J segments, germline λ chain V and J segments, and heavy chain V, D and J segments, respectively. To which subfamily a given antibody variable chain belongs is determined by the corresponding V segment, and in particular by the framework regions FR1 to FR3. Thus, any VH sequence that is characterized in the present application by a particular set of framework regions HFR1 to HFR3 only, may be combined with any HFR4 sequence, for example a HFR4 sequence taken from one of the heavy chain germline J segments, or a HFR4 sequence taken from a rearranged VH sequence.

Suitably, the CD137-BD of the multispecific antibody of the present invention comprises a VH4 or VH3 domain, preferably VH4 domain.

Suitably, the CD137-BD of the multispecific antibody of the present invention comprises Vκ frameworks FR1, FR2 and FR3, particularly Vκ1 or Yκ3 frameworks, preferably Vκ1 frameworks FR1 to 3, and a framework FR4, which is selected from a Vκ FR4, particularly Vκ1 FR4, Yκ3 FR4, and a Vλ FR4. Suitable Vλ FR4 are as set forth in SEQ ID NO: 149 to SEQ ID NO: 155. In one embodiment, the CD137-BD of the multispecific antibody of the present invention comprises Vλ FR4 comprising the amino acid sequence having at least 60, 70, 80, 90 percent identity to comprising an amino acid sequence selected from any of SEQ ID NO: 149 to SEQ ID NO: 155.

Thus, in one embodiment, the CD137-BD of the multispecific antibody of the present invention comprises:
(i) the HCDR1, HCDR2, and HCDR3 sequences of:
   a. SEQ ID NOs: 1, 3, and 4, respectively, and the LCDR1, LCDR2, and LCDR3 sequences of SEQ ID NOs: 14, 15, and 16, respectively;
   b. SEQ ID NOs: 2, 3, and 4, respectively, and the LCDR1, LCDR2, and LCDR3 sequences of SEQ ID NOs: 14, 15, and 16, respectively; or
   c. SEQ ID NOs: 30, 31, and 32, respectively, and the LCDR1, LCDR2, and LCDR3 sequences of SEQ ID NOs: 40, 41, and 42, respectively
(ii) a VH3 or VH4 domain, preferably VH4 domain; and
(iii) a VL domain comprising a VL framework comprising Vκ frameworks FR1, FR2 and FR3, particularly Vκ1 or Yκ3 FR1 to FR3, preferably Vκ1 FR1 to FR3, and a framework FR4, which is selected from a Vκ FR4, particularly Vκ1 FR4, Yκ3 FR4, and a Vλ FR4, particularly Vλ FR4 comprising the amino acid sequence having at least 60, 70, 80, 90 percent identity to comprising an amino acid sequence selected from any of SEQ ID NO: 149 to SEQ ID NO: 155, more particularly Vλ FR4 comprising an amino acid sequence selected from any of SEQ ID NO: 149 to SEQ ID NO: 155, preferably Vλ FR4 comprising an amino acid sequence SEQ ID NO: 149.

In one embodiment, the CD137-BD of the multispecific antibody of the present invention comprises a VL comprising:
(i) CDR domains CDR1, CDR2 and CDR3;
(ii) human Vκ framework regions FR1 to FR3, particularly human Vκ1 framework regions FR1 to FR3;
(iii) FR4, which is selected from (a) a human Vλ germ line sequence for FR4, particularly a Vλ germ line sequence selected from the list of: SEQ ID NO: 149 to 155, preferably Vλ FR4 comprising an amino acid sequence SEQ ID NO: 149; and (b) a Vλ-based sequence, which has one or two mutations, particularly one mutation, compared to the closest human Vλ germ line sequence for FR4 comprising an amino acid sequence selected from any of SEQ ID NO: 149 to SEQ ID NO: 155, preferably Vλ FR4 comprising an amino acid sequence SEQ ID NO: 149.

In a preferred embodiment, the CD137-BD of the multispecific antibody of the present invention comprises:
(iv) the HCDR1, HCDR2, and HCDR3 sequences of:
   a. SEQ ID NOs: 1, 3, and 4, respectively, and the LCDR1, LCDR2, and LCDR3 sequences of SEQ ID NOs: 14, 15, and 16, respectively;
   b. SEQ ID NOs: 2, 3, and 4, respectively, and the LCDR1, LCDR2, and LCDR3 sequences of SEQ ID NOs: 14, 15, and 16, respectively; or
   c. SEQ ID NOs: 30, 31, and 32, respectively, and the LCDR1, LCDR2, and LCDR3 sequences of SEQ ID NOs: 40, 41, and 42, respectively
(v) a VH4 domain; and
(vi) a VL domain comprising a VL framework comprising Vκ1 frameworks FR1, FR2 and FR3, and a Vλ FR4 comprising the amino acid sequence having at least 60, 70, 80, 90 percent identity to comprising an amino acid sequence selected from any of SEQ ID NO: 149 to SEQ ID NO: 155, particularly Vλ FR4 as set forth in SEQ ID NO: 149 to SEQ ID NO: 155, preferably SEQ ID NO: 149.

Suitably, the CD137-BD of the invention comprises a VH domain listed in Table 1. Suitably, the CD137-BD of the invention comprises (or alternatively, consisting of) a VH amino acid sequence listed in Table 1, wherein no more than about 10 amino acids in a framework sequence (for example, a sequence which is not a CDR) have been mutated (wherein a mutation is, as various non-limiting examples, an addition, substitution or deletion). Suitably, the CD137-BD of the invention comprises (or alternatively, consisting of) a VH amino acid sequence listed in Table 1, wherein no more than about 20 amino acids in a framework sequence (for example, a sequence which is not a CDR) have been mutated (wherein a mutation is, as various non-limiting examples, an addition, substitution or deletion). Other CD137-BD of the invention include amino acids that have been mutated, yet have at least 60, 70, 80, 90, 91, 92, 93, 94, 95, 96, 97, 98 or 99 percent identity in the VH regions with the VH regions depicted in the sequences described in Table 1.

Suitably, the CD137-BD of the invention comprises a VL domain listed in Table 1. Suitably, the CD137-BD of the invention comprises (or alternatively, consisting of) a VL amino acid sequence listed in Table 1, wherein no more than about 10 amino acids in a framework sequence (for example, a sequence which is not a CDR) have been mutated (wherein a mutation is, as various non- limiting examples, an addition, substitution or deletion). Suitably, the CD137-BD of the invention comprises (or alternatively, consisting of) a VL amino acid sequence listed in Table 1, wherein no more than about 20 amino acids in a framework sequence (for example, a sequence which is not a CDR) have been mutated (wherein a mutation is, as various non- limiting examples, an addition, substitution or deletion). Other CD137-BD of the invention include amino acids that have been mutated, yet have at least 60, 70, 80, 90, 91, 92, 93, 94, 95, 96, 97, 98 or 99 percent identity in the VL regions with the VL regions depicted in the sequences described in Table 1.

Suitably, the CD137-BD of the invention comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 11, 12, 13 and 39; and a light chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 23, 24, 25 and 49.

Suitably, the CD137-BD of the present invention comprises: a heavy chain variable region comprising an amino acid sequence selected from any of SEQ ID NOs: 11, 12, 13 and 39; and a light chain variable region comprising an amino acid sequence selected from any of SEQ ID NOs: 23, 24, 25 and 49.

In a further embodiment, the CD137-BD of the present invention comprises: (a) a VH sequence of SEQ ID NO: 11 and a VL sequence of SEQ ID NO: 23; (b) a VH sequence of SEQ ID NO: 12 and a VL sequence of SEQ ID NO: 24; (c) a VH sequence of SEQ ID NO: 13 and a VL sequence of SEQ ID NO: 25; or (d) a VH sequence of SEQ ID NO: 39 and a VL sequence of SEQ ID NO: 49.

In one embodiment, the CD137-BD of the present invention comprises:
(a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence at least 60, 70, 80, 90, 91, 92, 93, 94, 95, 96, 97, 98 or 99 percent identical to the amino acid sequence SEQ ID NO: 11, and a VL sequence at least 60, 70, 80, 90, 91, 92, 93, 94, 95, 96, 97, 98 or 99 percent identical to the amino acid sequence SEQ ID NO: 23;
(b) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence at least 60, 70, 80, 90, 91, 92, 93, 94, 95, 96, 97, 98 or 99 percent identical to the amino acid sequence SEQ ID NO: 12, and a VL sequence at least 60, 70, 80, 90, 91, 92, 93, 94, 95, 96, 97, 98 or 99 percent identical to the amino acid sequence SEQ ID NO: 24;
(c) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively, a VH sequence at least 60, 70, 80, 90, 91, 92, 93, 94, 95, 96, 97, 98 or 99 percent identical to the amino acid sequence SEQ ID NO: 13, and a VL sequence at least 60, 70, 80, 90, 91, 92, 93, 94, 95, 96, 97, 98 or 99 percent identical to the amino acid sequence SEQ ID NO: 25; or
(d) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively, a VH sequence at least 60, 70, 80, 90, 91, 92, 93, 94, 95, 96, 97, 98 or 99 percent identical to the amino acid sequence SEQ ID NO: 39, and a VL sequence at least 60, 70, 80, 90, 91, 92, 93, 94, 95, 96, 97, 98 or 99 percent identical to the amino acid sequence SEQ ID NO: 49.

In one embodiment, the CD137-BD of the present invention comprises:
(a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence of SEQ ID NO: 11 and a VL sequence of SEQ ID NO: 23;
(b) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence of SEQ ID NO: 12 and a VL sequence of SEQ ID NO: 24;
(c) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively, a VH sequence of SEQ ID NO: 13 and a VL sequence of SEQ ID NO: 25; or
(d) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively, a VH sequence of SEQ ID NO: 39 and a VL sequence of SEQ ID NO: 49.

In one embodiment, the CD137-BD of the present invention is described in Table 1. In one embodiment, the CD137-BD of the present invention is at least 60, 70, 80, 90, 91, 92, 93, 94, 95, 96, 97, 98 or 99 percent identical to the amino acid sequence selected from the group consisting of SEQ ID NO: 27, SEQ ID NO: 28, SEQ ID NO: 29, or SEQ ID NO: 51. In one embodiment, the CD137-BD of the present invention is as set forth in SEQ ID NO: 27, SEQ ID NO: 28, SEQ ID NO: 29, or SEQ ID NO: 51. In one embodiment, the CD137-BD of the present invention is as set forth in SEQ ID NO: 28 or SEQ ID NO: 29. In a preferred embodiment, the CD137-BD of the present invention is as set forth in SEQ ID NO: 29.

Other CD137-BD of the present invention include those wherein the amino acids or nucleic acids encoding the amino acids have been mutated, yet have at least 60, 70, 80, 90 or 95 percent identity to the sequences described in Table 1. In one embodiment, it includes mutant amino acid sequences wherein no more than 1, 2, 3, 4 or 5 amino acids have been mutated in the variable regions when compared with the variable regions depicted in the sequence described in Table 1, while retaining substantially the same therapeutic activity.

Given that each of these binding domains or antibodies can bind to CD137 and that antigen-binding specificity is provided primarily by the CDR1, 2 and 3 regions, the VH CDR1, 2 and 3 sequences and VL CDR1, 2 and 3 sequences can be "mixed and matched" (i.e., CDRs from different binding domains or antibodies can be mixed and match, although each binding domains or each antibody must contain a VH CDR1, 2 and 3 and a VL CDR1, 2 and 3 to create other CD137-binding molecules of the invention. Such "mixed and matched" CD137-BD can be tested using the binding assays known in the art and those described in the Examples (e.g., ELISAs). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s). It will be readily apparent to the ordinarily skilled artisan that novel VH and VL sequences can be created by mutating one or more VH and/or VL CDR region sequences with structurally similar sequences from the CDR sequences shown herein for monoclonal antibodies or binding domains of the present invention.

In yet another embodiment, the present invention provides a CD137-BD comprising amino acid sequences that are homologous to the sequences described in Table 1, and said binding domain binds to CD137, and retains the desired functional properties of those binding domains described in Table 1.

For example, the invention provides a CD137-BD comprising a heavy chain variable region and a light chain variable region, wherein the heavy chain variable region comprises an amino acid sequence that is at least 80 percent, at least 90 percent, or at least 95 percent identical to an amino acid sequence selected from the group consisting of SEQ ID NOs: 11, 12, 13 and 39; the light chain variable region comprises an amino acid sequence that is at least 80 percent, at least 90 percent, or at least 95 percent identical to an amino acid sequence selected from the group consisting of SEQ ID NOs: 23, 24, 25 and 49; wherein the binding domain specifically binds to human CD137 protein.

In one embodiment, the VH and/or VL amino acid sequences may be 50 percent, 60 percent, 70 percent, 80 percent, 90 percent, 95 percent, 96 percent, 97 percent, 98 percent or 99 percent identical to the sequences set forth in Table 1. In one embodiment, the VH and/or VL amino acid sequences may be identical except an amino acid substitution in no more than 1, 2, 3, 4 or 5 amino acid positions.

In one embodiment, the CD137-BD of the invention has a heavy chain variable region comprising CDR1, CDR2, and CDR3 sequences and a light chain variable region comprising CDR1, CDR2, and CDR3 sequences, wherein one or more of these CDR sequences have specified amino acid sequences based on the CD137-BDs described herein or conservative modifications thereof, and wherein the CD137-BD retains the desired functional properties of the CD137-BD of the invention.

The term "conservatively modified variant" or "conservative variants" applies to both amino acid and nucleic acid sequences. With respect to particular nucleic acid sequences, conservatively modified variants refer to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" or "conservative variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In one embodiment, the term "conservative sequence modifications" are used to refer to amino acid modifications that do not significantly affect or alter the binding characteristics of the antibody containing the amino acid sequence.

Accordingly, the invention provides a CD137-BD consisting of a heavy chain variable region comprising CDR1, CDR2, and CDR3 sequences and a light chain variable region comprising CDR1, CDR2, and CDR3 sequences, wherein: the heavy chain variable region CDR1 comprises an amino acid sequence selected from any of SEQ ID NOs: 1, 2, 5, 8, 30, 33 and 36, or conservative variants thereof; the heavy chain variable region CDR2 comprises an amino acid sequence selected from any of SEQ ID NOs: 3, 6, 9, 31, 34 and 37, or conservative variants thereof; the heavy chain variable region CDR3 comprises an amino acid sequence selected from any of SEQ ID NOs: 4, 7, 10, 32, 35 and 38, or conservative variants thereof; the light chain variable region CDR1 comprises an amino acid sequence selected from any of SEQ ID NOs: 14, 17, 20, 40, 43, and 46, or conservative variants thereof; the light chain variable region CDR2 comprises an amino acid sequence selected from any of SEQ ID NOs: 15, 18, 21, 41, 44, and 47, or conservative variants thereof; and the light chain variable region CDR3 comprises an amino acid sequence selected from any of SEQ ID NOs: 16, 19, 22, 42, 45 and 48, or conservative variants thereof; wherein said CD137-BD is capable of activating CD137 signaling with or without additional cross-linking.

In one embodiment, the CD137-BD or the multispecific antibody comprising said CD137-BD of the invention optimized for expression in a mammalian cell has a heavy chain variable region and a light chain variable region, wherein one or more of these sequences have specified amino acid sequences based on the CD137-BDs described herein or conservative modifications thereof, and wherein the CD137-BD or the multispecific antibody comprising said CD137-BD retains the desired functional properties of the CD137-BD of the invention. Accordingly, the invention provides the CD137-BD or the multispecific antibody comprising said CD137-BD of the invention optimized for expression in a mammalian cell comprising a heavy chain variable region and a light chain variable region wherein: the heavy chain variable region comprises an amino acid sequence selected from any of SEQ ID NOs: 11, 12, 13 and 39, and conservative modifications thereof; and the light chain variable region comprises an amino acid sequence selected from any of SEQ ID NOs: 23, 24, 25 and 49, and conservative modifications thereof; wherein said CD137-BD is capable of activating CD137 signaling with or without additional cross-linking.

As used herein, the term, "optimized" means that a nucleotide sequence has been altered to encode an amino acid sequence using codons that are preferred in the production cell or organism, generally a eukaryotic cell, for example, a cell of Pichia, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence. The optimized sequences herein have been engineered to have codons that are preferred in mammalian cells. However, optimized expression of these sequences in other eukaryotic cells or prokaryotic cells is also envisioned herein. The amino acid sequences encoded by optimized nucleotide sequences are also referred to as optimized.

Another type of variable region modification is to mutate amino acid residues within the VH and/or VL CDR1, CDR2 and/or CDR3 regions to thereby improve one or more binding properties (e.g., affinity) of the antibody of interest, known as "affinity maturation." Site-directed mutagenesis or PCR-mediated mutagenesis can be performed to introduce the mutation (s) and the effect on antibody binding, or other functional property of interest, can be evaluated in in vitro or in vivo assays as described herein and provided in the Examples. Conservative modifications (as discussed above) can be introduced. The mutations may be amino acid substitutions, additions or deletions. Moreover, typically no more than one, two, three, four or five residues within a CDR region are altered.

An "affinity-matured" antibody or binding domain is one with one or more alterations in one or more variable domains thereof that result in an improvement in the affinity of the antibody or binding domain for antigen, compared to a parent antibody or binding domain that does not possess those alteration(s). In one embodiment, an affinity-matured antibody or binding domain has nanomolar or even picomolar affinities for the target antigen. Affinity-matured antibodies or domains are produced by procedures known in the art. For example, Marks et al, Bio/Technology 10:779-783 (1992) describes affinity maturation by VH- and VL-domain shuffling. Random mutagenesis of HVR and/or framework residues is described by, for example: Barbas et al. Proc Nat. Acad. ScL USA 91:3809-3813 (1994); Schier et al. Gene 169:147-155 (1995); Jackson et al, J. Immunol. 154(7):3310- 9 (1995); and Hawkins et al, J. Mol. Biol. 226:889-896 (1992).

In one embodiment, an "affinity-matured" CD137-BD of the invention comprises: a VH4 comprising I44V; F89V; Y105F mutations, in particular comprising an amino acid sequence according to SEQ ID NO: 12; and a VL comprising A51P mutation, in particular comprising an amino acid sequence according to SEQ ID NO: 24.

In another embodiment, an "affinity-matured" CD137-BD of the invention comprises: a VH4 comprising V25A; I44V; V82K; F89V; Y105F mutations, in particular comprising an amino acid sequence according to SEQ ID NO: 13; and a VL comprising I2L; A51P mutations, in particular comprising an amino acid sequence according to SEQ ID NO: 25.

A CD137-BD of the invention can be prepared using an antibody or a binding domain thereof having one or more of the VH and/or VL sequences shown herein as starting material to engineer a modified binding domain, which may have altered properties from the starting binding domain. A binding domain can be engineered by modifying one or more residues within one or both variable regions (i.e., VH and/or VL), for example within one or more CDR regions and/or within one or more framework regions.

One type of variable region engineering that can be performed is CDR grafting. Antibodies or binding domains thereof interact with target antigens predominantly through amino acid residues that are located in the six heavy and light chain complementarity determining regions (CDRs). For this reason, the amino acid sequences within CDRs are more diverse between individual antibodies or binding domains thereof than sequences outside of CDRs. Because CDR sequences are responsible for most antibody-antigen interactions, it is possible to express recombinant antibodies or binding domains that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998 Nature 332:323-327; Jones, P. et al., 1986 Nature 321:522- 525; Queen, C. et al., 1989 Proc. Natl. Acad., U.S.A. 86: 10029-10033; U.S. Pat. No. 5,225,539 to Winter, and U.S. Pat. Nos. 5,530,101; 5,585,089; 5,693,762 and 6,180,370 to Queen et al.).

Such framework sequences can be obtained from public DNA databases or published references that include germine antibody gene sequences or rearranged antibody sequences. For example, germine DNA sequences for human heavy and light chain variable region genes can be found in the "VBase" human germline sequence database (available on the Internet at www.mrc-cpe.cam.ac.uk/vbase), as well as in Kabat, E. A., et al., 1991 Sequences of Proteins of Immunological Interest, Fifth Edition, U.S. Department of Health and Human Services, NIH Publication No. 91-3242; Tomlinson, I. M., et al., 1992 J. fol. Biol. 227:776-798; and Cox, J. P. L. et al., 1994 Eur. J Immunol. 24:827-836; the contents of each of which are expressly incorporated herein by reference. For example, germline DNA sequences for human heavy and light chain variable region genes and rearranged antibody sequences can be found in "IMGT" database (available on the Internet at www.imgt.org; see Lefranc, M.P. et al., 1999 Nucleic Acids Res. 27:209-212; the contents of each of which are expressly incorporated herein by reference).

An example of framework sequences for use in the CD137-BD of the invention are those that are structurally similar to the framework sequences used by selected CD137-BDs the invention. The VH CDR1, 2 and 3 sequences, and the VL CDR1, 2 and 3 sequences, can be grafted onto framework regions that have the identical sequence as that found in the germline immunoglobulin gene from which the framework sequence derive, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody or binding domain thereof (see e.g., U.S. Pat. Nos. 5,530,101; 5,585,089; 5,693,762 and 6,180,370 to Queen et al).

Suitably, the CD137-BD of the invention is selected from the group consisting of: a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).

Suitably, the CD137-BD of the invention is scFv antibody fragment. "Single-chain Fv" or "scFv" or "sFv" antibody fragments comprise the VH and VL domains of an antibody, wherein these domains are present in a single polypeptide chain. Generally, the Fv polypeptide further comprises a polypeptide linker between the VH and VL domains which enables the sFv to form the desired structure for target binding. "Single-chain Fv" or "scFv" antibody fragments comprise the VH and VL domains of antibody, wherein these domains are present in a single polypeptide chain. Generally, the scFv polypeptides further comprises a polypeptide linker between the VH and VL domains which enables the scFv to form the desired structure for antigen binding (see, for example, Plückthun, The pharmacology of Monoclonal Antibodies, vol. 113, Rosenburg and Moore eds., (Springer-Verlag, New York, 1994), pp. 269-315). In particular embodiments, said CD137-BD is an scFv comprising the linker according to SEQ ID NO: 26. In a further embodiment, the CD137-BD of the invention is a single-chain variable fragment (scFv) as shown in SEQ ID NO:27, SEQ ID NO: 28, SEQ ID NO: 29 and SEQ ID NO: 51. In one embodiment, the CD137-BD of the invention is a single-chain variable fragment (scFv) as shown in SEQ ID NO: 28. In another embodiment, the CD137-BD of the invention is a single-chain variable fragment (scFv) as shown in SEQ ID NO: 29.

Other suitable CD137 binding domain for use in the multispecific antibody of the present invention comprises or is derived from an antibody selected from the group consisting of: (i) urelumab (BMS-663513; a fully humanized IgG4 mAb; Bristol-Myers Squibb; described in WO04/010947, US 6,887,673 and US 7,214,493, which are hereby incorporated into the present application by reference in their entirety); and (ii) utomilumab (PF-05082566; a fully human IgG2 mAb; Pfizer; described in WO12/032433 and US 8,821,867, which is hereby incorporated into the present application by reference in its entirety).

The multispecific antibody of the present invention comprises at least one PDL1 binding domain (PDL1-BD).

The term "PDL1" refers in particular to human PDL1 with UniProt ID number Q9NZQ7, or a variant thereof, reproduced herein as SEQ ID NO: 148. Suitably, the PDL1-BD of the present invention target PDL1, in particular human PDL1 as shown in UniProt ID number Q9NZQ7, or a variant thereof, reproduced herein as SEQ ID NO: 148. Suitably, the antibodies of the present invention or antigen-binding fragments thereof comprising a PDL1-BD target human and cynomoglous (Macaca fascicularis) PDL1, and preferably does not cross-react with Mus musculus PDL1. The PDL1-BD of the present invention specifically binds to human PDL1 protein.

The PDL1-BD of the present invention is a PDL1 inhibitor. The term "blocker" or "blocking antibody" or "inhibitor" or "inhibiting antibody" or "antagonist" or "antagonist antibody" or "blocking binding domains" or "inhibiting binding domain" refers to an antibody or binding domain thereof that inhibits or reduces a biological activity of the antigen it binds to. In some embodiments, blocking antibodies or blocking binding domains or antagonist antibodies or antagonist binding domains substantially or completely inhibit the biological activity of the antigen. The PDL1-BD of the present invention targets, decreases, inhibits the binding ability of PDL1 to its binding partners, thereby interfering with the PDL1 function. In particular, the PDL1-BD of the present invention blocks the interaction of PDL1 with its receptor, specifically with PD-1. In some embodiments, the PDL1-BD of the present invention blocks the interaction of PDL1 with its receptor or receptors, specifically with PD-1 and/or B7-1.

In some embodiments, the PDL1-BD is derived from a monoclonal antibody or antibody fragment.

Suitable PDL1-BDs for use in the multispecific antibody of the present invention are novel binding domains provided in the present disclosure. The novel PDL1 binding domains of the present invention include, but are not limited to, the humanized monoclonal antibodies isolated as described herein, including in the Examples. Examples of such PDL1-BDs are antibodies or binding domains thereof whose sequences are listed in Table 2.

Suitably, the PDL1-BD of the present invention specifically binds to PDL1 and is characterized by one or more of the following parameters:
(i) binds to human PDL1 with a dissociation constant (KD) of less than 50nM, particularly less than10 nM, particularly less than 5 nM, particularly less than 1nM, particularly less than 500 pM, more particularly less than 100 pM, preferably less than 10pM, more preferably 5pM;
(ii) binds to human PDL1 with a K_{off} rate of 10⁻³ s⁻¹ or less, or 10⁻⁴ s⁻¹ or less, or 10⁻⁵ s⁻¹ or less as measured by SPR;
(iii) binds to human PDL1 with a Kₒₙ rate of at least 10³ M-¹s-¹ or greater, at least 10⁴ M⁻¹s⁻¹ or greater, at least 10⁵ M⁻¹s⁻¹ or greater, at least 10⁶ M⁻¹s⁻¹ or greater as measured by SPR;
(iv) is cross-reactive with Macaca fascicularis (Cynomolgus) PDL1; and/or
(v) is non-cross-reactive to Mus musculus PDL1.

In one embodiment, the PDL1-BD of the present invention has a high affinity to PDL1, e.g., human PDL1. In a suitable embodiment, the PDL1-BD of the invention binds to human PDL1 with a KD of between 1 to 50000 pM, 1 to 40000 pM, 1 to 30000 pM, 1 to 20000 pM, 1 to 10000 pM, 1 to 5000 pM, 1 to 2500 pM, 1 to 1000 pM, 1 to 750 pM, 1 to 500 pM, 1 to 250 pM, 1 to 100 pM. In a suitable embodiment, the PDL1-BD of the invention binds to human PDL1 with a KD of less than approximately 50nM, less than approximately 45nM, less than approximately 40nM, less than approximately 35nM, less than approximately 30nM, less than approximately 25nM, less than 20nM, less than approximately 15nM, less than approximately 10nM, less than approximately 9nM, less than approximately 8nM, less than approximately 7nM, less than approximately 6nM, less than approximately 5nM, less than approximately 4nM, less than approximately 3nM, less than 2nM, less than 1nM, less than 0.5nM, less than 0.25nM, less than 100pM, less than 10pM, or less than 5pM. Suitably, the PDL1-BD of the invention binds to human PDL1 with a KD of less than 1nM. Suitably, the PDL1-BD of the invention binds to human PDL1 with a KD of less than 0.5nM. Suitably, the PDL1-BD of the invention binds to human PDL1 with a KD of less than 100pM. Preferably, the PDL1-BD of the invention binds to human PDL1 with a KD of less than 10pM. More preferably, the PDL1-BD of the invention binds to human PDL1 with a KD of less than 5pM.

Suitably, the PDL1-BD of the invention binds to human PDL1 with a Kₒₙ rate of at least 10³ M⁻¹s⁻¹ or greater, at least 10⁴ M⁻¹s⁻¹ or greater, at least 5x10⁴ M⁻¹s⁻¹ or greater, at least 10⁵ M⁻¹s⁻¹ or greater, at least 5x10⁵ M⁻¹s⁻¹ or greater, at least 10⁶ M⁻¹s⁻¹ or greater, at least 5x10⁶ M⁻¹s⁻¹ or greater, at least 10⁷ M⁻¹s⁻¹ or greater, at least 5x10⁷ M⁻¹s⁻¹ or greater as measured by surface plasmon resonance (SPR). Preferably, the PDL1-BD of the invention binds to human PDL1 with a Kₒₙ rate of at least 10⁵ M⁻¹s⁻¹ or greater, in particular at least 10⁶ M⁻¹s⁻¹ or greater, as measured by SPR.

Suitably, the PDL1-BD of the invention binds to human PDL1 with a K_{off} rate of 10⁻³ s⁻¹ or less, 3x10⁻³ s⁻¹ or less, 5x10⁻³ s⁻¹ or less, 10⁻⁴ s⁻¹ or less, 5x10⁻⁴ s⁻¹ or less, 10⁻⁵ s⁻¹ or less, 5x10⁻⁵ s⁻¹ or less, 10⁻⁶ s⁻¹ or less, or 10⁻⁷ s⁻¹ or less as measured by surface plasmon resonance (SPR). Preferably, the PDL1-BD of the invention binds to human PDL1 with a K_{off} rate of 10⁻³ s⁻¹ or less, 10⁻⁴ s⁻¹ or less, in particular 10⁻⁵ s⁻¹ or less as measured by SPR.

The present invention also provides PDL1-BDs that bind to the same epitope as do the PDL1-BDs listed in Table 2. Additional binding domains can therefore be identified based on their ability to cross-compete (e.g., to competitively inhibit the binding of, in a statistically significant manner) with other binding domains of the invention in PDL1 binding assays. The ability of a test antibody or binding domain to inhibit the binding of PDL1-BDs of the present invention to PDL1 protein demonstrates that the test antibody or binding domains can compete with that binding domain for binding to PDL1; such an antibody or binding domain may, according to non-limiting theory, bind to the same or a related (e.g., a structurally similar or spatially proximal) epitope on PDL1 as the binding domains with which it competes. In a certain embodiment, the antibody or binding domain that binds to the same epitope on PDL1 as the PDL1-BDs of the present invention is a human or humanized monoclonal antibody. Such human or humanized monoclonal antibodies can be prepared and isolated as described herein.

Suitably, the PDL1-BD of the invention comprises a VH CDR having an amino acid sequence of any one of the VH CDRs listed in Table 2. In particular, the PDL1-BD of the invention comprises (or alternatively, consisting of) one, two, three, or more VH CDRs having an amino acid sequence of any of the VH CDRs listed in Table 2.

Suitably, the PDL1-BD of the invention comprises a VL CDR having an amino acid sequence of any one of the VL CDRs listed in Table 2. In particular, the PDL1-BD of the invention comprises (or alternatively, consisting of) one, two, three or more VL CDRs having an amino acid sequence of any of the VL CDRs listed in Table 2.

Other PDL1-BDs of the invention include amino acids that have been mutated, yet have at least 60, 70, 80, 90, 91, 92, 93, 94, 95, 96, 97, 98 or 99 percent identity in the CDR regions with the CDR regions depicted in the sequences described in Table 2. Other PDL1-BDs of the invention include mutant amino acid sequences wherein no more than 1, 2, 3, 4 or 5 amino acids have been mutated in the CDR regions when compared with the CDR regions depicted in the sequence described in Table 2.

The present invention provides a PDL1-BD, which comprises(a) a heavy chain variable region CDR1 comprising an amino acid sequence selected from any one of SEQ ID NOs: 52, 53, 56, 59, 77, 78, 81 and 84; (b) a heavy chain variable region CDR2 comprising an amino acid sequence selected from any of SEQ ID NOs: 54, 57, 60, 79, 82 and 85; (c) a heavy chain variable region CDR3 comprising an amino acid sequence selected from any of SEQ ID NOs: 55, 58, 61, 80, 83 and 86; (d) a light chain variable region CDR1 comprising an amino acid sequence selected from any of SEQ ID NOs: 64, 67, 70, 89, 92 and 95; (e) a light chain variable region CDR2 comprising an amino acid sequence selected from any of SEQ ID NOs: 65, 68, 71, 90, 93 and 96; and (f) a light chain variable region CDR3 comprising an amino acid sequence selected from any of SEQ ID NOs: 66, 69, 72, 91, 94 and 97.

Suitably, the isolated antibody of the invention or antigen-binding fragment thereof comprises: (a) a heavy chain variable region CDR1 comprising an amino acid sequence having at least 60, 70, 80, 90, 91, 92, 93, 94, 95, 96, 97, 98 or 99 percent identity to any one of SEQ ID NOs: 52, 53, 56, 59, 77, 78, 81 and 84; (b) a heavy chain variable region CDR2 comprising an amino acid sequence having at least 60, 70, 80, 90, 91, 92, 93, 94, 95, 96, 97, 98 or 99 percent identity to any of SEQ ID NOs: 54, 57, 60, 79, 82 and 85; (c) a heavy chain variable region CDR3 comprising an amino acid sequence having at least 60, 70, 80, 90, 91, 92, 93, 94, 95, 96, 97, 98 or 99 percent identity to any of SEQ ID NOs: 55, 58, 61, 80, 83 and 86; (d) a light chain variable region CDR1 comprising an amino acid sequence having at least 60, 70, 80, 90, 91, 92, 93, 94, 95, 96, 97, 98 or 99 percent identity to any of SEQ ID NOs: 64, 67, 70, 89, 92 and 95; (e) a light chain variable region CDR2 comprising an amino acid sequence having at least 60, 70, 80, 90, 91, 92, 93, 94, 95, 96, 97, 98 or 99 percent identity to any of SEQ ID NOs: 65, 68, 71, 90, 93 and 96; and (f) a light chain variable region CDR3 comprising an amino acid sequence having at least 60, 70, 80, 90, 91, 92, 93, 94, 95, 96, 97, 98 or 99 percent identity to any of SEQ ID NOs: 66, 69, 72, 91, 94 and 97.

In one embodiment, the PDL1-BD of the invention comprises: (a) HCDR1, HCDR2, and HCDR3 sequences of SEQ ID NOs: 52, 54, and 55, respectively, and LCDR1, LCDR2, and LCDR3 sequences of SEQ ID NOs: 64, 65, and 66, respectively; (b) HCDR1, HCDR2, and HCDR3 sequences of SEQ ID NOs: 53, 54, and 55, respectively, and LCDR1, LCDR2, and LCDR3 sequences of SEQ ID NOs: 64, 65, and 66, respectively; (c) HCDR1, HCDR2, and HCDR3 sequences of SEQ ID NOs: 56, 57, and 58, respectively, and LCDR1, LCDR2, and LCDR3 sequences of SEQ ID NOs: 67, 68, and 69, respectively; (d) HCDR1, HCDR2, and HCDR3 sequences of SEQ ID NOs: 59, 60, and 61, respectively, and LCDR1, LCDR2, and LCDR3 sequences of SEQ ID NOs: 70, 71, and 72, respectively; (e) HCDR1, HCDR2, and HCDR3 sequences of SEQ ID NOs: 77, 79, and 80, respectively, and LCDR1, LCDR2, and LCDR3 sequences of SEQ ID NOs: 89, 90, and 91, respectively; (f) HCDR1, HCDR2, and HCDR3 sequences of SEQ ID NOs: 78, 79, and 80, respectively, and LCDR1, LCDR2, and LCDR3 sequences of SEQ ID NOs: 89, 90, and 91, respectively; (g) HCDR1, HCDR2, and HCDR3 sequences of SEQ ID NOs: 81, 82, and 83, respectively, and LCDR1, LCDR2, and LCDR3 sequences of SEQ ID NOs: 92, 93, and 94, respectively; (h) HCDR1, HCDR2, and HCDR3 sequences of SEQ ID NOs: 84, 85, and 86, respectively, and LCDR1, LCDR2, and LCDR3 sequences of SEQ ID NOs: 95, 96, and 97, respectively.

Suitably, the PDL1-BD of the invention comprises: (a) HCDR1, HCDR2, and HCDR3 sequences having at least 60, 70, 80, 90, 91, 92, 93, 94, 95, 96, 97, 98 or 99 percent identity to SEQ ID NOs: 52, 54, and 55, respectively, and LCDR1, LCDR2, and LCDR3 sequences having at least 60, 70, 80, 90, 91, 92, 93, 94, 95, 96, 97, 98 or 99 percent identity to SEQ ID NOs: 64, 65, and 66,respectively; (b) HCDR1, HCDR2, and HCDR3 sequences having at least 60, 70, 80, 90, 91, 92, 93, 94, 95, 96, 97, 98 or 99 percent identity to SEQ ID NOs: 53, 54, and 55, respectively, and LCDR1, LCDR2, and LCDR3 sequences having at least 60, 70, 80, 90, 91, 92, 93, 94, 95, 96, 97, 98 or 99 percent identity to SEQ ID NOs: 64, 65, and 66, respectively; (c) HCDR1, HCDR2, and HCDR3 sequences having at least 60, 70, 80, 90, 91, 92, 93, 94, 95, 96, 97, 98 or 99 percent identity to SEQ ID NOs: 56, 57, and 58, respectively, and LCDR1, LCDR2, and LCDR3 sequences having at least 60, 70, 80, 90, 91, 92, 93, 94, 95, 96, 97, 98 or 99 percent identity to SEQ ID NOs: 67, 68, and 69, respectively; (d) HCDR1, HCDR2, and HCDR3 sequences having at least 60, 70, 80, 90, 91, 92, 93, 94, 95, 96, 97, 98 or 99 percent identity to SEQ ID NOs: 59, 60, and 61, respectively, and LCDR1, LCDR2, and LCDR3 sequences having at least 60, 70, 80, 90, 91, 92, 93, 94, 95, 96, 97, 98 or 99 percent identity to SEQ ID NOs: 70, 71, and 72, respectively; (e) HCDR1, HCDR2, and HCDR3 sequences having at least 60, 70, 80, 90, 91, 92, 93, 94, 95, 96, 97, 98 or 99 percent identity to SEQ ID NOs: 77, 79, and 80, respectively, and LCDR1, LCDR2, and LCDR3 sequences having at least 60, 70, 80, 90, 91, 92, 93, 94, 95, 96, 97, 98 or 99 percent identity to SEQ ID NOs: 89, 90, and 91, respectively; (f) HCDR1, HCDR2, and HCDR3 sequences having at least 60, 70, 80, 90, 91, 92, 93, 94, 95, 96, 97, 98 or 99 percent identity to SEQ ID NOs: 78, 79, and 80, respectively, and LCDR1, LCDR2, and LCDR3 sequences having at least 60, 70, 80, 90, 91, 92, 93, 94, 95, 96, 97, 98 or 99 percent identity to SEQ ID NOs: 89, 90, and 91, respectively; (g) HCDR1, HCDR2, and HCDR3 sequences having at least 60, 70, 80, 90, 91, 92, 93, 94, 95, 96, 97, 98 or 99 percent identity to SEQ ID NOs: 81, 82, and 83, respectively, and LCDR1, LCDR2, and LCDR3 sequences having at least 60, 70, 80, 90, 91, 92, 93, 94, 95, 96, 97, 98 or 99 percent identity to SEQ ID NOs: 92, 93, and 94, respectively; (h) HCDR1, HCDR2, and HCDR3 sequences having at least 60, 70, 80, 90, 91, 92, 93, 94, 95, 96, 97, 98 or 99 percent identity to SEQ ID NOs: 84, 85, and 86, respectively, and LCDR1, LCDR2, and LCDR3 sequences having at least 60, 70, 80, 90, 91, 92, 93, 94, 95, 96, 97, 98 or 99 percent identity to SEQ ID NOs: 95, 96, and 97, respectively.

In a further embodiment, the PDL1-BD of the invention comprises: (a) a HCDR1 comprising the amino acid sequence of SEQ ID NO: 52 or SEQ ID NO: 53; (b) a HCDR2 comprising the amino acid sequence of SEQ ID NO: 54; (c) a HCDR3 comprising the amino acid sequence of SEQ ID NO: 55; (d) a LCDR1 comprising the amino acid sequence of SEQ ID NOs: 64; (e) a LCDR2 comprising the amino acid sequence of SEQ ID NOs: 65; and (f) a LCDR3 comprising the amino acid sequence of SEQ ID NO: 66.

Suitably, the PDL1-BD of the invention comprises: (a) a HCDR1 comprising the amino acid sequence having at least 60, 70, 80, 90, 91, 92, 93, 94, 95, 96, 97, 98 or 99 percent identity to SEQ ID NO: 52 or SEQ ID NO: 53; (b) a HCDR2 comprising the amino acid sequence having at least 60, 70, 80, 90, 91, 92, 93, 94, 95, 96, 97, 98 or 99 percent identity to SEQ ID NO: 54; (c) a HCDR3 comprising the amino acid sequence having at least 60, 70, 80, 90, 91, 92, 93, 94, 95, 96, 97, 98 or 99 percent identity to SEQ ID NO: 55; (d) a LCDR1 comprising the amino acid sequence having at least 60, 70, 80, 90, 91, 92, 93, 94, 95, 96, 97, 98 or 99 percent identity to SEQ ID NOs: 64; (e) a LCDR2 comprising the amino acid sequence having at least 60, 70, 80, 90, 91, 92, 93, 94, 95, 96, 97, 98 or 99 percent identity to SEQ ID NOs: 65; and (f) a LCDR3 comprising the amino acid sequence having at least 60, 70, 80, 90, 91, 92, 93, 94, 95, 96, 97, 98 or 99 percent identity to SEQ ID NO: 66.

In a further embodiment, the PDL1-BD of the invention comprises: (a) a HCDR1 comprising the amino acid sequence of SEQ ID NO: 77 or SEQ ID NO: 78; (b) a HCDR2 comprising the amino acid sequence of SEQ ID NO: 79; (c) a HCDR3 comprising the amino acid sequence of SEQ ID NO: 80; (d) a LCDR1 comprising the amino acid sequence of SEQ ID NOs: 89; (e) a LCDR2 comprising the amino acid sequence of SEQ ID NOs: 90; and (f) a LCDR3 comprising the amino acid sequence of SEQ ID NO: 91.

Suitably, the PDL1-BD of the invention comprises: (a) a HCDR1 comprising the amino acid sequence having at least 60, 70, 80, 90, 91, 92, 93, 94, 95, 96, 97, 98 or 99 percent identity to SEQ ID NO: 77 or SEQ ID NO: 78; (b) a HCDR2 comprising the amino acid sequence having at least 60, 70, 80, 90, 91, 92, 93, 94, 95, 96, 97, 98 or 99 percent identity to SEQ ID NO: 79; (c) a HCDR3 comprising the amino acid sequence having at least 60, 70, 80, 90, 91, 92, 93, 94, 95, 96, 97, 98 or 99 percent identity to SEQ ID NO: 80; (d) a LCDR1 comprising the amino acid sequence having at least 60, 70, 80, 90, 91, 92, 93, 94, 95, 96, 97, 98 or 99 percent identity to SEQ ID NOs: 89; (e) a LCDR2 comprising the amino acid sequence having at least 60, 70, 80, 90, 91, 92, 93, 94, 95, 96, 97, 98 or 99 percent identity to SEQ ID NOs: 90; and (f) a LCDR3 comprising the amino acid sequence of SEQ ID NO: 91.

In a further embodiment, the present invention provides a PDL1-BD that specifically binds PDL1 (e.g., human PDL1 protein), wherein said binding domain comprises a VH domain and a VL domain.

Suitably, the PDL1-BD of the present invention comprises a VH1A, VH1B, VH3 or VH4. In one embodiment, the PDL1-BD of the present invention comprises VH4 domain. In another embodiment, the PDL1-BD of the present invention comprises VH1A or VH1B domain.

Suitably, the PDL1-BD of the present invention comprises: Vκ frameworks FR1, FR2 and FR3, particularly Vκ1 or Vκ3 frameworks, preferably Vκ1 frameworks FR1 to 3, and a framework FR4, which is selected from a Vκ FR4, particularly Vκ1 FR4, Vκ3 FR4, and a Vλ FR4. Suitable Vλ FR4 are as set forth in SEQ ID NO: 149 to SEQ ID NO: 155. In one embodiment, the PDL1-BD of the present invention comprises Vλ FR4 comprising the amino acid sequence having at least 60, 70, 80, 90 percent identity to comprising an amino acid sequence selected from any of SEQ ID NO: 149 to SEQ ID NO: 155.

Thus, in one embodiment, the PDL1-BD of the present invention comprises:
(i) the HCDR1, HCDR2, HCDR3, LCDR1, LCDR2, and LCDR3 sequences of:
   a. the HCDR1, HCDR2, and HCDR3 sequences of SEQ ID NOs: 52, 54, and 55, respectively, and the LCDR1, LCDR2, and LCDR3 sequences of SEQ ID NOs: 64, 65, and 66, respectively;
   b. the HCDR1, HCDR2, and HCDR3 sequences of SEQ ID NOs: 77, 79, and 80, respectively, and the LCDR1, LCDR2, and LCDR3 sequences of SEQ ID NOs: 89, 90, and 91, respectively; or
   c. the HCDR1, HCDR2, and HCDR3 sequences of SEQ ID NOs: 78, 79, and 80, respectively, and the LCDR1, LCDR2, and LCDR3 sequences of SEQ ID NOs: 89, 90, and 91, respectively;
(ii) a VH3 or VH4 domain, preferably VH4 domain; and
(iii) a VL domain comprising a VL framework comprising Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and a Vλ FR4, preferably Vλ FR4 comprising the amino acid sequence having at least 60, 70, 80, 90 percent identity to comprising an amino acid sequence selected from any of SEQ ID NO: 149 to SEQ ID NO: 155, preferably Vλ FR4 is as set forth in SEQ ID NO: 149 to SEQ ID NO: 155, more preferably Vλ FR4 is as set forth in SEQ ID NO: 149.

In another embodiment, the PDL1-BD of the present invention comprises:
(i) the HCDR1, HCDR2, and HCDR3 sequences of: SEQ ID NOs: 53, 54, and 55, respectively, and the LCDR1, LCDR2, and LCDR3 sequences of SEQ ID NOs: 64, 65, and 66, respectively;
(ii) a VH1A, VH1B, VH3 or VH4 domain, preferably VH1A or VH1B domain; and
(iii) a VL domain comprising a VL framework comprising Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and a Vλ FR4, preferably Vλ FR4 comprising the amino acid sequence having at least 60, 70, 80, 90 percent identity to comprising an amino acid sequence selected from any of SEQ ID NO: 149 to SEQ ID NO: 155, preferably Vλ FR4 comprising an amino acid sequence selected from any of SEQ ID NO: 149 to SEQ ID NO: 155, more preferably Vλ FR4 is as set forth in SEQ ID NO: 149.

In one embodiment, the PDL1-BD of the present invention comprises a VL comprising:
(i) CDR domains CDR1, CDR2 and CDR3;
(ii) human Vκ framework regions FR1 to FR3, particularly human Vκ1 framework regions FR1 to FR3;
(iii) FR4, which is selected from (a) a human Vλ germ line sequence for FR4, particularly a Vλ germ line sequence selected from the list of: SEQ ID NO: 149 to 155, preferably Vλ FR4 is as set forth in SEQ ID NO: 149; and (b) a Vλ-based sequence, which has one or two mutations, particularly one mutation, compared to the closest human Vλ germ line sequence for FR4 comprising an amino acid sequence selected from any of SEQ ID NO: 149 to SEQ ID NO: 155, preferably SEQ ID NO: 149.

The PDL1-BD of the invention comprises a VH domain listed in Table 2. Suitably, the PDL1-BD of the invention comprises (or alternatively, consisting of) a VH amino acid sequence listed in Table 2, wherein no more than about 10 amino acids in a framework sequence (for example, a sequence which is not a CDR) have been mutated (wherein a mutation is, as various non- limiting examples, an addition, substitution or deletion). Suitably, the PDL1-BD of the invention comprises (or alternatively, consisting of) a VH amino acid sequence listed in Table 2, wherein no more than about 20 amino acids in a framework sequence (for example, a sequence which is not a CDR) have been mutated (wherein a mutation is, as various non-limiting examples, an addition, substitution or deletion). Other PDL1-BDs of the invention include amino acids that have been mutated, yet have at least 60, 70, 80, 90, 91, 92, 93, 94, 95, 96, 97, 98 or 99 percent identity in the VH regions with the VH regions depicted in the sequences described in Table 2.

The PDL1-BD of the invention comprises a VL domain listed in Table 2. Suitably, the PDL1-BD of the invention comprises (or alternatively, consisting of) a VL amino acid sequence listed in Table 2, wherein no more than about 10 amino acids in a framework sequence (for example, a sequence which is not a CDR) have been mutated (wherein a mutation is, as various non-limiting examples, an addition, substitution or deletion). Suitably, the PDL1-BD of the invention comprises (or alternatively, consisting of) a VL amino acid sequence listed in Table 2, wherein no more than about 20 amino acids in a framework sequence (for example, a sequence which is not a CDR) have been mutated (wherein a mutation is, as various non-limiting examples, an addition, substitution or deletion). Other PDL1-BDs of the invention include amino acids that have been mutated, yet have at least 60, 70, 80, 90, 91, 92, 93, 94, 95, 96, 97, 98 or 99 percent identity in the VL regions with the VL regions depicted in the sequences described in Table 2.

Suitably, the PDL1-BD of the invention comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 62, 63, 87 and 88; and a light chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 73, 98 and 99.

In one embodiment, the PDL1-BD of the invention comprises: a heavy chain variable region comprising an amino acid sequence selected from any of SEQ ID NOs: 62, 63, 87 and 88; and a light chain variable region comprising an amino acid sequence selected from any of SEQ ID NOs: 73, 98 and 99.

In a further embodiment, the PDL1-BD of the invention comprises: (a) a VH sequence of SEQ ID NO: 62 and a VL sequence of SEQ ID NO: 73; (b) a VH sequence of SEQ ID NO: 63 and a VL sequence of SEQ ID NO: 73; (c) a VH sequence of SEQ ID NO: 87 and a VL sequence of SEQ ID NO: 98; or (d) a VH sequence of SEQ ID NO: 88 and a VL sequence of SEQ ID NO: 99.

In one embodiment, the PDL1-BD of the present invention comprises:
(a) HCDR1, HCDR2, and HCDR3 sequences of SEQ ID NOs: 52, 54, and 55, respectively, and the LCDR1, LCDR2, and LCDR3 sequences of SEQ ID NOs: 64, 65, and 66, respectively, a VH sequence at least 60, 70, 80, 90, 91, 92, 93, 94, 95, 96, 97, 98 or 99 percent identical to SEQ ID NO: 62, and a VL sequence at least 60, 70, 80, 90, 91, 92, 93, 94, 95, 96, 97, 98 or 99 percent identical to SEQ ID NO: 73;
(b) the HCDR1, HCDR2, and HCDR3 sequences of: SEQ ID NOs: 53, 54, and 55, respectively, and the LCDR1, LCDR2, and LCDR3 sequences of SEQ ID NOs: 64, 65, and 66, respectively, a VH sequence at least 60, 70, 80, 90, 91, 92, 93, 94, 95, 96, 97, 98 or 99 percent identical to SEQ ID NO: 63, and a VL sequence at least 60, 70, 80, 90, 91, 92, 93, 94, 95, 96, 97, 98 or 99 percent identical to SEQ ID NO: 73;
(c) HCDR1, HCDR2, and HCDR3 sequences of SEQ ID NOs: 77, 79, and 80, respectively, and the LCDR1, LCDR2, and LCDR3 sequences of SEQ ID NOs: 89, 90, and 91, respectively, a VH sequence at least 60, 70, 80, 90, 91, 92, 93, 94, 95, 96, 97, 98 or 99 percent identical to SEQ ID NO: 87, and a VL sequence at least 60, 70, 80, 90, 91, 92, 93, 94, 95, 96, 97, 98 or 99 percent identical to SEQ ID NO: 98; or
(d) HCDR1, HCDR2, and HCDR3 sequences of SEQ ID NOs: 78, 79, and 80, respectively, and the LCDR1, LCDR2, and LCDR3 sequences of SEQ ID NOs: 89, 90, and 91, respectively, a VH sequence at least 60, 70, 80, 90, 91, 92, 93, 94, 95, 96, 97, 98 or 99 percent identical to SEQ ID NO: 88, and a VL sequence at least 60, 70, 80, 90, 91, 92, 93, 94, 95, 96, 97, 98 or 99 percent identical to SEQ ID NO: 99.

In one embodiment, the PDL1-BD of the present invention comprises:
(a) HCDR1, HCDR2, and HCDR3 sequences of SEQ ID NOs: 52, 54, and 55, respectively, and the LCDR1, LCDR2, and LCDR3 sequences of SEQ ID NOs: 64, 65, and 66, respectively, a VH sequence of SEQ ID NO: 62 and a VL sequence of SEQ ID NO: 73;
(b) the HCDR1, HCDR2, and HCDR3 sequences of: SEQ ID NOs: 53, 54, and 55, respectively, and the LCDR1, LCDR2, and LCDR3 sequences of SEQ ID NOs: 64, 65, and 66, respectively, a VH sequence of SEQ ID NO: 63 and a VL sequence of SEQ ID NO: 73;
(c) HCDR1, HCDR2, and HCDR3 sequences of SEQ ID NOs: 77, 79, and 80, respectively, and the LCDR1, LCDR2, and LCDR3 sequences of SEQ ID NOs: 89, 90, and 91, respectively, a VH sequence of SEQ ID NO: 87 and a VL sequence of SEQ ID NO: 98; or
(d) HCDR1, HCDR2, and HCDR3 sequences of SEQ ID NOs: 78, 79, and 80, respectively, and the LCDR1, LCDR2, and LCDR3 sequences of SEQ ID NOs: 89, 90, and 91, respectively, a VH sequence of SEQ ID NO: 88 and a VL sequence of SEQ ID NO: 99.

In one embodiment, a PDL1-BD that specifically binds to PDL1 is a binding domain that is described in Table 2. In one embodiment, the PDL1-BD of the invention that specifically binds to PDL1 is as set forth in SEQ ID NO: 75, SEQ ID NO: 76, SEQ ID NO: 101, or SEQ ID NO: 102. In one embodiment, the PDL1-BD of the invention that specifically binds to PDL1 is as set forth in SEQ ID NO: 75 or SEQ ID NO: 76. In one embodiment, the PDL1-BD of the invention that specifically binds to PDL1 is as set forth in SEQ ID NO: 101 or SEQ ID NO: 102.

Other PDL1-BDs of the invention include those wherein the amino acids or nucleic acids encoding the amino acids have been mutated, yet have at least 60, 70, 80, 90 or 95 percent identity to the sequences described in Table 2. In one embodiment, it includes mutant amino acid sequences wherein no more than 1, 2, 3, 4 or 5 amino acids have been mutated in the variable regions when compared with the variable regions depicted in the sequence described in Table 2, while retaining substantially the same activity.

Given that each of these binding domains can bind to PDL1 and that antigen-binding specificity is provided primarily by the CDR1, 2 and 3 regions, the VH CDR1, 2 and 3 sequences and VL CDR1, 2 and 3 sequences can be "mixed and matched". Such "mixed and matched" PDL1-BDs can be tested using the binding assays known in the art and those described in the Examples (e.g., ELISAs). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s).

In yet another embodiment, the PDL1-BD of the invention comprises amino acid sequences that are homologous to the sequences described in Table 2, and said BD binds to PDL1, and retains the desired functional properties of those antibodies described in Table 2.

For example, the invention provides a PDL1-BD comprising a heavy chain variable region and a light chain variable region, wherein the heavy chain variable region comprises an amino acid sequence that is at least 80 percent, at least 90 percent, or at least 95 percent identical to an amino acid sequence selected from the group consisting of SEQ ID NOs: 62, 63, 87 and 88; the light chain variable region comprises an amino acid sequence that is at least 80 percent, at least 90 percent, or at least 95 percent identical to an amino acid sequence selected from the group consisting of SEQ ID NOs: 73, 98 and 99.

In one embodiment, the VH and/or VL amino acid sequences may be 50 percent, 60 percent, 70 percent, 80 percent, 90 percent, 95 percent, 96 percent, 97 percent, 98 percent or 99 percent identical to the sequences set forth in Table 2. In one embodiment, the VH and/or VL amino acid sequences may be identical except an amino acid substitution in no more than 1, 2, 3, 4 or 5 amino acid positions.

In one embodiment, the invention provides a PDL1-BD comprising a heavy chain variable region comprising CDR1, CDR2, and CDR3 sequences and a light chain variable region comprising CDR1, CDR2, and CDR3 sequences, wherein one or more of these CDR sequences have specified amino acid sequences based on the PDL1-BDs described herein or conservative modifications thereof, and wherein the PDL1-BDs retain the desired functional properties of the PDL1-BDs of the invention.

Accordingly, the invention provides a PDL1-BD comprising a heavy chain variable region comprising CDR1, CDR2, and CDR3 sequences and a light chain variable region comprising CDR1, CDR2, and CDR3 sequences, wherein: the heavy chain variable region CDR1 comprises an amino acid sequence selected from any of SEQ ID NOs: 52, 53, 56, 59, 77, 78, 81 and 84, or conservative variants thereof; the heavy chain variable region CDR2 comprises an amino acid sequence selected from any of SEQ ID NOs: 54, 57, 60, 79, 82 and 85, or conservative variants thereof; the heavy chain variable region CDR3 comprises an amino acid sequence selected from any of SEQ ID NOs: 55, 58, 61, 80, 83 and 86, or conservative variants thereof; the light chain variable region CDR1 comprises an amino acid sequence selected from any of SEQ ID NOs: 64, 67, 70, 89, 92 and 95, or conservative variants thereof; the light chain variable region CDR2 comprises an amino acid sequence selected from any of SEQ ID NOs: 65, 68, 71, 90, 93 and 96, or conservative variants thereof; and the light chain variable region CDR3 comprises an amino acid sequence selected from any of SEQ ID NOs: 66, 69, 72, 91, 94 and 97, or conservative variants thereof; wherein the PDL1-BD specifically binds to PDL1 and is capable of blocking PD-1/PDL1 interaction.

In one embodiment, a PDL1-BD of the invention is optimized for expression in a mammalian cell has a heavy chain variable region and a light chain variable region, wherein one or more of these sequences have specified amino acid sequences based on the binding domains described herein or conservative modifications thereof, and wherein the binding domains retain the desired functional properties of the PDL1-BD of the invention. Accordingly, the invention provides a PDL1-BD optimized for expression in a mammalian cell comprising a heavy chain variable region and a light chain variable region wherein: the heavy chain variable region comprises an amino acid sequence selected from any of SEQ ID NOs: 62, 63, 87 and 88, and conservative modifications thereof; and the light chain variable region comprises an amino acid sequence selected from any of SEQ ID NOs: 73, 98 and 99, and conservative modifications thereof; wherein the PDL1-BD specifically binds to PDL1 and is capable of blocking PD-1/PDL1 interaction.

In one embodiment, an "affinity-matured" PDL1-BD of the invention comprises: a VH4 comprising V2S; V25A; I44V; G56A; V82K; F89V; Y105F mutations, in particular comprising an amino acid sequence according to SEQ ID NO: 88; and a VL comprising I2F; M4L; A51P mutations, in particular comprising an amino acid sequence according to SEQ ID NO: 99.

A PDL1-BD of the invention further can be prepared using an antibody or binding domain having one or more of the VH and/or VL sequences shown herein as starting material to engineer a modified binding domain, which modified binding domain may have altered properties from the starting antibody or binding domain. A binding domain can be engineered by modifying one or more residues within one or both variable regions (i.e., VH and/or VL), for example within one or more CDR regions and/or within one or more framework regions.

Suitably, the PDL1-BD of the invention is selected from the group consisting of: Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).

Suitably, the PDL1-BD of the invention is a scFv antibody fragment. In particular embodiments, said PDL1-BD is a scFv comprising the linker according to SEQ ID NO: 74. In a further embodiment, the PDL1-BD of the invention is a single-chain variable fragment (scFv) as shown in SEQ ID NO: 75, SEQ ID NO: 76, SEQ ID NO: 101 and SEQ ID NO: 102. In one embodiment, the PDL1-BD of the invention is a scFv as shown in SEQ ID NO: 75 or SEQ ID NO: 76. In one embodiment, the PDL1-BD of the invention is a scFv as shown in SEQ ID NO: 101 or SEQ ID NO: 102.

Other suitable PDL1-BDs comprise or are derived from an antibody selected from the group consisting of: (i) avelumab (MSB0010718C; human IgG1 anti-PDLl monoclonal antibody; Merck-Serono; described in WO2013/079174, which is hereby incorporated into the present application by reference in its entirety); (ii) atezolizumab (MPDL3280A, RG7446; human IgG anti-PDLl monoclonal antibody; Hoffmann-La Roche); (iii) MDX-1105 (BMS-936559; human IgG4 anti-PDL1 monoclonal antibody; Bristol-Myers Squibb; described in WO2007/005874, which is hereby incorporated into the present application by reference in its entirety); (iv) Durvalumab (MEDI4736; humanized IgG1 anti-PDL1 monoclonal antibody; AstraZeneca; described in WO2011/066389 and US2013/034559, which are hereby incorporated into the present application by reference in their entirety); (v) KN035 (anti-PDL1 monoclonal antibody; 3D Medicines); (vi) LY3300054 (anti-PDL1 monoclonal antibody; Eli Lilly); and (vii) YW243.55.S70 (described in WO2010/077634 and U.S. Pat. No. 8,217,149, the entirety of each of which is incorporated herein by reference).

The inventors have further surprisingly found that at certain ratios between affinities or avidities of the CD137-BD(s) and PDL1-BD(s), the concentration window of maximal activity is extended, which is predicted to be beneficial for therapeutic applications and allows higher flexibility in dosing the multispecific antibody of the invention or pharmaceutical compositions comprising thereof. Thus, in one embodiment, the multispecific antibody of the present invention comprises at least one CD137-BD and at least one PDL1-BD, the affinity of said CD137-BD relative to the affinity of said PDL1-BD is at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times weaker. In other words, the multispecific antibody of the present invention comprises at least one CD137-BD and at least one PDL1-BD, wherein said CD137-BD binds to human CD137 with a dissociation constant (KD) of at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times higher relative to a dissociation constant (KD) of binding to human PDL1 of said PDL1-BD. In a further embodiment, said CD137-BD binds to human CD137 with a dissociation constant (KD) between 10nM and 10pM, preferably between 10nM and 0.1nM, more preferably between 5nM and 1nM. In another embodiment, the multispecific antibody of the present invention comprises at least one CD137-BD and at least one PDL1-BD, wherein said CD137-BD has the avidity of at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times weaker (lower) relative to the avidity of said PDL1-BD.

In one embodiment, the multispecific antibody of the present invention comprises at least one CD137-BD and at least one PDL1-BD, wherein the affinity of said PDL1-BD relative to the affinity of said CD137-BD is at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times stronger. In one embodiment, the multispecific antibody of the present invention comprises at least one CD137-BD and at least one PDL1-BD, wherein said PDL1-BD binds to human PDL1 with a dissociation constant (KD) of at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times lower relative to a dissociation constant (KD) of binding to human CD137 of said CD137-BD. In another embodiment, the multispecific antibody of the present invention comprises at least one CD137-BD and at least one PDL1-BD, wherein said PDL1-BD has the avidity of at least at least 0.2 times, e.g., at least 0.5, at least 1.0, at least 5.0, preferably at least 10 times, e.g., at least 50, at least 100, at least 200, at least 300, at least 400, more preferably at least 500 times, e.g., at least 600, at least 700, at least 800, at least 900, at least 1000 times stronger (higher) relative to the avidity of said CD137-BD.

In one embodiment, the multispecific antibody of the present invention comprises: (i) at least one CD137-BD, wherein said CD137-BD is a scFv comprising an amino acid sequence that is at least 80 percent, at least 90 percent, or at least 95 percent identical to an amino acid sequence of SEQ ID NO: 27; and (ii) at least one PDL1-BD, wherein said PDL1-BD is a scFv comprising an amino acid sequence that is at least 80 percent, at least 90 percent, or at least 95 percent identical to an amino acid sequence selected from the list consisting of SEQ ID NO: 75, 76, 101 and 102. In a further embodiment, the multispecific antibody of the present invention comprises: (i) at least one CD137-BD, wherein said CD137-BD is a scFv comprising an amino acid sequence of SEQ ID NO: 27; and (ii) at least one PDL1-BD, wherein said PDL1-BD is a scFv comprising an amino acid sequence selected from the list consisting of SEQ ID NO: 75, 76, 101 and 102.

In one embodiment, the multispecific antibody of the present invention comprises: (i) at least one CD137-BD, wherein said CD137-BD is a Fv comprising an amino acid sequence that is at least 80 percent, at least 90 percent, or at least 95 percent identical to an amino acid sequence of SEQ ID NO: 28; and (ii) at least one PDL1-BD, wherein said PDL1-BD is a Fv comprising an amino acid sequence that is at least 80 percent, at least 90 percent, or at least 95 percent identical to an amino acid sequence selected from the list consisting of SEQ ID NO: 75, 76, 101 and 102. In a further embodiment, the multispecific antibody of the present invention comprises: (i) at least one CD137-BD, wherein said CD137-BD is a Fv comprising an amino acid sequence of SEQ ID NO: 28; and (ii) at least one PDL1-BD, wherein said PDL1-BD is a Fv comprising an amino acid sequence selected from the list consisting of SEQ ID NO: 75, 76, 101 and 102.

In one embodiment, the multispecific antibody of the present invention comprises: (i) at least one CD137-BD, wherein said CD137-BD is a Fv comprising an amino acid sequence that is at least 80 percent, at least 90 percent, or at least 95 percent identical to an amino acid sequence of SEQ ID NO: 29; and (ii) at least one PDL1-BD, wherein said PDL1-BD is a Fv comprising an amino acid sequence that is at least 80 percent, at least 90 percent, or at least 95 percent identical to an amino acid sequence selected from the list consisting of SEQ ID NO: 75, 76, 101 and 102. In a further embodiment, the multispecific antibody of the present invention comprises: (i) at least one CD137-BD, wherein said CD137-BD is a Fv comprising an amino acid sequence of SEQ ID NO: 29; and (ii) at least one PDL1-BD, wherein said PDL1-BD is a Fv comprising an amino acid sequence selected from the list consisting of SEQ ID NO: 75, 76, 101 and 102.

Suitably, the multispecific antibody of the invention has two different specificities (PDL1 and CD137). Suitably, the multispecific antibody of the invention is a bispecific antibody. The multispecific antibody of the present invention may comprise a further specificity (trispecific) or specificities (tetraspecific, pentaspecific or hexaspecific antibody). In one embodiment, the multispecific antibody is trispecific.

In one embodiment, the multispecific antibody of the invention comprises an immunoglobulin Fc region polypeptide. The term "Fc region" herein is used to define a C-terminal region of an immunoglobulin heavy chain, including native-sequence Fc regions and variant Fc regions. Suitable native-sequence Fc regions include human lgG1, lgG2 (lgG2A, JgG2B), lgG3 and lgG4. "Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcyRII, and FcγRIII subclasses, including allelic variants and alternatively spliced forms of these receptors, FcγRII receptors include FcγRIIA (an "activating receptor") and FcγRI IB (an "inhibiting receptor"), which have similar amino acid sequences that differ primarily in the cytoplasmic domains thereof. Activating receptor FcγRIIA contains an immunoreceptor tyrosine -based activation motif (ITAM) in its cytoplasmic domain. Inhibiting receptor FcyRIIB contains an immunoreceptor tyrosine-based inhibition motif (ITIM) in its cytoplasmic domain, (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al, J. Immunol. 117: 587 (1976) and Kim et al., J. Immunol. 24: 249 (1994). Methods of measuring binding to FcRn are known (see, e.g., Ghetie and Ward, Immunol. Today 18: (12): 592-8 (1997); Ghetie et al, Nature Biotechnology 15 (7): 637-40 (1997); Hinton et al, J. Biol. Chem. TJI (8): 6213-6 (2004); WO 2004/92219 (Hinton et al). Binding to FcRn in vivo and serum half-life of human FcRn high-affinity binding polypeptides can be assayed, e.g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, e.g., Shields et al, J. Biol. Chem. 9(2): 6591-6604 (2001).

In another embodiment, the antibody of the invention does not comprise an immunoglobulin Fc region polypeptide.

In order to increase the number of specificities/functionalities at the same or lower molecular weight, it is advantageous to use antibodies comprising antibody fragments, such as Fv, Fab, Fab' and F(ab')2 fragments and other antibody fragments. These smaller molecules retain the antigen binding activity of the whole antibody and can also exhibit improved tissue penetration and pharmacokinetic properties in comparison to the whole immunoglobulin molecules. Whilst such fragments appear to exhibit a number of advantages over whole immunoglobulins, they also suffer from an increased rate of clearance from serum since they lack the Fc domain that imparts a long half-life in vivo (Medasan et al., 1997, J. Immunol. 158:2211-2217). Molecules with lower molecular weights penetrate more efficiently into target tissues (e.g. solid cancers) and thus hold the promise for improved efficacy at the same or lower dose.

The inventors have surprisingly found that an addition of human serum albumin binding domain (HSA-BD) to the multispecific antibody of the invention comprising (a) at least one CD137-BD, and (b) at least one PDL1-BD has the following beneficial effects:
(i) a serum half-life of the multispecific antibody of the invention comprising at least one human serum albumin domain is comparable to that of an IgG;
(ii) addition of a human serum albumin binding domain to the multispecific antibody of the invention is compatible with the functionalities of other binding domains, e.g., the PDL1-BD retains its blocking activity and CD137-BD retains its ability to activate CD137 signaling upon clustering;
(iii) even though, human serum albumin binding domain increases the EC50 of the multispecific antibody of the invention to activate CD137, it unexpectedly improves the maximal effect size, e.g., the maximal activation of CD137 signaling is significantly higher.

Suitably, the multispecific antibody of the present invention may comprise a further binding domain having a specificity to human serum albumin. In one embodiment, the multispecific antibody comprises: (i) at least one CD137-BD; (ii) at least one PDL1-BD; and (iii) at least one HSA-BD. Suitably, the multispecific antibody of the present invention comprises: (i) one CD137-BD; (ii) at least one PDL1-BD, preferably one PDL1-BD or two PDL1-BDs, more preferably one PDL1-BD; and (iii) at least one HSA-BD, preferably one HSA-BD.

The term "HSA" refers in particular to human serum albumin with UniProt ID number P02768, or a variant thereof. Human Serum Albumin (HSA) is 66.4 kDa abundant protein in human serum (50% of total protein) composing of 585 amino acids (Sugio, Protein Eng, Vol. 12, 1999, 439-446). Multifunctional HSA protein is associated with its structure that allowed to bind and transport a number of metabolizes such as fatty acids, metal ions, bilirubin and some drugs (Fanali, Molecular Aspects of Medicine, Vol. 33, 2012, 209-290). HSA concentration in serum is around 3.5-5 g/dL. Albumin binding antibodies and fragments thereof may be used for example, for extending the in vivo serum half-life of drugs or proteins conjugated thereto.

In some embodiments, the HSA-BD is derived from a monoclonal antibody or antibody fragment.

Suitable HSA-BDs for use in the multispecific antibody of the invention are binding domains provided in the present disclosure. The HSA-BDs of the invention include, but are not limited to, the humanized monoclonal antibodies whose sequences are listed in Table 3.

The HSA-BDs of the invention specifically bind to human serum albumin. The HSA-BDs of the invention comprise a VH CDR having an amino acid sequence of any one of the VH CDRs listed in Table 3. In particular, the invention provides HSA-BDs comprising (or alternatively, consisting of) one, two, three, or more VH CDRs having an amino acid sequence of any of the VH CDRs listed in Table 3.

The invention also provides HSA-BDs comprising a VL CDR having an amino acid sequence of any one of the VL CDRs listed in Table 3. In particular, the invention provides HSA-BDs comprising (or alternatively, consisting of) one, two, three or more VL CDRs having an amino acid sequence of any of the VL CDRs listed in Table 3.

Other HSA-BDs of the invention include amino acids that have been mutated, yet have at least 60, 70, 80, 90, 91, 92, 93, 94, 95, 96, 97, 98 or 99 percent identity in the CDR regions with the CDR regions depicted in the sequences described in Table 3. Other HSA-BDs of the invention include mutant amino acid sequences wherein no more than 1, 2, 3, 4 or 5 amino acids have been mutated in the CDR regions when compared with the CDR regions depicted in the sequence described in Table 3.

The HSA-BD of the present invention comprises: (a) a heavy chain variable region CDR1 comprising an amino acid sequence selected from any one of SEQ ID NOs: 103, 106, 109, 125, 128 and 131; (b) a heavy chain variable region CDR2 comprising an amino acid sequence selected from any of SEQ ID NOs: 104, 107, 110, 126, 129 and 132; (c) a heavy chain variable region CDR3 comprising an amino acid sequence selected from any of SEQ ID NOs: 105, 108, 111, 127, 130 and 133; (d) a light chain variable region CDR1 comprising an amino acid sequence selected from any of SEQ ID NOs: 113, 116, 119, 135, 138 and 141; (e) a light chain variable region CDR2 comprising an amino acid sequence selected from any of SEQ ID NOs: 114, 117, 120, 136, 139 and 142; and (f) a light chain variable region CDR3 comprising an amino acid sequence selected from any of SEQ ID NOs: 115, 118, 121, 137, 140 and 143. Suitably, the HSA-BD of the present invention comprises: (a) a heavy chain variable region CDR1 comprising an amino acid sequence having at least 60, 70, 80, 90, 91, 92, 93, 94, 95, 96, 97, 98 or 99 percent identity to any one of SEQ ID NOs: 103, 106, 109, 125, 128 and 131; (b) a heavy chain variable region CDR2 comprising an amino acid sequence having at least 60, 70, 80, 90, 91, 92, 93, 94, 95, 96, 97, 98 or 99 percent identity to any of SEQ ID NOs: 104, 107, 110, 126, 129 and 132; (c) a heavy chain variable region CDR3 comprising an amino acid sequence having at least 60, 70, 80, 90, 91, 92, 93, 94, 95, 96, 97, 98 or 99 percent identity to any of SEQ ID NOs: 105, 108, 111, 127, 130 and 133; (d) a light chain variable region CDR1 comprising an amino acid sequence selected having at least 60, 70, 80, 90, 91, 92, 93, 94, 95, 96, 97, 98 or 99 percent identity to any of SEQ ID NOs: 113, 116, 119, 135, 138 and 141; (e) a light chain variable region CDR2 comprising an amino acid sequence having at least 60, 70, 80, 90, 91, 92, 93, 94, 95, 96, 97, 98 or 99 percent identity to any of SEQ ID NOs: 114, 117, 120, 136, 139 and 142; and (f) a light chain variable region CDR3 comprising an amino acid sequence having at least 60, 70, 80, 90, 91, 92, 93, 94, 95, 96, 97, 98 or 99 percent identity to any of SEQ ID NOs: 115, 118, 121, 137, 140 and 143.

In one embodiment, the HSA-BD of the invention comprises: (a) HCDR1, HCDR2, and HCDR3 sequences of SEQ ID NOs: 103, 104, and 105, respectively, and LCDR1, LCDR2, and LCDR3 sequences of SEQ ID NOs: 113, 114, and 115, respectively; or (b) HCDR1, HCDR2, and HCDR3 sequences of SEQ ID NOs: 125, 126, and 127, respectively, and LCDR1, LCDR2, and LCDR3 sequences of SEQ ID NOs: 135, 136, and 137, respectively. Suitably, the HSA-BD of the invention comprises: (a) HCDR1, HCDR2, and HCDR3 sequences having at least 60, 70, 80, 90, 91, 92, 93, 94, 95, 96, 97, 98 or 99 percent identity to SEQ ID NOs: 103, 104, and 105, respectively, and LCDR1, LCDR2, and LCDR3 sequences having at least 60, 70, 80, 90, 91, 92, 93, 94, 95, 96, 97, 98 or 99 percent identity to SEQ ID NOs: 113, 114, and 115, respectively; or (b) HCDR1, HCDR2, and HCDR3 sequences having at least 60, 70, 80, 90, 91, 92, 93, 94, 95, 96, 97, 98 or 99 percent identity to SEQ ID NOs: 125, 126, and 127, respectively, and LCDR1, LCDR2, and LCDR3 sequences having at least 60, 70, 80, 90, 91, 92, 93, 94, 95, 96, 97, 98 or 99 percent identity to SEQ ID NOs: 135, 136, and 137, respectively.

In a further embodiment, the invention provides a HSA-BD that specifically binds human serum albumin, wherein said binding domain comprises a VH domain and a VL domain.

Suitably, the HSA-BD of the invention comprises a VH3 or VH4. In one embodiment, the HSA-BD of the invention comprises VH3 domain.

Suitably, the HSA-BD of the invention comprises: Vκ frameworks FR1, FR2 and FR3, particularly Vκ1 or Vκ3 frameworks, preferably Vκ1 frameworks FR1 to 3, and a framework FR4, which is selected from a Vκ FR4, particularly Vκ1 FR4, Vκ3 FR4, and a Vλ FR4. Suitable Vλ FR4 are as set forth in SEQ ID NO: 149 to SEQ ID NO: 155. In one embodiment, the HSA-BD of the present invention comprises Vλ FR4 comprising the amino acid sequence having at least 60, 70, 80, 90 percent identity to an amino acid sequence selected from any of SEQ ID NO: 149 to SEQ ID NO: 155, preferably to SEQ ID NO: 149.

Thus, in one embodiment, the HSA-BD of the present invention comprises:
(i) the HCDR1, HCDR2, HCDR3, LCDR1, LCDR2, and LCDR3 sequences of:
   (a) HCDR1, HCDR2, and HCDR3 sequences of SEQ ID NOs: 103, 104, and 105, respectively, and LCDR1, LCDR2, and LCDR3 sequences of SEQ ID NOs: 113, 114, and 115, respectively; or
   (b) HCDR1, HCDR2, and HCDR3 sequences of SEQ ID NOs: 125, 126, and 127, respectively, and LCDR1, LCDR2, and LCDR3 sequences of SEQ ID NOs: 135, 136, and 137, respectively;
(ii) a VH3 or VH4 domain, preferably VH3 domain; and
(iii) a VL domain comprising a VL framework comprising Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and a Vλ FR4, preferably Vλ FR4 comprising the amino acid sequence having at least 60, 70, 80, 90 percent identity to comprising an amino acid sequence selected from any of SEQ ID NO: 149 to SEQ ID NO: 155, preferably Vλ FR4 is as set forth in an amino acid sequence selected from any of SEQ ID NO: 149 to SEQ ID NO: 155, more preferably Vλ FR4 is as set forth in SEQ ID NO: 149.

In one embodiment, the HSA-BD of the present invention comprises a VL comprising:
(iv) CDR domains CDR1, CDR2 and CDR3;
(v) human Vκ framework regions FR1 to FR3, particularly human Vκ1 framework regions FR1 to FR3;
(vi) FR4, which is selected from (a) a human Vλ germ line sequence for FR4, particularly a Vλ germ line sequence selected from the list of: SEQ ID NO: 149 to 155, preferably SEQ ID NO: 149; and (b) a Vλ-based sequence, which has one or two mutations, particularly one mutation, compared to the closest human Vλ germ line sequence for FR4 comprising an amino acid sequence selected from any of SEQ ID NO: 149 to SEQ ID NO: 155, preferably SEQ ID NO: 149.

The HSA-BD of the invention comprises a VH domain listed in Table 3. Suitably, the HSA-BD of the invention comprises (or alternatively, consisting of) a VH amino acid sequence listed in Table 3, wherein no more than about 10 amino acids in a framework sequence (for example, a sequence which is not a CDR) have been mutated (wherein a mutation is, as various non-limiting examples, an addition, substitution or deletion). Suitably, the HSA-BD of the present invention comprises (or alternatively, consisting of) a VH amino acid sequence listed in Table 3, wherein no more than about 20 amino acids in a framework sequence (for example, a sequence which is not a CDR) have been mutated (wherein a mutation is, as various non-limiting examples, an addition, substitution or deletion). Other HSA-BDs of the invention include amino acids that have been mutated, yet have at least 60, 70, 80, 90, 91, 92, 93, 94, 95, 96, 97, 98 or 99 percent identity in the VH regions with the VH regions depicted in the sequences described in Table 3.

The HSA-BD of the invention comprises a VL domain listed in Table 3. Suitably, the HSA-BD of the invention comprises (or alternatively, consisting of) a VL amino acid sequence listed in Table 3, wherein no more than about 10 amino acids in a framework sequence (for example, a sequence which is not a CDR) have been mutated (wherein a mutation is, as various non-limiting examples, an addition, substitution or deletion). Suitably, the HSA-BD of the invention comprises (or alternatively, consisting of) a VL amino acid sequence listed in Table 3, wherein no more than about 20 amino acids in a framework sequence (for example, a sequence which is not a CDR) have been mutated (wherein a mutation is, as various non-limiting examples, an addition, substitution or deletion). Other HSA-BDs of the invention include amino acids that have been mutated, yet have at least 60, 70, 80, 90, 91, 92, 93, 94, 95, 96, 97, 98 or 99 percent identity in the VL regions with the VL regions depicted in the sequences described in Table 3.

Suitably, the HSA-BD of the invention comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 112 and 134; and a light chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 122 and 144.

In one embodiment, the HSA-BD of the invention comprises: a heavy chain variable region comprising an amino acid sequence selected from any of SEQ ID NOs: 112 and 134; and a light chain variable region comprising an amino acid sequence selected from any of SEQ ID NOs: 122 and 144.

In a further embodiment, the HSA-BD of the invention comprises: (a) a VH sequence of SEQ ID NO: 112 and a VL sequence of SEQ ID NO: 122; or (b) a VH sequence of SEQ ID NO: 134 and a VL sequence of SEQ ID NO: 144.

In one embodiment, the HSA-BD of the invention comprises:
(a) HCDR1, HCDR2, and HCDR3 sequences of SEQ ID NOs: 103, 104, and 105, respectively, and LCDR1, LCDR2, and LCDR3 sequences of SEQ ID NOs: 113, 114, and 115, respectively, a VH sequence having at least 60, 70, 80, 90, 91, 92, 93, 94, 95, 96, 97, 98 or 99 percent identity to SEQ ID NO: 112, and a VL sequence having at least 60, 70, 80, 90, 91, 92, 93, 94, 95, 96, 97, 98 or 99 percent identity to SEQ ID NO: 122;
(b) HCDR1, HCDR2, and HCDR3 sequences of SEQ ID NOs: 125, 126, and 127, respectively, and LCDR1, LCDR2, and LCDR3 sequences of SEQ ID NOs: 135, 136, and 137, respectively, a VH sequence having at least 60, 70, 80, 90, 91, 92, 93, 94, 95, 96, 97, 98 or 99 percent identity to SEQ ID NO: 134, and a VL sequence having at least 60, 70, 80, 90, 91, 92, 93, 94, 95, 96, 97, 98 or 99 percent identity to SEQ ID NO: 144.

In one embodiment, the HSA-BD of the invention comprises:
(a) HCDR1, HCDR2, and HCDR3 sequences of SEQ ID NOs: 103, 104, and 105, respectively, and LCDR1, LCDR2, and LCDR3 sequences of SEQ ID NOs: 113, 114, and 115, respectively, a VH sequence of SEQ ID NO: 112 and a VL sequence of SEQ ID NO: 122;
(b) HCDR1, HCDR2, and HCDR3 sequences of SEQ ID NOs: 125, 126, and 127, respectively, and LCDR1, LCDR2, and LCDR3 sequences of SEQ ID NOs: 135, 136, and 137, respectively, a VH sequence of SEQ ID NO: 134 and a VL sequence of SEQ ID NO: 144.

Suitably, the HSA-BD of the invention is selected from the group consisting of: a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).

Suitably, the HSA-BD of the invention is scFv antibody fragment. In one embodiment, the HSA-BD of the present invention is as set forth in SEQ ID NO: 124 or SEQ ID NO: 146.

Other suitable HSA-BD for use in the multispecific antibody of the invention comprises or is derived from an antibody selected from the group consisting of: (i) polypeptides that bind serum albumin (see, for example, Smith et al., 2001, Bioconjugate Chem. 12:750-756; EP0486525; US6267964; WO04/001064; WO02/076489; and WO01/45746); (ii) anti-serum albumin binding single variable domains described in Holt et al., Protein Engineering, Design & Selection, vol 21, 5, pp283-288, WO04003019, WO2008/096158, WO05118642, WO2006/0591056 and WO2011/006915; (iii) anti-serum albumin antibodies described in WO2009/040562, WO2010/035012 and WO2011/086091.

The multispecific antibody of the invention may be in any suitable format.

Suitably, the binding domains of the multispecific antibody are operably linked. The binding domains of the multispecific antibody of the invention are capable of binding to their respective antigens or receptors simultaneously.

In one embodiment, the multispecific antibody of the invention comprises at least one CD137-BD, at least one PDL1-BD, wherein: (i) said CD137-BD and said PDL1-BD are both operably linked to each other. In one embodiment, the multispecific antibody of the invention comprises at least one CD137-BD, at least one PDL1-BD, at least one HSA-BD, wherein: (i) said CD137-BD and said PDL1-BD are both operably linked to said HSA-BD; or (ii) said CD137-BD and said HSA-BD are both operably linked to said PDL1-BD; or (iii) said PDL1-BD and said HSA-BD are both operably linked to said CD137-BD.

The term "operably linked", as used herein, indicates that two molecules (e.g., polypeptides, domains, binding domains) are attached so as to each retain functional activity. Two molecules can be "operably linked" whether they are attached directly or indirectly (e.g., via a linker, via a moiety, via a linker to a moiety). The term "linker" refers to a peptide or other moiety that is optionally located between binding domains or antibody fragments of the invention. A number of strategies may be used to covalently link molecules together. These include, but are not limited to polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (e.g., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e.g., 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)n, (GSGGS)n (GGGGS)n and (GGGS)n, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since both of these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single chain antibodies.

Suitably, the multispecific antibody is in a format selected from any suitable multispecific, e.g. bispecific, format known in the art, including, by way of non-limiting example, formats based on a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab,, Fab-Fv2, Morrison (IgG CH₃-scFv fusion (Morrison L) or IgG CL-scFv fusion (Morrison H)), triabody (scDb-scFv), bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs) (bispecific IgGs prepared by the KiH technology); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH (described in WO2016/0202457; Egan T., et al., mAbs 9 (2017) 68-84) and DuoBodies(bispecific IgGs prepared by the Duobody technology) (MAbs. 2017 Feb/Mar;9(2):182-212. doi: 10.1080/19420862.2016.1268307).Particularly suitable for use herein is a single-chain diabody (scDb) or triabody (scDb-scFv).

In one embodiment, the multispecific antibody of the invention is in a format selected from the list consisting of scDb, scDb-scFv, tribody, triabody, and sc triabody. Particularly suitable for use herein is a single-chain diabody (scDb), in particular a bispecific monomeric scDb. Also, particularly suitable for use herein is a triabody.

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP404097, WO1993/01161, Hudson et al., Nat. Med. 9:129-134 (2003), and Hollinger et al., Proc. Natl. Acad. Sci. USA 90: 6444-6448 (1993). Triabodies and tetrabodies are also described in Hudson et al., Nat. Med. 9:129-134 (2003).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3 -VLA, VHA-L1-VHB-L2-VLB-L3 -VLA, VLA-L1 -VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen binding site for the first antigen, and VLB and VHB jointly form the antigen binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. The middle linker L2 particularly is a peptide of 10-40 amino acids, more particularly 15-30 amino acids, and most particularly 20-25 amino acids.

In one embodiment, the multispecific antibody of the invention is a scDb. In a specific embodiment, the multispecific antibody of the invention is a scDb comprising an amino acid sequence having at least 60, 70, 80, 90, 91, 92, 93, 94, 95, 96, 97, 98 or 99 percent identity to any of SEQ ID NOs: 156, 157, 158, 159, 160, 161, and 162. In one embodiment, the multispecific antibody of the invention is a scDb comprising an amino acid sequence selected from any of SEQ ID NOs: 156, 157, 158, 159, 160, 161, and 162. In a further embodiment, the multispecific antibody of the present invention is a scDb consisting of an amino acid sequence selected from any of SEQ ID NOs: 156, 157, 158, 159, 160, 161, and 162.

In one embodiment, the multispecific antibody of the invention is a scTriabody. In a specific embodiment, the multispecific antibody of the invention is a scTriabody comprising an amino acid sequence having at least 60, 70, 80, 90, 91, 92, 93, 94, 95, 96, 97, 98 or 99 percent identity to any of SEQ ID NOs: 163, 164, 165, and 166. In one embodiment, the multispecific antibody of the invention is a scTriabody comprising an amino acid sequence selected from any of SEQ ID NOs: 163, 164, 165, and 166. In a further embodiment, the multispecific antibody of the invention is a scTriabody consisting of an amino acid sequence selected from any of SEQ ID NOs: 163, 164, 165, and 166.

In one embodiment of the present invention, the multispecific antibody of the invention is in Morrison-L format.

In a specific embodiment, the multispecific antibody of the invention comprises (i) a Morrison-L Light chain comprising an amino acid sequence having at least 60, 70, 80, 90, 91, 92, 93, 94, 95, 96, 97, 98 or 99 percent identity to any of SEQ ID NO: 167, and (ii) a Morrison-L Heavy chain comprising an amino acid sequence having at least 60, 70, 80, 90, 91, 92, 93, 94, 95, 96, 97, 98 or 99 percent identity to any of SEQ ID NO: 168. In one embodiment, the multispecific antibody of the invention comprises (i) a Morrison-L Light chain comprising an amino acid of SEQ ID NO: 167, and (ii) a Morrison-L Heavy chain comprising an amino acid sequence of SEQ ID NO: 168. In a further embodiment, the multispecific antibody of the invention consists of (i) a Morrison-L Light chain comprising an amino acid of SEQ ID NO: 167, and (ii) a Morrison-L Heavy chain comprising an amino acid sequence of SEQ ID NO: 168.

In another embodiment, the multispecific antibody of the invention comprises (i) a Morrison-L Light chain comprising an amino acid sequence having at least 60, 70, 80, 90, 91, 92, 93, 94, 95, 96, 97, 98 or 99 percent identity to any of SEQ ID NO: 171, and (ii) a Morrison-L Heavy chain comprising an amino acid sequence having at least 60, 70, 80, 90, 91, 92, 93, 94, 95, 96, 97, 98 or 99 percent identity to any of SEQ ID NO: 172. In one embodiment, the multispecific antibody of the invention comprises (i) a Morrison-L Light chain comprising an amino acid of SEQ ID NO: 171, and (ii) a Morrison-L Heavy chain comprising an amino acid sequence of SEQ ID NO: 172. In a further embodiment, the multispecific antibody of the invention consists of (i) a Morrison-L Light chain comprising an amino acid of SEQ ID NO: 171, and (ii) a Morrison-L Heavy chain comprising an amino acid sequence of SEQ ID NO: 172.

In one embodiment of the present invention, the multispecific antibody of the invention is in Morrison-H format.

In a specific embodiment, the multispecific antibody of the invention comprises (i) a Morrison-H Light chain comprising an amino acid sequence having at least 60, 70, 80, 90, 91, 92, 93, 94, 95, 96, 97, 98 or 99 percent identity to any of SEQ ID NO: 169, and (ii) a Morrison-H Heavy chain comprising an amino acid sequence having at least 60, 70, 80, 90, 91, 92, 93, 94, 95, 96, 97, 98 or 99 percent identity to any of SEQ ID NO: 170. In one embodiment, the multispecific antibody of the invention comprises (i) a Morrison-H Light chain comprising an amino acid of SEQ ID NO: 169, and (ii) a Morrison-H Heavy chain comprising an amino acid sequence of SEQ ID NO: 170. In a further embodiment, the multispecific antibody of the invention consists of (i) a Morrison-L Light chain comprising an amino acid of SEQ ID NO: 169, and (ii) a Morrison-L Heavy chain comprising an amino acid sequence of SEQ ID NO: 170.

In another embodiment, the multispecific antibody of the invention comprises (i) a Morrison-H Light chain comprising an amino acid sequence having at least 60, 70, 80, 90, 91, 92, 93, 94, 95, 96, 97, 98 or 99 percent identity to any of SEQ ID NO: 173, and (ii) a Morrison-H Heavy chain comprising an amino acid sequence having at least 60, 70, 80, 90, 91, 92, 93, 94, 95, 96, 97, 98 or 99 percent identity to any of SEQ ID NO: 174. In one embodiment, the multispecific antibody of the invention comprises (i) a Morrison-H Light chain comprising an amino acid of SEQ ID NO: 173, and (ii) a Morrison-H Heavy chain comprising an amino acid sequence of SEQ ID NO: 174. In a further embodiment, the multispecific antibody of the invention consists of (i) a Morrison-L Light chain comprising an amino acid of SEQ ID NO: 173, and (ii) a Morrison-L Heavy chain comprising an amino acid sequence of SEQ ID NO: 174.

In one embodiment of the present invention, the multispecific antibody of the invention is in a MATCH format described in WO2016/0202457; Egan T., et al., mAbs 9 (2017) 68-84.

The multispecific antibody of the invention can be produced using any convenient antibody manufacturing method known in the art (see, e.g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct of the invention further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e.g., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e.g., Yokoyama et al., Curr. Protoc. Immunol. Chapter 2, Unit 2.5, 2006) or by means of recombinant antibody engineering (repertoire cloning or phage display/yeast display) (see, e.g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific molecules of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N- succinimidyl-3- (2-pyridyldithio)propionate (SPDP), and sulfosuccinimidyl 4- (N-maleimidomethyl)cyclohaxane-1-carboxylate (sulfo-SMCC) (see e.g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111.).

When the binding specificities are antibodies, they can be conjugated by sulfhydryl bonding of the C-terminus hinge regions of the two heavy chains. In a particularly embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb X mAb, mAb X Fab, Fab X F (ab')2 or ligand X Fab fusion protein. A multispecific antibody of the invention can be a single chain molecule comprising one single chain antibody and a binding determinant, or a single chain multispecific antibody comprising two binding determinants. Multispecific antibody may comprise at least two single chain molecules. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest.

In a further aspect, the invention provides a nucleic acid encoding the multispecific antibody of the invention or fragments thereof or binding domains thereof. Such nucleic acid sequences can be optimized for expression in mammalian cells.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the multispecific antibody described above. When expressed from appropriate expression vectors, polypeptides encoded by these nucleic acid molecules are capable of exhibiting antigen binding capacity or capacities of the multispecific antibody of the present invention.

Also provided in the invention are polynucleotides which encode at least one CDR region and usually all three CDR regions of the binding domains of the multispecific antibody of the present invention set forth in Tables 1 to 3. Because of the degeneracy of the code, a variety of nucleic acid sequences will encode each of the immunoglobulin amino acid sequences.

The polynucleotide sequences can be produced by de novo solid-phase DNA synthesis or by PCR mutagenesis of an existing sequence (e.g., sequences as described in the Examples below) encoding the multispecific antibody of the invention or fragments thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the multispecific antibody of the invention or fragments thereof or binding domains thereof.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno- associated viruses), which serve equivalent functions. In this particular context, the term "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the multispecific antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., Nat Genet. 15:345, 1997). For example, nonviral vectors useful for expression of the CD137-binding polynucleotides and polypeptides in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e.g., enhancers) that are operably linked to the polynucleotides encoding a multispecific antibody chain or a fragment. In one embodiment, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of a multispecific antibody chain or a fragment. These elements typically include an ATG initiation codon and adjacent ribosome binding site or other sequences. In addition, the efficiency of expression may be enhanced by the inclusion of enhancers appropriate to the cell system in use (see, e.g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted the multispecific antibody of the invention or fragments thereof or binding domains thereof sequences. More often, the inserted the multispecific antibody of the invention or fragments thereof or binding domains thereof sequences are linked to signal sequences before inclusion in the vector. Vectors to be used to receive sequences encoding binding domains of the multispecific antibody light and heavy chain variable domains sometimes also encode constant regions or parts thereof. Such vectors allow expression of the variable regions as fusion proteins with the constant regions thereby leading to production of intact antibodies and antigen-binding fragments thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the multispecific antibody of the invention or fragments thereof or binding domains thereof can be either prokaryotic or eukaryotic. E. coli is one prokaryotic host useful for cloning and expressing the polynucleotides of the present invention. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express CD137-binding polypeptides of the invention. Insect cells in combination with baculovirus vectors can also be used.

In one embodiment, mammalian host cells are used to express and produce the multispecific antibody of the invention or fragments thereof or binding domains thereof. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP poIIII promoter, the constitutive MPS V promoter, the tetracycline-inducible CMV promoter (such as the human immediate-early CMV promoter), the constitutive CMV promoter, and promoter- enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycatiomnucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express the multispecific antibody of the invention or fragments thereof or binding domains thereof can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell expressing a nucleic acid encoding the antibody of the invention or antigen-binding fragment thereof.

In one aspect, the present invention relates to a method of producing the multispecific antibody of the invention or a binding domain thereof or a fragment thereof, the method comprising the step of culturing a host cell expressing a nucleic acid encoding the multispecific antibody of the invention or a binding domain thereof or a fragment thereof.

In a further aspect, the present invention relates to a pharmaceutical composition comprising the multispecific antibody of the invention, and a pharmaceutically acceptable carrier. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e.g., by injection or infusion). Depending on the route of administration, the active compound, i.e., the multispecific antibody of the invention, may be coated in a material to protect the compound from the action of acids and other natural conditions that may inactivate the compound.

Pharmaceutical compositions of the invention can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the multispecific antibody of the invention or the pharmaceutical composition of the invention for use as a medicament. In a suitable embodiment, the present invention provides the multispecific antibody or the pharmaceutical composition for use in treatment of cancer in a subject in need thereof.

In another aspect, the present invention provides the multispecific antibody or the pharmaceutical composition for use in a manufacture of a medicament. In a suitable embodiment, the present invention provides the multispecific antibody or the pharmaceutical composition for use in a manufacture of a medicament for treatment of cancer.

In another aspect, the present invention relates to use of the multispecific antibody or the pharmaceutical composition for treating cancer in a subject in need thereof.

In a further aspect, the present invention relates to use of the multispecific antibody or the pharmaceutical composition in the manufacture of a medicament for treatment of a cancer, in a subject in need thereof.

In another aspect, the present invention relates to a method of treating a subject comprising administering to the subject a therapeutically effective amount of the multispecific antibody of the present invention. In a suitable embodiment, the present invention relates to a method of treating cancer in a a subject comprising administering to the subject a therapeutically effective amount of the multispecific antibody of the present invention.

The term "subject" includes human and non-human animals. Non-human animals include all vertebrates, e.g., mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e.g., in a human, and includes: (a) inhibiting the disease, i.e., arresting its development; and (c) relieving the disease, i.e., causing regression of the disease.

The term "therapeutically effective amount" or "efficacious amount" refers to the amount of an agent that, when administered to a mammal or other subject for treating a disease, is sufficient to effect such treatment for the disease. The "therapeutically effective amount" will vary depending on the agent, the disease and its severity and the age, weight, etc., of the subject to be treated.

The term "cancer" refers to a disease characterized by the rapid and uncontrolled growth of aberrant cells. Cancer cells can spread locally or through the bloodstream and lymphatic system to other parts of the body. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

In one embodiment, said cancer is PDL1-positive, preferably wherein said cancer expresses high levels of PDL1 in comparison to a healthy tissue. In some embodiments, said cancer is malignant. In some embodiments, said cancer is benign. In some embodiments, said cancer is primary. In some embodiments, said cancer is secondary. In one embodiment, said cancer is lung cancer, preferably non-small cell lung cancer (NSCLC). In another embodiment, said cancer is colorectal cancer.

**TABLE 1. Examples of CD137 binding domains of the present invention.**

| **SEQ ID NUMBER** | **Ab region** | **Sequence** |
|---|---|---|
| **38-02-A04** | | |
| SEQ ID NO: 1 (AHo) | HCDR1 | VSGFSFSNSYW |
| | (38-02-A04 sc01) | |
| | (38-02-A04 sc05 IF) | |
| SEQ ID NO: 2 (AHo) | HCDR1 | ASGFSFSNSYW |
| | (38-02-A04 sc06 Full) | |
| SEQ ID NO: 3 (AHo) | HCDR2 | TFVGSSDSTYYANWAKGR |
| | (38-02-A04 sc01) | |
| | (38-02-A04 sc05 IF) | |
| | (38-02-A04 sc06 Full) | |
| SEQ ID NO: 4 | HCDR3 | HPSDAVYGYANN |
| (AHo) | | |
| | (38-02-A04 sc01) | |
| | (38-02-A04 sc05 IF) | |
| | (38-02-A04 sc06 Full) | |
| SEQ ID NO: 5 (Kabat) | HCDR1 | NSYWIC |
| | (38-02-A04 sc01) | |
| | (38-02-A04 sc05 IF) | |
| | (38-02-A04 sc06 Full) | |
| SEQ ID NO: 6 (Kabat) | HCDR2 | CTFVGSSDSTYYANWAKG |
| | (38-02-A04 sc01) | |
| | (38-02-A04 sc05 IF) | |
| | (38-02-A04 sc06 Full) | |
| SEQ ID NO: 7 (Kabat) | HCDR3 | HPSDAVYGYANNL |
| | (38-02-A04 sc01) | |
| | (38-02-A04 sc05 IF) | |
| | (38-02-A04 sc06 Full) | |
| SEQ ID NO: 8 (Chothia) | HCDR1 | GFSFSNSY |
| | (38-02-A04 sc01) | |
| | (38-02-A04 sc05 IF) | |
| | (38-02-A04 sc06 Full) | |
| SEQ ID NO: 9 (Chothia) | HCDR2 | FVGSSDS |
| | (38-02-A04 sc01) | |
| | (38-02-A04 sc05 IF) | |
| | (38-02-A04 sc06 Full) | |
| SEQ ID NO: 10 (Chothia) | HCDR3 | HPSDAVYGYANNL |
| | (38-02-A04 sc01) | |
| | (38-02-A04 sc05 IF) | |
| | (38-02-A04 sc06 Full) | |
| SEQ ID NO: 11 | VH | QVQLQESGPGLVKPSETLSLTCKVSG FSFSNSYWICWIRQPPGKGLEWIGCTF VGSSDSTYYANWAKGRVTISVDSSK NQFSLKLSSVTAADTAVYYCARHPS DAVYGYANNLWGQGTLVTVSS |
| | (VH4) | |
| | (38-02-A04 sc01) | |
| SEQ ID NO: 12 | VH | QVQLQESGPGLVKPSETLSLTCKVSG FSFSNSYWICWVRQPPGKGLEWIGCT FVGSSDSTYYANWAKGRVTISVDSSK NQVSLKLSSVTAADTAVYFCARHPS DAVYGYANNLWGQGTLVTVSS |
| | (VH4) | |
| | (38-02-A04 sc05 IF) | |
| | Mutations VH: I44V; F89V; Y105F. | |
| SEQ ID NO: 13 | VH | QVQLQESGPGLVKPSETLSLTCKASG FSFSNSYWICWVRQPPGKGLEWIGCT FVGSSDSTYYANWAKGRVTISKDSSK |
| | (VH4) | |
| | (38-02-A04 sc06 Full) | NQVSLKLSSVTAADTAVYFCARHPS DAVYGYANNLWGQGTLVTVSS |
| | Mutations VH: V25A; 144V; V82K; F89V; Y105F | |
| SEQ ID NO: 14 (AHo) | LCDR1 | ASQSINNV |
| | (38-02-A04 sc01) | |
| | (38-02-A04 sc05 IF) | |
| | (38-02-A04 sc06 Full) | |
| SEQ ID NO: 15 (AHo) | LCDR2 | RASTLASGVPSR |
| | (38-02-A04 sc01) | |
| | (38-02-A04 sc05 IF) | |
| | (38-02-A04 sc06 Full) | |
| SEQ ID NO: 16 (AHo) | LCDR3 | SYGNYG |
| | (38-02-A04 sc01) | |
| | (38-02-A04 sc05 IF) | |
| | (38-02-A04 sc06 Full) | |
| SEQ ID NO: 17 (Kabat) | LCDR1 | QASQSINNVLA |
| | (38-02-A04 sc01) | |
| | (38-02-A04 sc05 IF) | |
| | (38-02-A04 sc06 Full) | |
| SEQ ID NO: 18 (Kabat) | LCDR2 | RASTLAS |
| | (38-02-A04 sc01) | |
| | (38-02-A04 sc05 IF) | |
| | (38-02-A04 sc06 Full) | |
| SEQ ID NO: 19 (Kabat) | LCDR3 | QSSYGNYGD |
| | (38-02-A04 sc01) | |
| | (38-02-A04 sc05 IF) | |
| | (38-02-A04 sc06 Full) | |
| SEQ ID NO: 20 (Chothia) | LCDR1 | QASQSINNVLA |
| | (38-02-A04 sc01) | |
| | (38-02-A04 sc05 IF) | |
| | (38-02-A04 sc06 Full) | |
| SEQ ID NO: 21 (Chothia) | LCDR2 | RASTLAS |
| | (38-02-A04 sc01) | |
| | (38-02-A04 sc05 IF) | |
| | (38-02-A04 sc06 Full) | |
| SEQ ID NO: 22 (Chothia) | LCDR3 | QSSYGNYGD |
| | (38-02-A04 sc01) | |
| | (38-02-A04 sc05 IF) | |
| | (38-02-A04 sc06 Full) | |
| SEQ ID NO: 23 | VL | 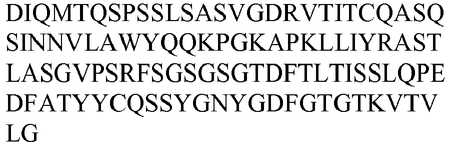 |
| | (Vk1-sk17) | |
| | (38-02-A04 sc01) | |
| SEQ ID NO: 24 | VL | 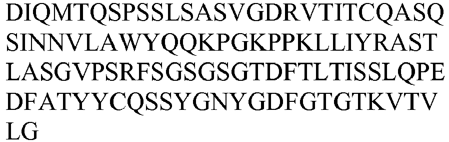 |
| | (Vk1-sk17) | |
| | (38-02-A04 sc05 IF) | |
| | Mutations VL: A51P | |
| SEQ ID NO: 25 | VL | DLQMTQSPSSLSASVGDRVTITCQAS QSINNVLAWYQQKPGKPPKLLIYRAS TLASGVPSRFSGSGSGTDFTLTISSLQP EDFATYYCQSSYGNYGDFGTGTKVT VLG |
| | (Vk1-sk17) | |
| | (38-02-A04 sc06 Full) | |
| | Mutations VL: I2L; A51P | |
| SEQ ID NO: 26 | Linker | GGGGSGGGGSGGGGSGGGGS |
| SEQ ID NO: 27 | scFv (VL-linker-VH) | DIQMTQSPSSLSASVGDRVTITCQASQ SINNVLAWYQQKPGKAPKLLIYRAST LASGVPSRFSGSGSGTDFTLTISSLQPE DFATYYCQSSYGNYGDFGTGTKVTV LGGGGGSGGGGSGGGGSGGGGSQV QLQESGPGLVKPSETLSLTCKVSGFSF SNSYWICWIRQPPGKGLEWIGCTFVG SSDSTYYANWAKGRVTISVDSSKNQF SLKLSSVTAADTAVYYCARHPSDAV YGYANNLWGQGTLVTVSS |
| | (38-02-A04 sc01) | |
| SEQ ID NO: 28 | scFv (VL-linker-VH) | 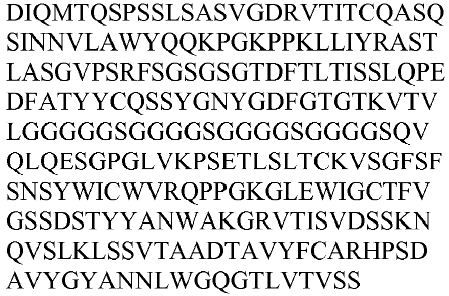 |
| | (38-02-A04 sc05 IF) | |
| SEQ ID NO: 29 | scFv (VL-linker-VH) | 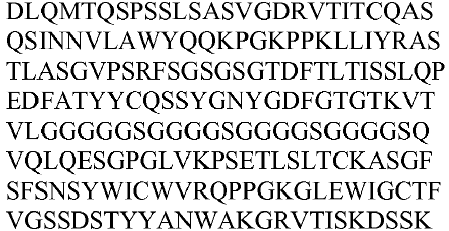 |
| | (38-02-A04 sc06 Full) | |
| | | NQVSLKLSSVTAADTAVYFCARHPS DAVYGYANNLWGQGTLVTVSS |

| **38-27-C05 sc01** | | |
|---|---|---|
| SEQ ID NO: 30 (AHo) | HCDR1 | VSGFSFNNDYD |
| SEQ ID NO: 31 (AHo) | HCDR2 | IDTGDGSTYYASWAKGR |
| SEQ ID NO: 32 (AHo) | HCDR3 | EAASSSGYGMGYFD |
| SEQ ID NO: 33 (Kabat) | HCDR1 | NDYDMC |
| SEQ ID NO: 34 (Kabat) | HCDR2 | CIDTGDGSTYYASWAKG |
| SEQ ID NO: 35 (Kabat) | HCDR3 | EAASSSGYGMGYFDL |
| SEQ ID NO: 36 (Chothia) | HCDR1 | GFSFNNDY |
| SEQ ID NO: 37 (Chothia) | HCDR2 | DTGDGS |
| SEQ ID NO: 38 (Chothia) | HCDR3 | EAASSSGYGMGYFDL |
| SEQ ID NO: 39 | VH | QVQLQESGPGLVKPSETLSLTCKVSG FSFNNDYDMCWIRQPPGKGLEWIGCI DTGDGSTYYASWAKGRVTISVDSSK NQFSLKLSSVTAADTAVYYCAREAA SSSGYGMGYFDLWGQGTLVTVSS |
| | (VH4) | |
| SEQ ID NO: 40 (AHo) | LCDR1 | SSQSVYDNNW |
| SEQ ID NO: 41 (AHo) | LCDR2 | RASNLASGVPSR |
| SEQ ID NO: 42 (AHo) | LCDR3 | TYLSSNWYW |
| SEQ ID NO: 43 (Kabat) | LCDR1 | QSSQSVYDNNWLA |
| SEQ ID NO: 44 (Kabat) | LCDR2 | RASNLAS |
| SEQ ID NO: 45 (Kabat) | LCDR3 | QGTYLSSNWYWA |
| SEQ ID NO: 46 (Chothia) | LCDR1 | QSSQSVYDNNWLA |
| SEQ ID NO: 47 (Chothia) | LCDR2 | RASNLAS |
| SEQ ID NO: 48 (Chothia) | LCDR3 | QGTYLSSNWYWA |
| SEQ ID NO: 49 | VL | DIQMTQSPSSLSASVGDRVTITCQSSQ SVYDNNWLAWYQQKPGKAPKLLIY RASNLASGVPSRFSGSGSGTDFTLTIS SLQPEDFATYYCQGTYLSSNWYWAF GTGTKVTVLG |
| | (Vk1-sk17) | |
| SEQ ID NO: 50 | Linker | GGGGSGGGGSGGGGSGGGGS |
| SEQ ID NO: 51 | scFv (VL-linker-VH) | DIQMTQSPSSLSASVGDRVTITCQSSQ SVYDNNWLAWYQQKPGKAPKLLIY RASNLASGVPSRFSGSGSGTDFTLTIS SLQPEDFATYYCQGTYLSSNWYWAF GTGTKVTVLGGGGGSGGGGSGGGGS GGGGSQVQLQESGPGLVKPSETLSLT CKVSGFSFNNDYDMCWIRQPPGKGL EWIGCIDTGDGSTYYASWAKGRVTIS VDSSKNQFSLKLSSVTAADTAVYYC AREAASSSGYGMGYFDLWGQGTLVT VSS |

**TABLE 2. Examples of PDL1 binding domains of the present invention.**

| **SEQ ID NUMBER** | **Ab region** | **Sequence** |
|---|---|---|
| **37-20-B03sc01 and 37-20-B03sc02** | | |
| SEQ ID NO: 52 (AHo) | **HCDR1** | VSGFSFNSDYW |
| | (37-20-B03sc01) | |
| SEQ ID NO: 53 (AHo) | **HCDR1** | ASGFSFNSDYW |
| | (37-20-B03sc02) | |
| SEQ ID NO: 54 (AHo) | **HCDR2** | IYGGSSGNTQYASWAQGR |
| | (37-20-B03sc01) | |
| | (37-20-B03sc02) | |
| SEQ ID NO: 55 (AHo) | **HCDR3** | GYVDYGGATD |
| | (37-20-B03sc01) | |
| | (37-20-B03sc02) | |
| SEQ ID NO: 56 (Kabat) | **HCDR1** | SDYWIY |
| | (37-20-B03sc01) | |
| | (37-20-B03sc02) | |
| SEQ ID NO: 57 (Kabat) | **HCDR2** | SIYGGSSGNTQYASWAQG |
| | (37-20-B03sc01) | |
| | (37-20-B03sc02) | |
| SEQ ID NO: 58 (Kabat) | **HCDR3** | GYVDYGGATDL |
| | (37-20-B03sc01) | |
| | (37-20-B03sc02) | |
| SEQ ID NO: 59 (Chothia) | **HCDR1** | GFSFNSDY |
| | (37-20-B03sc01) | |
| | (37-20-B03sc02) | |
| SEQ ID NO: 60 (Chothia) | **HCDR2** | YGGSSGN |
| | (37-20-B03sc01) | |
| | (37-20-B03sc02) | |
| SEQ ID NO: 61 (Chothia) | **HCDR3** | GYVDYGGATDL |
| | (37-20-B03sc01) | |
| | (37-20-B03sc02) | |
| SEQ ID NO: 62 | **VH** | QVQLQESGPGLVKPSETLSLTCKVSGF |
| | (VH4) | SFNSDYWIYWIRQPPGKGLEWIGSIYG GSSGNTQYASWAQGRVTISVDSSKNQ FSLKLSSVTAADTAVYYCARGYVDYG GATDLWGQGTLVTVSS |
| | (37-20-B03sc01) | |
| SEQ ID NO: 63 | **VH** | QVQLVQSGAEVKKPGASVKVSCKASG FSFNSDYWIYWVRQAPGQGLEWMGSI YGGSSGNTQYASWAQGRVTMTRDTSI STAYMELSSLRSEDTAVYYCARGYVD YGGATDLWGQGTLVTVSS |
| | (VH1) | |
| | (37-20-B03sc02) | |
| SEQ ID NO: 64 (AHo) | **LCDR1** | ASQSIGTY |
| | (37-20-B03sc01) | |
| | (37-20-B03sc02) | |
| SEQ ID NO: 65 (AHo) | **LCDR2** | RAFILASGVPSR |
| | (37-20-B03sc01) | |
| | (37-20-B03sc02) | |
| SEQ ID NO: 66 (AHo) | **LCDR3** | NFYSDSTTIGPN |
| | (37-20-B03sc01) | |
| | (37-20-B03sc02) | |
| SEQ ID NO: 67 (Kabat) | **LCDR1** | QASQSIGTYLA |
| | (37-20-B03sc01) | |
| | (37-20-B03sc02) | |
| SEQ ID NO: 68 (Kabat) | **LCDR2** | RAFILAS |
| | (37-20-B03sc01) | |
| | (37-20-B03sc02) | |
| SEQ ID NO: 69 (Kabat) | **LCDR3** | QSNFYSDSTTIGPNA |
| | (37-20-B03sc01) | |
| | (37-20-B03sc02) | |
| SEQ ID NO: 70 (Chothia) | **LCDR1** | QASQSIGTYLA |
| | (37-20-B03sc01) | |
| | (37-20-B03sc02) | |
| SEQ ID NO: 71 (Chothia) | **LCDR2** | RAFILAS |
| | (37-20-B03sc01) | |
| | (37-20-B03sc02) | |
| SEQ ID NO: 72 (Chothia) | **LCDR3** | QSNFYSDSTTIGPNA |
| | (37-20-B03sc01) | |
| | (37-20-B03sc02) | |
| SEQ ID NO: 73 | **VL** | DIQMTQSPSSLSASVGDRVTITCQASQS IGTYLAWYQQKPGKAPKLLIYRAFILA SGVPSRFSGSGSGTDFTLTISSLQPEDF ATYYCQSNFYSDSTTIGPNAFGTGTKV TVLG |
| | (Vk1-sk17) | |
| | (37-20-B03sc01) | |
| | (37-20-B03sc02) | |
| SEQ ID NO: 74 | **Linker** | GGGGSGGGGSGGGGGSGGGGS |
| SEQ ID NO: 75 | **scFv (VL-linker-VH)** | DIQMTQSPSSLSASVGDRVTITCQASQS IGTYLAWYQQKPGKAPKLLIYRAFILA SGVPSRFSGSGSGTDFTLTISSLQPEDF ATYYCQSNFYSDSTTIGPNAFGTGTKV TVLGGGGGSGGGGSGGGGSGGGGSQ VQLQESGPGLVKPSETLSLTCKVSGFSF NSDYWIYWIRQPPGKGLEWIGSIYGGS |
| | (37-20-B03sc01) | |
| | (PRO997) | |
| | | SGNTQYASWAQGRVTISVDSSKNQFSL KLSSVTAADTAVYYCARGYVDYGGA TDLWGQGTLVTVSS |
| SEQ ID NO: 76 | **scFv (VL-linker-VH)** | DIQMTQSPSSLSASVGDRVTITCQASQS IGTYLAWYQQKPGKAPKLLIYRAFILA SGVPSRFSGSGSGTDFTLTISSLQPEDF ATYYCQSNFYSDSTTIGPNAFGTGTKV TVLGGGGGSGGGGSGGGGSGGGGSQ VQLVQSGAEVKKPGASVKVSCKASGF SFNSDYWIYWVRQAPGQGLEWMGSIY GGSSGNTQYASWAQGRVTMTRDTSIS TAYMELSSLRSEDTAVYYCARGYVDY GGATDLWGQGTLVTVSS |
| | (37-20-B03sc02) | |
| | (PRO1013) | |

| **33_03_G02 sc01 and 33_03_G02 sc03 Full** | | |
|---|---|---|
| SEQ ID NO: 77 (AHo) | **HCDR1** | VSGFSFSSGYD |
| | (33_03_G02 sc01) | |
| SEQ ID NO: 78 (AHo) | **HCDR1** | ASGFSFSSGYD |
| | (33_03_G02 sc03 Full) | |
| SEQ ID NO: 79 (AHo) | **HCDR2** | VVAGSVDITYYASWAKGR |
| | (33_03_G02 sc01) | |
| | (33_03_G02 sc03 Full) | |
| SEQ ID NO: 80 (AHo) | **HCDR3** | KDAYSDAFN |
| | (33_03_G02 sc01) | |
| | (33_03_G02 sc03 Full) | |
| SEQ ID NO: 81 (Kabat) | **HCDR1** | SGYDMC |
| | (33_03_G02 sc01) | |
| | (33_03_G02 sc03 Full) | |
| SEQ ID NO: 82 (Kabat) | **HCDR2** | CVVAGSVDITYYASWAKG |
| | (33_03_G02 sc01) | |
| | (33_03_G02 sc03 Full) | |
| SEQ ID NO: 83 (Kabat) | **HCDR3** | KDAYSDAFNL |
| | (33_03_G02 sc01) | |
| | (33_03_G02 sc03 Full) | |
| SEQ ID NO: 84 (Chothia) | **HCDR1** | GFSFSSGY |
| | (33_03_G02 sc01) | |
| | (33_03_G02 sc03 Full) | |
| SEQ ID NO: 85 (Chothia) | **HCDR2** | VAGSVDI |
| | (33_03_G02 sc01) | |
| | (33_03_G02 sc03 Full) | |
| SEQ ID NO: 86 (Chothia) | **HCDR3** | KDAYSDAFNL |
| | (33_03_G02 sc01) | |
| | (33_03_G02 sc03 Full) | |
| SEQ ID NO: 87 | **VH** | QVQLQESGPGLVKPSETLSLTCKVSGF SFSSGYDMCWIRQPPGKGLEWIGCVV AGSVDITYYASWAKGRVTISVDSSKN QFSLKLSSVTAADTAVYYCARKDAYS |
| | (VH4) | |
| | (33_03_G02 sc01) | |
| | | DAFNLWGQGTLVTVSS |
| SEQ ID NO: 88 | **VH** | 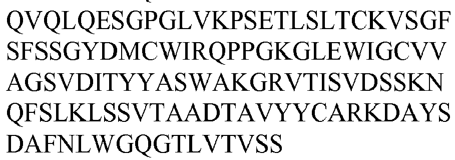 |
| | (VH4) | |
| | (33_03_G02 sc03 Full) | |
| | (Mutations: V2S; V25A; I44V; G56A; V82K; F89V; Y105F) | |
| SEQ ID NO: 89 (AHo) | **LCDR1** | ASQSINDY |
| | (33_03_G02 sc01) | |
| | (33_03_G02 sc03 Full) | |
| SEQ ID NO: 90 (AHo) | **LCDR2** | KASTLASGVPSR |
| | (33_03_G02 sc01) | |
| | (33_03_G02 sc03 Full) | |
| SEQ ID NO: 91 (AHo) | **LCDR3** | GYIITDIDN |
| | (33_03_G02 sc01) | |
| | (33 03 G02 sc03 Full) | |
| SEQ ID NO: 92 (Kabat) | **LCDR1** | QASQSINDYLA |
| | (33_03_G02 sc01) | |
| | (33_03_G02 sc03 Full) | |
| SEQ ID NO: 93 (Kabat) | **LCDR2** | KASTLAS |
| | (33_03_G02 sc01) | |
| | (33_03_G02 sc03 Full | |
| SEQ ID NO: 94 (Kabat) | **LCDR3** | QQGYIITDIDNV |
| | (33_03_G02 sc01) | |
| | (33_03_G02 sc03 Full) | |
| SEQ ID NO: 95 (Chothia) | **LCDR1** | QASQSINDYLA |
| | (33_03_G02 sc01) | |
| | (33_03_G02 sc03 Full) | |
| SEQ ID NO: 96 (Chothia) | **LCDR2** (33_03_G02 sc01) (33_03_G02 sc03 Full) | KASTLAS |
| SEQ ID NO: 97 (Chothia) | **LCDR3** | QQGYIITDIDNV |
| | (33_03_G02 sc01) | |
| | (33_03_G02 sc03 Full) | |
| SEQ ID NO: 98 | **VL** | 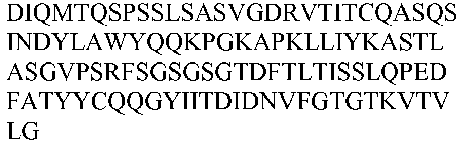 |
| | (Vk1-sk17) | |
| | (33_03_G02 sc01) | |
| SEQ ID NO: 99 | VL | 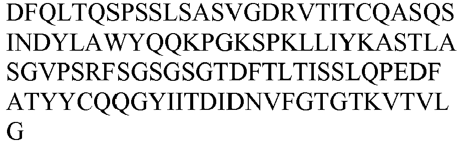 |
| | (Vk1-sk17) | |
| | (33_03_G02 sc03 Full) (Mutations VL: I2F; M4L; A51P) | |
| SEQ ID NO: 100 | **Linker** | GGGGSGGGGSGGGGSGGGGS |
| SEQ ID NO: 101 | **scFv (VL-linker-VH)** | DIQMTQSPSSLSASVGDRVTITCQASQS INDYLAWYQQKPGKAPKLLIYKASTL ASGVPSRFSGSGSGTDFTLTISSLQPED FATYYCQQGYIITDIDNVFGTGTKVTV |
| | (33_03_G02 sc01) | |
| | (PRO830) | |
| | | LGGGGGSGGGGSGGGGSGGGGSQVQ LQESGPGLVKPSETLSLTCKVSGFSFSS GYDMCWIRQPPGKGLEWIGCVVAGSV DITYYASWAKGRVTISVDSSKNQFSLK LSSVTAADTAVYYCARKDAYSDAFNL WGQGTLVTVSS |
| SEQ ID NO: 102 | **scFv (VL-linker-VH)** | DFQLTQSPSSLSASVGDRVTITCQASQS INDYLAWYQQKPGKSPKLLIYKASTLA SGVPSRFSGSGSGTDFTLTISSLQPEDF ATYYCQQGYIITDIDNVFGTGTKVTVL GGGGGSGGGGSGGGGSGGGGSQSQL QESGPGLVKPSETLSLTCKASGFSFSSG YDMCWVRQPPGKGLEWIACVVAGSV DITYYASWAKGRVTISKDSSKNQVSLK LSSVTAADTAVYFCARKDAYSDAFNL WGQGTLVTVSS |
| | (33_03_G02 sc03 Full) | |

**TABLE 3. Examples of human serum albumin binding domains of the present invention.**

| **SEQ ID NUMBER** | **Ab region** | **Sequence** |
|---|---|---|
| **19-01-H04-sc03** | | |
| SEQ ID NO: 103 (AHo) | HCDR1 | ASGFSLSSNAMG |
| SEQ ID NO: 104 (AHo) | HCDR2 | ISVGGFTYYASWAKGR |
| SEQ ID NO: 105 (AHo) | HCDR3 | DRHGGDSSGAFYL |
| SEQ ID NO: 106 (Kabat) | HCDR1 | SNAMG |
| SEQ ID NO: 107 (Kabat) | HCDR2 | IISVGGFTYYASWAKG |
| SEQ ID NO: 108 (Kabat) | HCDR3 | DRHGGDSSGAFYL |
| SEQ ID NO: 109 (Chothia) | HCDR1 | AASGFSLSSNAMG |
| SEQ ID NO: 110 (Chothia) | HCDR2 | ISVGGFTYYAS |
| SEQ ID NO: 111 (Chothia) | HCDR3 | ARDRHGGDSSGAFYL |
| SEQ ID NO: 112 | VH | EVQLVESGGGLVQPGGSLRLSCAAS GFSLSSNAMGWVRQAPGKGLEYIGI ISVGGFTYYASWAKGRFTISRDNSK NTVYLQMNSLRAEDTATYFCARDR HGGDSSGAFYLWGQGTLVTVSS |
| SEQ ID NO: 113 (AHo) | LCDR1 | SSESVYSNNQLS |
| SEQ ID NO: 114 (AHo) | LCDR2 | DASDLASGVPSR |
| SEQ ID NO: 115 (AHo) | LCDR3 | GFSSSSDTA |
| SEQ ID NO: 116 (Kabat) | LCDR1 | QSSESVYSNNQLS |
| SEQ ID NO: 117 (Kabat) | LCDR2 | DASDLAS |
| SEQ ID NO: 118 (Kabat) | LCDR3 | AGGFSSSSDTA |
| SEQ ID NO: 119 (Chothia) | LCDR1 | QSSESVYSNNQLS |
| SEQ ID NO: 120 (Chothia) | LCDR2 | DASDLAS |
| SEQ ID NO: 121 (Chothia) | LCDR3 | AGGFSSSSDTA |
| SEQ ID NO: 122 | VL | DIQMTQSPSSLSASVGDRVTITCQSS ESVYSNNQLSWYQQKPGQPPKLLIY DASDLASGVPSRFSGSGSGTDFTLTI SSLQPEDFATYYCAGGFSSSSDTAF GGGTKLTVLG |
| SEQ ID NO: 123 | Linker | GGGGSGGGGSGGGGSGGGGS |
| SEQ ID NO: 124 | scFv (VL-linker-VH) | DIQMTQSPSSLSASVGDRVTITCQSS ESVYSNNQLSWYQQKPGQPPKLLIY DASDLASGVPSRFSGSGSGTDFTLTI SSLQPEDFATYYCAGGFSSSSDTAF GGGTKLTVLGGGGGSGGGGSGGGG SGGGGSEVQLVESGGGLVQPGGSLR LSCAASGFSLSSNAMGWVRQAPGK GLEYIGIISVGGFTYYASWAKGRFTI SRDNSKNTVYLQMNSLRAEDTATY FCARDRHGGDSSGAFYLWGQGTLV TVSS |
| **23-13-A01-sc03** | | |
| SEQ ID NO: 125 (AHo) | HCDR1 | ASGFSFSSSYWIC |
| SEQ ID NO: 126 (AHo) | HCDR2 | VFTGDGTTYYASWAKGR |
| SEQ ID NO: 127 (AHo) | HCDR3 | PVSVYYYGMDL |
| SEQ ID NO: 128 (Kabat) | HCDR1 | SSYWIC |
| SEQ ID NO: 129 (Kabat) | HCDR2 | CVFTGDGTTYYASWAKG |
| SEQ ID NO: 130 (Kabat) | HCDR3 | PVSVYYYGMDL |
| SEQ ID NO: 131 (Chothia) | HCDR1 | SGFSFSSSYWIC |
| SEQ ID NO: 132 (Chothia) | HCDR2 | VFTGDGTTYYAS |
| SEQ ID NO: 133 (Chothia) | HCDR3 | ARPVSVYYYGMDL |
| SEQ ID NO: 134 | VH | EVQLVESGGGLVQPGGSLRLSCAAS |
| | | GFSFSSSYWICWVRQAPGKGLEWV GCVFTGDGTTYYASWAKGRFTISRD NSKNTVYLQMNSLRAEDTATYFCA RPVSVYYYGMDLWGQGTLVTVSS |
| SEQ ID NO: 135 (AHo) | LCDR1 | ASQIISSRSA |
| SEQ ID NO: 136 (AHo) | LCDR2 | QASKLASGVPSR |
| SEQ ID NO: 137 (AHo) | LCDR3 | TYIDSNFGA |
| SEQ ID NO: 138 (Kabat) | LCDR1 | QASQIISSRSA |
| SEQ ID NO: 139 (Kabat) | LCDR2 | QASKLAS |
| SEQ ID NO: 140 (Kabat) | LCDR3 | QCTYIDSNFGA |
| SEQ ID NO: 141 (Chothia) | LCDR1 | QASQIISSRSA |
| SEQ ID NO: 142 (Chothia) | LCDR2 | QASKLAS |
| SEQ ID NO: 143 (Chothia) | LCDR3 | QCTYIDSNFGA |
| SEQ ID NO: 144 | VL | DVVMTQSPSSLSASVGDRVTITCQA SQIISSRSAWYQQKPGQPPKLLIYQA SKLASGVPSRFSGSGSGTDFTLTISSL QPEDFATYYCQCTYIDSNFGAFGGG TKLTVLG |
| SEQ ID NO: 145 | Linker | GGGGSGGGGSGGGGSGGGGS |
| SEQ ID NO: 146 | scFv (VL-linker-VH) | DVVMTQSPSSLSASVGDRVTITCQA SQIISSRSAWYQQKPGQPPKLLIYQA SKLASGVPSRFSGSGSGTDFTLTISSL QPEDFATYYCQCTYIDSNFGAFGGG TKLTVLGGGGGSGGGGSGGGGSGG GGSEVQLVESGGGLVQPGGSLRLSC AASGFSFSSSYWICWVRQAPGKGLE WVGCVFTGDGTTYYASWAKGRFTI SRDNSKNTVYLQMNSLRAEDTATY FCARPVSVYYYGMDLWGQGTLVT VSS |

**TABLE 4. Other sequences related to the present invention.**

| **SEQ ID NUMBER** | **Ab region** | **Sequence** |
|---|---|---|
| SEQ ID NO: 147 | Human CD137 | MGNSCYNIVATLLLVLNFERTRSLQDPC SNCPAGTFCDNNRNQICSPCPPNSFSSAG GQRTCDICRQCKGVFRTRKECSSTSNAE CDCTPGFHCLGAGCSMCEQDCKQGQEL TKKGCKDCCFGTFNDQKRGICRPWTNC SLDGKSVLVNGTKERDVVCGPSPADLS |
| | | PGASSVTPPAPAREPGHSPQIISFFLALTS TALLFLLFFLTLRFSVVKRGRKKLLYIFK QPFMRPVQTTQEEDGCSCRFPEEEEGGC EL |
| SEQ ID NO: 148 | Human PDL1 | MRIFAVFIFMTYWHLLNAFTVTVPKDL YVVEYGSNMTIECKFPVEKQLDLAALIV YWEMEDKNIIQFVHGEEDLKVQHSSYR QRARLLKDQLSLGNAALQITDVKLQDA GVYRCMISYGGADYKRITVKVNAPYNK INQRILVVDPVTSEHELTCQAEGYPKAE VIWTSSDHQVLSGKTTTTNSKREEKLFN VTSTLRINTTTNEIFYCTFRRLDPEENHT AELVIPELPLAHPPNERTHLVILGAILLC LGVALTFIFRLRKGRMMDVKKCGIQDT NSKKQSDTHLEET |
| SEQ ID NO: 149 | Vλ germline-based FR4 (Sk17) | FGTGTKVTVLG |
| SEQ ID NO: 150 | Vλ germline-based FR4 (Sk12) | FGGGTKLTVLG |
| SEQ ID NO: 151 | Vλ germline-based FR4 | FGGGTQLIILG |
| SEQ ID NO: 152 | Vλ germline-based FR4 | FGEGTELTVLG |
| SEQ ID NO: 153 | Vλ germline-based FR4 | FGSGTKVTVLG |
| SEQ ID NO: 154 | Vλ germline-based FR4 | FGGGTQLTVLG |
| SEQ ID NO: 155 | Vλ germline-based FR4 | FGGGTQLTALG |

**TABLE 5. Examples of multispecific molecules of the present invention.**

| **SEQ ID NUMBER** | **Ab Format** | **Sequence** |
|---|---|---|
| **PRO885** | | |
| 38-02-A04 sc01 scDb- i/33-03-G02 sc01 scDb-o | | |
| SEQ ID NO: 156 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP |
| | | GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS KNQFSLKLSSVTAADTAVYYCARK DAYSDAFNLWGQGTLVTVSS |
| **PRO951** | | |
| 38-27-C05 sc02 scDb-i/33-03-G02 sc01 scDb-o | | |
| SEQ ID NO: 157 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSEVQLVESGGGL VQPGGSLRLSCAASGFSFNNDYDM CWVRQAPGKGLEWIGCIDTGDGST YYASWAKGRFTISRDNSKNTVYLQ MNSLRAEDTAVYYCAREAASSSGY GMGYFDLWGQGTLVTVSSGGGGS GGGGSGGGGSGGGGSIQMTQSPSS LSASVGDRVTITCQSSQSVYDNNWL AWYQQKPGKAPKLLIYRASNLASG VPSRFSGSGSGTDFTLTISSLQPEDFA TYYCQGTYLSSNWYWAFGTGTKVT VLGGGGGSQVQLQESGPGLVKPSE TLSLTCKVSGFSFSSGYDMCWIRQP PGKGLEWIGCVVAGSVDITYYASW AKGRVTISVDSSKNQFSLKLSSVTA ADTAVYYCARKDAYSDAFNLWGQ GTLVTVSS |
| **PRO1123** | | |
| 38-02-A04 sc05 IF scDb-i/33 03 G02 sc01 scDb-o | | |
| SEQ ID NO: 158 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WVRQPPGKGLEWIGCTFVGSSDSTY YANWAKGRVTISVDSSKNQVSLKL SSVTAADTAVYFCARHPSDAVYGY ANNLWGQGTLVTVSSGGGGSGGG GSGGGGSGGGGSIQMTQSPSSLSA SVGDRVTITCQASQSINNVLAWYQQ KPGKPPKLLIYRASTLASGVPSRFSG SGSGTDFTLTISSLQPEDFATYYCQS SYGNYGDFGTGTKVTVLGGGGGSQ |
| | | VQLQESGPGLVKPSETLSLTCKVSG FSFSSGYDMCWIRQPPGKGLEWIGC VVAGSVDITYYASWAKGRVTISVDS SKNQFSLKLSSVTAADTAVYYCAR KDAYSDAFNLWGQGTLVTVSS |
| **PRO1124** | | |
| 38-02-A04 sc06 Full scDb-i/33_03_G02 sc01 scDb-o | | |
| SEQ ID NO: 159 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKASGFSFSNSYWIC WVRQPPGKGLEWIGCTFVGSSDSTY YANWAKGRVTISKDSSKNQVSLKL SSVTAADTAVYFCARHPSDAVYGY ANNLWGQGTLVTVSSGGGGSGGG GSGGGGSGGGGSLQMTQSPSSLSA SVGDRVTITCQASQSINNVLAWYQQ KPGKPPKLLIYRASTLASGVPSRFSG SGSGTDFTLTISSLQPEDFATYYCQS SYGNYGDFGTGTKVTVLGGGGGSQ VQLQESGPGLVKPSETLSLTCKVSG FSFSSGYDMCWIRQPPGKGLEWIGC VVAGSVDITYYASWAKGRVTISVDS SKNQFSLKLSSVTAADTAVYYCAR KDAYSDAFNLWGQGTLVTVSS |
| **PRO1125** | | |
| 38-02-A04 sc01 scDb-i/33_03_G02 sc02 IF scDb-o | | |
| SEQ ID NO: 160 | scDb | DIQMTQSPSSLSASVGDRVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS |
| | | FSSGYDMCWVRQPPGKGLEWIACV VAGSVDITYYASWAKGRVTISVDSS KNQFSLKLSSVTAADTAVYFCARK DAYSDAFNLWGQGTLVTVSS |
| **PRO1126** | | |
| 38-02-A04 sc01 scDb-i/33_03_G02 sc03 Full scDb-o | | |
| SEQ ID NO: 161 | scDb | DFQLTQSPSSLSASVGDRVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQSQ LQESGPGLVKPSETLSLTCKASGFSF SSGYDMCWVRQPPGKGLEWIACVV AGSVDITYYASWAKGRVTISKDSSK NQVSLKLSSVTAADTAVYFCARKD AYSDAFNLWGQGTLVTVSS |
| **PRO1134** | | |
| 38-02-A04 sc01 scDb-i/33_03_G02 sc07 GL VH3 scDb-o | | |
| SEQ ID NO: 162 | scDb | DIQMTQSPSSLSASVGDAVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSEV QLVESGGGLVQPGGSLRLSCAASGF |
| | | SFSSGYDMCWVRQAPGKGLEWVG CVVAGSVDITYYASWAKGRFTISRD NSKNTVYLQMNSLRAEDTATYYCA RKDAYSDAFNLWGPGTLVTVSS |
| **PRO963** (= **PRO1051)** | | |
| 38_02_A04 sc01 scDb-i/33-03-G02 sc01 scDb-o/19-01-H04-sc03 scFv | | |
| SEQ ID NO: 163 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS KNQFSLKLSSVTAADTAVYYCARK DAYSDAFNLWGQGTLVTVSSGGG GSGGGGSIQMTQSPSSLSASVGDRV TITCQSSESVYSNNQLSWYQQKPGQ PPKLLIYDASDLASGVPSRFSGSGSG TDFTLTISSLQPEDFATYYCAGGFSS SSDTAFGGGTKLTVLGGGGGSGGG GSGGGGSGGGGSEVQLVESGGGL VQPGGSLRLSCAASGFSLSSNAMG WVRQAPGKGLEYIGIISVGGFTYYA SWAKGRFTISRDNSKNTVYLQMNS LRAEDTATYFCARDRHGGDSSGAF YLWGQGTLVTVSS |
| **PRO966 (= PRO1052)** | | |
| 38_27_C05 sc01 scDb-i/33-03-G02 sc01 scDb-o/19-01-H04-sc03 scFv | | |
| SEQ ID NO: 164 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL |
| | | VKPSETLSLTCKVSGFSFNNDYDMC WIRQPPGKGLEWIGCIDTGDGSTYY ASWAKGRVTISVDSSKNQFSLKLSS VTAADTAVYYCAREAASSSGYGMG YFDLWGQGTLVTVSSGGGGSGGG GSGGGGSGGGGSIQMTQSPSSLSA SVGDRVTITCQSSQSVYDNNWLAW YQQKPGKAPKLLIYRASNLASGVPS RFSGSGSGTDFTLTISSLQPEDFATY YCQGTYLSSNWYWAFGTGTKVTVL GGGGGSQVQLQESGPGLVKPSETL SLTCKVSGFSFSSGYDMCWIRQPPG KGLEWIGCVVAGSVDITYYASWAK GRVTISVDSSKNQFSLKLSSVTAAD TAVYYCARKDAYSDAFNLWGQGT LVTVSSGGGGSGGGGSIQMTQSPS SLSASVGDRVTITCQSSESVYSNNQL SWYQQKPGQPPKLLIYDASDLASGV PSRFSGSGSGTDFTLTISSLQPEDFAT YYCAGGFSSSSDTAFGGGTKLTVLG GGGGSGGGGSGGGGSGGGGSEV QLVESGGGLVQPGGSLRLSCAASGF SLSSNAMGWVRQAPGKGLEYIGIIS VGGFTYYASWAKGRFTISRDNSKNT VYLQMNSLRAEDTATYFCARDRHG GDSSGAFYLWGQGTLVTVSS |
| **PRO1057** | | |
| 38_02_A04 sc01 scDb-i/33-03-G02 sc01 scDb-o/mxr HSA (23-13-A01-sc03, sk17sh4) | | |
| SEQ ID NO: 165 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS |
| | | KNQFSLKLSSVTAADTAVYYCARK DAYSDAFNLWGQGTLVTVSSGGG GSGGGGSVVMTQSPSSLSASVGDR VTITCQASQIISSRSAWYQQKPGQPP KLLIYQASKLASGVPSRFSGSGSGTD FTLTISSLQPEDFATYYCQCTYIDSN FGAFGGGTKLTVLGGGGGSGGGG SGGGGSGGGGSEVQLVESGGGLV QPGGSLRLSCAASGFSFSSSYWICW VRQAPGKGLEWVGCVFTGDGTTYY ASWAKGRFTISRDNSKNTVYLQMN SLRAEDTATYFCARPVSVYYYGMD LWGQGTLVTVSS |
| **PRO1058** | | |
| 38_27_C05 sc01 scDb-i/33-03-G02 sc01 scDb-o/mxr HSA (23-13-A01-sc03, sk17sh4) | | |
| SEQ ID NO: 166 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFNNDYDMC WIRQPPGKGLEWIGCIDTGDGSTYY ASWAKGRVTISVDSSKNQFSLKLSS VTAADTAVYYCAREAASSSGYGMG YFDLWGQGTLVTVSSGGGGSGGG GSGGGGSGGGGSIQMTQSPSSLSA SVGDRVTITCQSSQSVYDNNWLAW YQQKPGKAPKLLIYRASNLASGVPS RFSGSGSGTDFTLTISSLQPEDFATY YCQGTYLSSNWYWAFGTGTKVTVL GGGGGSQVQLQESGPGLVKPSETL SLTCKVSGFSFSSGYDMCWIRQPPG KGLEWIGCVVAGSVDITYYASWAK GRVTISVDSSKNQFSLKLSSVTAAD TAVYYCARKDAYSDAFNLWGQGT LVTVSSGGGGSGGGGSVVMTQSPS SLSASVGDRVTITCQASQIISSRSAW YQQKPGQPPKLLIYQASKLASGVPS RFSGSGSGTDFTLTISSLQPEDFATY YCQCTYIDSNFGAFGGGTKLTVLGG GGGSGGGGSGGGGSGGGGSEVQL VESGGGLVQPGGSLRLSCAASGFSF SSSYWICWVRQAPGKGLEWVGCVF TGDGTTYYASWAKGRFTISRDNSK NTVYLQMNSLRAEDTATYFCARPV SVYYYGMDLWGQGTLVTVSS |
| **PRO1059** | | |
| 33-03-G02 IgG1 LC with 38_02_A04 sc01 scFV, PDL1/CD137(scFv) silent Morrison | | |
| SEQ ID NO: 167 | Morrison-L Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGECGGGGSGGGG SIQMTQSPSSLSASVGDRVTITCQAS QSINNVLAWYQQKPGKAPKLLIYRA STLASGVPSRFSGSGSGTDFTLTISSL QPEDFATYYCQSSYGNYGDFGTGT KVTVLGGGGGSGGGGSGGGGSG GGGSQVQLQESGPGLVKPSETLSLT CKVSGFSFSNSYWICWIRQPPGKGL EWIGCTFVGSSDSTYYANWAKGRV TISVDSSKNQFSLKLSSVTAADTAV YYCARHPSDAVYGYANNLWGQGT LVTVSS |
| SEQ ID NO: 168 | Morrison-L Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGK |
| **PRO1060** | | |
| 33-03-G02 IgG1 HC with 38_02 A04 sc01 scFV, | | |
| PDL1/CD137(scFv) silent Morrison | | |
| SEQ ID NO: 169 | Morrison-H Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGEC |
| SEQ ID NO: 170 | Morrison-H Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGKGGGGSGGGGSIQMTQSPSSLS ASVGDRVTITCQASQSINNVLAWYQ QKPGKAPKLLIYRASTLASGVPSRFS GSGSGTDFTLTISSLQPEDFATYYCQ SSYGNYGDFGTGTKVTVLGGGGGS GGGGSGGGGSGGGGSQVQLQESG PGLVKPSETLSLTCKVSGFSFSNSY WICWIRQPPGKGLEWIGCTFVGSSD STYYANWAKGRVTISVDSSKNQFSL KLSSVTAADTAVYYCARHPSDAVY GYANNLWGQGTLVTVSS |
| **PRO1061** | | |
| 33-03-G02 sc01 IgG1 LC with 38_27_C05 sc01 scFv, PDL1/CD137(scFv) silent Morrison | | |
| SEQ ID NO: 171 | Morrison-L Light chain | DIQMTQSPSSLSASVGDRVTITCQAS QSINDYLAWYQQKPGKAPKLLIYK ASTLASGVPSRFSGSGSGTDFTLTISS |
| | | LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGECGGGGSGGGG SIQMTQSPSSLSASVGDRVTITCQAS QSINNVLAWYQQKPGKAPKLLIYRA STLASGVPSRFSGSGSGTDFTLTISSL QPEDFATYYCQSSYGNYGDFGTGT KVTVLGGGGGSGGGGSGGGGSG GGGSQVQLQESGPGLVKPSETLSLT CKVSGFSFSNSYWICWIRQPPGKGL EWIGCTFVGSSDSTYYANWAKGRV TISVDSSKNQFSLKLSSVTAADTAV YYCARHPSDAVYGYANNLWGQGT LVTVSS |
| SEQ ID NO: 172 | Morrison-L Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGK |
| **PRO1062** | | |
| 33-03-G02 sc01 IgG1 HC with 38_27_C05 sc01 scFv, PDL1/CD137(scFv) silent Morrison | | |
| SEQ ID NO: 173 | Morrison-H Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL |
| | | SSTLTLSKADYEKHKVYACEVTHQ GLSSPVTKSFNRGEC |
| SEQ ID NO: 174 | Morrison-H Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGKGGGGSGGGGSIQMTQSPSSLS ASVGDRVTITCQASQSINNVLAWYQ QKPGKAPKLLIYRASTLASGVPSRFS GSGSGTDFTLTISSLQPEDFATYYCQ SSYGNYGDFGTGTKVTVLGGGGGS GGGGSGGGGSGGGGSQVQLQESG PGLVKPSETLSLTCKVSGFSFSNSY WICWIRQPPGKGLEWIGCTFVGSSD STYYANWAKGRVTISVDSSKNQFSL KLSSVTAADTAVYYCARHPSDAVY GYANNLWGQGTLVTVSS |

Throughout the text of this application, should there be a discrepancy between the text of the specification (e.g., Tables 1 to 5) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### Example 1: Affinities to PDL1, CD137, HSA and MSA.

### Methods:

Affinity to PD-L1 of the different species was determined by SPR measurements using a Biacore T200 device (GE Healthcare). An antibody specific for the Fc region of human IgGs was immobilized on a sensor chip (CM5 sensor chip, GE Healthcare) by amine-coupling. For all formats, with the exception of the Fc containing Morrison formats, PD-L1-Fc chimeric protein from different species were captured by the immobilized antibody. Three-fold serial dilutions of the molecules specific for PD-L1 (0.12-90 nM) were injected into the flow cells for three minutes and dissociation was monitored for 10 minutes. After each injection cycle, surfaces were regenerated with one injection of a 3 M MgCl₂ solution. The apparent dissociation (k_{d}) and association (kₐ) rate constants and the apparent dissociation equilibrium constant (KD) were calculated using one-to-one Langmuir binding model. Affinity to CD137 of the different species was determined using the identical setup as for PD-L1 with the exception that CD137-Fc chimeric protein from different species were captured by the immobilized antibody.

The Fc containing formats were directly captured by the antibody specific for the Fc region of human IgGs. Two-fold serial dilutions of PD-L1 extracellular domain or CD137 extracellular domain ranging from 90 to 0.35 nM were tested for binding to the IgG captured on the biosensor chip. After each injection cycle, surfaces were regenerated with one injection of a 3 M MgCl2 solution.

Affinity of molecules to serum albumin (SA) of the different species was determined by SPR measurements using a Biacore T200 device (GE Healthcare). SA was directly coupled to a CM5 sensor chip (GE Healthcare) using amine coupling chemistry. After performing a regeneration scouting and surface performance test to find best assay conditions, a dose response was measured and obtained binding curves were double-referenced (empty reference channel and zero analyte injection) and fitted using the 1:1 Langmuir model to retrieve kinetic parameters. The assay was run in a 1 X PBS-Tween buffer at pH 5.5.

### Results:

The measurements of the binding kinetics for the humanized constructs show a difference in binding affinity for PD-L1 when comparing the CDR and structural (STR) grafts of clone 33-03-G02 the STR graft shows a 20-fold improvement in affinity compared to the CDR graft of the same clone (PRO885 versus PRO1126 in Table 6). The CDR graft derived from clone 37-20-B03 (PRO997) shows approximately two-fold higher affinity when compared to the STR graft of clone 33-03-G02. The binding affinities for the CDR graft of 33-03-G02 are similar to the binding affinity of the parental scFv when they are combined into different multispecific formats (compare PRO830 to PRO885, PRO951, PRO1123, PRO1124, PRO963, PRO966, PRO1057, PRO1058, PRO1059 and PRO1060 in Table 6). The scFv derived from both clones show nearly identical affinity to human and cynomolgus monkey PD-L1 (see PRO977 and PRO830 in Table 6).

The measurement of binding kinetics for the CDR grafts of the two CD137 specific humanized constructs derived from clone 38-02-A04 and 38-27-C05 show nearly identical affinities (compare PRO885 and PRO951 in Table 6). For clone 38-02-04 the described structural residues engrafted in the framework regions led to an improvement of affinity of more than 200-fold (compare PRO885 and PRO1124 in Table 6). In addition, for constructs derived from clon 38-02-04 binding to mouse CD137 was observed, however with very much reduced affinity.
Binding of triabodies to serum albumin (SA) was confirmed by SPR. The triabodies containing the SA binding domain derived from clone 19-01-H04 show high affinity binding to human serum albumin, while no binding was observed to rodent SA. For the triabodies containing clone 23-13-A01 binding was observed for human SA, in addition the molecules bind with reduced affinity to rodent SA (see Table 6).

**TABLE 6 Affinities of different formats to PD-L1, CD137 and serum albumin from different species.**

| | | | | | **Affinity to human PD-L1** | | | **Affinity to cyno PD-L1** | | | **Affinity to human CD137** | | | **Affinity to mouse CD137** | | | **Affinity to human SA** | | | **Affinity to mouse SA** | | |
|---|---|---|---|---|---|---|---|---|---|---|---|---|---|---|---|---|---|---|---|---|---|---|
| PRO ID | Clone ID PD-L1 | Clone ID CD137 | Clone ID SA | Format | kₐ(M⁻¹s⁻¹) | k_{d}(s⁻¹) (₅⁻¹) | KD(M) | kₐ(M⁻¹s⁻¹) | k_{d}(s⁻¹) | KD(M) | kₐ(M⁻¹s⁻¹) | k_{d}(s⁻¹) | KD(M) | kₐ(M⁻¹s⁻¹) | k_{d}(s⁻¹) | KD(M) | kₐ(M⁻¹s⁻¹) | k_{d}(s⁻¹) | KD(M) | kₐ(M⁻¹s⁻¹) | k_{d}(s⁻¹) | KD(M) |
| PRO885 | 33-03-G02 CDR | 38-02-A04 CDR | NA | scDb | 2.1E+06 | 1.4E-04 | 6.5E-11 | ND | ND | ND | 2.4E+05 | 7.6E-04 | 3.2E-09 | 2.9E+05 | 1.8E-01 | 6.0E-07 | NA | NA | NA | NA | NA | NA |
| PRO951 | 33-03-G02 CDR | 38-27-C05 CDR | NA | scDb | 2.2E+06 | 1.5E-04 | 7.0E-11 | ND | ND | ND | 1.5E+06 | 6.3E-03 | 4.2E-09 | NB | NB | NB | NA | NA | NA | NA | NA | NA |
| PRO11 23 | 33-03-G02 CDR | 38-02-A04 IF | NA | scDb | 2.3E+06 | 1.7E-04 | 7.5E-11 | ND | ND | ND | 3.1E+05 | 2.7E-04 | 8.8E-10 | 6.5E+04 | 2.7E-02 | 4.1E-07 | NA | NA | NA | NA | NA | NA |
| PRO1124 | 33-03-G02 CDR | 38-02-A04 STR | NA | scDb | 3.1E+06 | 2.0E-04 | 6.7E-11 | ND | ND | ND | 6.8E+05 | < 1.0E-05 | 1.5E-11 | 2.0E+05 | 2.2E-03 | 1.1E-08 | NA | NA | NA | NA | NA | NA |
| PRO1125 | 33-03-G02 IF | 38-02-A04 CDR | NA | scDb | 1.7E+06 | 1.1E-04 | 6.7E-11 | ND | ND | ND | 2.0E+05 | 7.5E-04 | 3.7E-09 | 5.4E+05 | 2.7E-01 | 5.1E-07 | NA | NA | NA | NA | NA | NA |
| PRO1126 | 33-03-G02 STR | 38-02-A04 CDR | NA | scDb | 2.8E+06 | < 1.0E-05 | 3.5E-12 | ND | ND | ND | 2.1E+05 | 7.5E-04 | 3.5E-09 | | | | NA | NA | NA | NA | NA | NA |
| PRO1134 | 33-03-G02 STR2, VH3 | 38-02-A04 CDR | NA | scDb | 2.8E+06 | 7.6E-05 | 2.8E-11 | ND | ND | ND | 2.5E+05 | 8.4E-04 | 3.4E-09 | 4.5E+05 | 1.9E-01 | 4.2E-07 | NA | NA | NA | NA | NA | NA |
| | | | | | | | | | | | | | | | | | | | | | | |
| PRO963 | 33-03-G02 CDR | 38-02-A04 CDR | 19-01-H04 STR | Triabody | 2.0E+06 | 1.3E-04 | 6.6E-11 | ND | ND | ND | 2.0E+05 | 6.2E-04 | 3.0E-09 | 3.5E+05 | 5.9E-02 | 1.7E-07 | 1.1E+05 | 3.0E-04 | 2.8E-09 | NB | NB | NB |
| PRO966 | 33-03-G02 CDR | 38-27-C05 CDR | 19-01-H04 STR | Triabody | 1.6E+06 | 1.4E-04 | 8.3E-11 | ND | ND | ND | 1.0E+06 | 2.2E-03 | 2.2E-09 | | | | ND | ND | ND | NA | NA | NA |
| PRO1057 | 33-03-G02 CDR | 38-02-A04 CDR | 23-13-A01 STR | Triabody | 1.6E+06 | 1.7E-04 | 1.1E-10 | ND | ND | ND | 1.4E+05 | 7.0E-04 | 5.1E-09 | 6.9E+04 | 8.5E-02 | 1.2E-06 | 2.4E+05 | 6.7E-04 | 2.8E-09 | 1.3E+05 | 8.5E-03 | 6.6E-08 |
| PRO1058 | 33-03-G02 CDR | 38-27-C05 CDR | 23-13-A01 STR | Triabody | 1.2E+06 | 1.9E-04 | 1.6E-10 | ND | ND | ND | 1.7E+06 | 2.1E-03 | 1.2E-09 | | | | ND | ND | ND | ND | ND | ND |
| | | | | | | | | | | | | | | | | | NA | NA | NA | NA | NA | NA |
| PRO1059 | 33-03-G02 CDR | 38-02-A04 CDR | NA | Morrison-L | 1.2E+06 | 6.5E-05 | 5.6E-11 | ND | ND | ND | 1.8E+05 | 4.2E-04 | 2.3E-09 | | | | NA | NA | NA | NA | NA | NA |
| PRO1060 | 33-03-G02 CDR | 38-02-A04 CDR | NA | Morrison-H | 1.3E+06 | 4.6E-05 | 3.6E-11 | ND | ND | ND | 3.0E+05 | 3.9E-04 | 1.3E-09 | | | | NA | NA | NA | NA | NA | NA |
| PRO1061 | 33-03-G02 CDR | 38-27-C05 CDR | NA | Morrison-L | ND | ND | ND | ND | ND | ND | ND | ND | ND | ND | ND | ND | NA | NA | NA | NA | NA | NA |
| PRO1062 | 33-03-G02 CDR | 38-27-C05 CDR | NA | Morrison-H | 1.3E+06 | 5.0E-05 | 3.8E-11 | ND | ND | ND | 2.8E+05 | 3.7E-04 | 1.3E-09 | | | | NA | NA | NA | NA | NA | NA |
| | | | | | | | | | | | | | | | | | | | | | | |
| PRO997 | 37-20-B03 CDR | NA | NA | scFv | 5.9E+06 | <1.0E-05 | 1.7E-12 | 6.0E+06 | <1.0E-05 | 1.7E-12 | NA | NA | NA | NA | NA | NA | NA | NA | NA | NA | NA | NA |
| PRO1013 | 37-20-B03 CDR, VH1 | NA | NA | scFv | 6.0E+06 | 2.7E-04 | 4.5E-11 | 5.9E+06 | 3.2E-04 | 5.3E-11 | NA | NA | NA | NA | NA | NA | NA | NA | NA | NA | NA | NA |
| PRO830 | 33-03-G02 CDR | NA | NA | scFv | 2.1E+06 | 1.6E-04 | 7.6E-11 | 2.2E+06 | 2.0E-04 | 9.4E-11 | NA | NA | NA | NA | NA | NA | NA | NA | NA | NA | NA | NA |

| | | | | | | | | | | | | | | | | | | | | | | |
|---|---|---|---|---|---|---|---|---|---|---|---|---|---|---|---|---|---|---|---|---|---|---|
| NA: not applicable TBD: to be determined NB: no significant binding ND: not determined | | | | | | | | | | | | | | | | | | | | | | |

### Example 2: Blockade of the PD-L1/PD-1 interaction in a cell-based reporter gene assay using CHO cells expressing PD-L1 and a TCR activator molecule, and Jurkat cells expressing PD-1 and containing a luciferase gene under the NFAT response element.

### Methods

In the bioluminescent reporter gene assay, engineered Jurkat T cells stably expressing NFAT (nuclear factor of activated T-cells)-luciferase reporter and human PD-1 act as effector T cells. Cells stably expressing human PD-L1 and a T cell receptor (TCR) activator act as antigen presenting cells. Co-cultivating the two cell lines induces activation of the Jurkat NFAT pathway via crosslinking of TCR activator/TCR complex. Upon engagement of PD-L1 expressing cells, PD-1 signaling in PD-1 effector T cells inhibits T-cell function, and results in NFAT pathway inhibition. Blockade of PD-1 and PD-L1 receptor interaction leads to re-activation of the NFAT pathway.

35'000 CHO/PD-L1/TCR activator (BPS Bioscience) cells in 100 µl of cell culture medium (DMEM/F12, 10% FCS) were added to the inner wells of a white cell culture plate and incubated for 16-20h at 37°C and 5% CO₂. Next day, 95 µl of cell culture medium was removed from each well and 50 µl of 2-fold concentrated serial dilutions of the respective molecules to be tested (from 3000 to 0.46 ng/ml), including the reference Avelumab, were added. Then, 50 µl of effector Jurkat cells expressing PD-1 (BPS Bioscience) diluted at 400'000 cell/ml in assay buffer (RPMI1640 with 10% FCS) were added to each well and plates were incubated 6h at 37°C and 5% CO₂. Finally, 50 µL luciferase substrate (BPS Bioscience) prepared according to manufacturer's protocol, was added per well and plates were incubated 30 min in the dark, luminescence was measured using Topcount.

### Results

In order to assess the influence of the CDR set and framework selection on potency to neutralize the PD-L1 binding to PD-1, three anti-PD-Ll scFvs were tested in the NFAT reporter gene cell-based assay. PRO830 comprises the CDR set of clone 33-03-G02 grafted on a VH4 framework and PRO997 and PRO1013 comprise the CDR set of clone 37-20-B03 grafted on either a VH4 or a VH1 framework, respectively. PRO830 has the lowest potency of the three scFvs tested with an IC₅₀ value of 42.88 ng/ml, and has similar potency as Avelumab with an IC₅₀ value of 34.09 ng/ml. PRO997 is the most potent molecule. Potency of the same CDR set was about 2-fold higher when grafted on a VH4 framework than on VH1 framework. IC₅₀ values were 11,12 ng/ml versus 21.29 ng/ml, respectively. (Figure 3 and Table 7)

Neutralization potency of the PD-L1 binding to PD-1 was determined for bi-specific molecules possessing the 33-03-G02 PD-L1 domain before (CDR graft) and after (structural graft) domain optimization. The CDR graft (PRO885) was compared to a structural graft (PRO1126). The domain optimization improved the neutralization potency by a factor of three with IC₅₀ values being 137.2 ng/ml for PRO885 and 48.15 ng/ml for PRO1126. (Figure 4 and Table 7).

Potency to neutralize the PD-L1/PD-1 interaction was also assessed for two tri-specific molecules possessing the anti-PD-Ll domain of the CDR graft of clone 33-03-G02 and two different human serum albumin binding domain, for half-life extension. The HSA domain of PRO1057 is also binding mouse serum albumin. Experiments were performed in presence of 25 mg/ml HSA. Neutralization potency (IC₅₀ = 665.1 ng/ml) was lower than for Avelumab. (Figure 5 and Table 7)

Another format which would extend the half-life in serum, the so-called Morrison format, was tested in the cell based potency reporter gene assay. In this format, one specificity is carried by the IgG moiety (bi-valency) and two scFvs with specificities to the second target are linked by flexible peptide linkers either to the heavy chain (HC) or light chain (LC) of the IgG. All Morrison molecules tested carried the anti-PD-Ll domain of the CDR graft of clone 33-03-G02 on both IgG arms. The two constructs PRO1059 and PRO1060 differ by the fusion of two anti-CD137 scFvs either on the heavy chain (HC) or to the (LC),. PRO1062 has the same architecture as PRO1060 with a different CD137 domain. Neutralization potencies of all molecules were similar (Figure 6 and Table 7).

**TABLE 7 Neutralization of PD-L1 PD-1 interaction in the NF-AT reporter gene assay.**

| | | | | | **Neutralization of PD-L1 in NF-AT Potency assay** | | |
|---|---|---|---|---|---|---|---|
| PRO ID | Clone ID PD-L1 | Clone ID CD137 | Clone ID SA | Format | IC₅₀ (ng/ml) | rel. IC₅₀* | HSA |
| PRO885 | 33-03-G02 CDR | 38-02-A04 CDR | NA | scDb | 137.20 | 0.28 | no |
| PRO951 | 33-03-G02 CDR | 38-27-C05 CDR | NA | scDb | 88.50 | 0.47 | no |
| | | | | | | | |
| PRO963 | 33-03-G02 CDR | 38-02-A04 CDR | 19-01-H04 STR | Triabody | 274.80 | 0.25 | yes |
| PRO1057 | 33-03-G02 CDR | 38-02-A04 CDR | 23-13-A01 STR | Triabody | 665.10 | 0.10 | yes |
| | | | | | | | |
| PRO1059 | 33-03-G02 CDR | 38-02-A04 CDR | NA | Morrison-L | 93.76 | 0.52 | no |
| PRO1060 | 33-03-G02 CDR | 38-02-A04 CDR | NA | Morrison-H | 132.70 | 0.44 | no |
| PRO1062 | 33-03-G02 CDR | 38-27-C05 CDR | NA | Morrison-H | 96.55 | 0.68 | no |
| | | | | | | | |
| PRO997 | 37-20-B03 CDR | NA | NA | scFv | 11.12 | 3.07 | no |
| PRO1013 | 37-20-B03 CDR, VH1 | NA | NA | scFv | 21.29 | 1.60 | no |
| PRO830 | 33-03-G02 CDR | NA | NA | scFv | 42.88 | 0.73 | no |

| | | | | | | | |
|---|---|---|---|---|---|---|---|
| NA: not applicable *: IC₅₀, Avelumab (ng/ml)/IC_{50,test molecule}(ng/ml) | | | | | | | |

### Example 3: Blockade of the interaction of PD-L1 with PD-1 and B7.1 using competition ELISA.

These assays were performed to assess the ability of PD-L1 inhibitors to block the interaction between PD-L1 and PD-1 or PD-L1 and B.71. Different formats including scFvs, scDbs, scDb-scFv and Morrison were analyzed in the competition ELISA and compared to the reference IgG Avelumab.

### PD-L1/PD-1 competition ELISA

### Method

ELISA microplates coated overnight at 4°C with 4 µg/ml human PD-1 were washed three times with 450 µl wash buffer per well. Plates were blocked for 1 hour at room temperature by adding 300 µl of PBS with 1%BSA and 0.2% tween (dilution buffer) to each well. Inhibitors were serially diluted in 3-fold steps to final concentrations ranging from 300 to 0.005 ng/ml in dilution buffer containing 1 ng/ml biotinylated human PD-L1. The mixtures were pre-incubated for 1 hour at room temperature under gentle agitation on a rotating mixer (21 rpm) and added to the microplates after 3 wash cycles with 450 µl wash buffer per well. Plates were incubated for 1.5 hours at room temperature under gentle agitation, then 10 ng/ml streptavidin-polyHRP40 was added to each microplate well after three washes with 450 µl of wash buffer per well. After 1h incubation at RT, plates were washed three times with 450 µl wash buffer and TMB substrate solution was added. The enzymatic reaction was stopped after 6 minutes by addition of 1M HCl and absorbance was measured at 450 nm using 690 nm as a reference wavelength. For calculation of IC₅₀ values, a four-parameter logistic (4PL) curve fit was performed in Graph Pad Prism using reference subtracted values.

### Results

As illustrated in Figure 7, all PD-L1 inhibitors blocked the interaction of PD-1 with PD-L1 when tested in the competition ELISA. The scFv PRO830 blocked the interaction with similar potency while PRO997 and PRO1013 exhibited significantly lower IC₅₀ values than Avelumab and are thus more potent inhibitors. When combined into multispecific formats, i.e. scDbs or Morrisons, all molecules conserved their inhibiting properties. PRO885 was less potent than Avelumab whereas a lower IC₅₀ value was determined for PRO1126 comprising an improved anti-PD-Ll domain. The Morrison formats were slightly less potent when compared to Avelumab. The neutralizing effect of PRO1057 was also shown in presence of human serum albumin, where IC₅₀ values were approximately two-fold higher.

### PD-L1/B7.1 competition ELISA

### Method

ELISA microplates coated overnight at 4°C with 4 µg/ml human B7.1 were washed three times with 450 µl wash buffer per well. Plates were blocked for 1 hour at room temperature by adding 300 µl of PBS with 1%BSA and 0.2% tween (dilution buffer) to each well. Inhibitors were serially diluted in 3-fold steps to final concentrations ranging from 900 to 0.015 ng/ml in dilution buffer containing 40 ng/ml biotinylated PD-L1. The mixtures were pre-incubated for 1 hour at room temperature under gentle agitation on a rotating mixer (21 rpm) and added to the microplates after 3 wash cycles with 450 µl wash buffer per well. Plates were incubated for 1.5 hours at room temperature under gentle agitation, then 10 ng/ml streptavidin-polyHRP40 was added to each microplate well after three washes with 450 µl of wash buffer per well. After 1h incubation at RT, plates were washed three times with 450 µl wash buffer and TMB substrate solution was added. The enzymatic reaction was stopped after 6 minutes by addition of 1M HCl and absorbance was measured at 450 nm using 690 nm as a reference wavelength. For calculation of IC₅₀ values, a four-parameter logistic (4PL) curve fit was performed in Graph Pad Prism using reference subtracted values.

### Results

Except for PRO 1126, all PD-L1 inhibitors were also tested for their ability to block the interaction of PD-1 with B7.1. PRO830 showed similar potency than Avelumab whereas lower IC₅₀ values were determined for PRO997 and PRO1013. All scDbs and Morrisons also inhibited the interaction between PD-L1 and B.7-1. The scDb PRO885 exhibited similar potency than Avelumab whereas the IC₅₀ values for the Morrisons were about 2 -3.4 fold lower.

### Example 4: No inhibition of CD137 and CD137 neutralization by humanized anti-CD137 domains.

### Methods:

To show that PRO885 does not interfere with the binding of CD137 ligand (CD137L) to CD137, a competitive ELISA was employed. The commercial inhibitory polyclonal anti-CD137 goat antibody (Antibodies online, Cat# ABIN636609) served as a reference. In brief, CD137 was coated on the ELISA plate overnight and serial dilutions of PRO885 were added to the ELISA plate. Afterwards, biotinylated CD137L was added and bound ligand was detected by addition of Streptavidin-HRP. Finally, the HRP substrate TMB was added. After development for 5 min, the reaction was stopped with 1 M HCl solution. The absorbance was measured at 450 nm and 690 nm as reference.

### Results:

The titration curves obtained for PRO885 containing the CD137 domain derived from clone 38-02-A04 are represented in Figure 9A and the binding curves obtained for PRO951 containing the CD137 domain derived from clone 38-27-C05 are represented in Figure 9B. While the reference antibody completely prevented binding of CD137L to CD137, PRO885 and PRO951 did not significantly inhibit the CD137L binding to CD137 and therefore were defined as non-neutralizing.

**TABLE 8 Blockade of the interaction of PD-L1 with PD-1 and B7.1 using competition ELISA.**

| | | | | | **Blocking of PD-L1/ PD-1 interaction** | | **Blocking of PD-L1/ B7.1 interaction** | |
|---|---|---|---|---|---|---|---|---|
| PRO ID | Clone ID PD-L1 | Clone ID CD137 | Clone ID SA | Format | IC₅₀ (ng/ml) | rel. IC₅₀* | IC₅₀ (ng/ml) | rel. IC₅₀* |
| PRO885 | 33-03-G02 CDR | 38-02-A04 CDR | NA | scDb | 8.35 | 0.17 | 12.2 | 0.59 |
| PRO951 | 33-03-G02 CDR | 38-27-C05 CDR | NA | scDb | 9.50 | 0.15 | 9.30 | 0.78 |
| PRO1126 | 33-03-G02 STR | 38-02-A04 CDR | NA | scDb | 1.28 | 1.59 | TBD | TBD |
| | | | | | | | | |
| PRO1057 | 33-03-G02 CDR | 38-02-A04 CDR | 23-13-A01 STR | Triabody | 8.61 | 0.20 | 16.29 | 0.53 |
| | | | | | | | | |
| PRO 1059 | 33-03-G02 CDR | 38-02-A04 CDR | NA | Morrison-L | 4.54 | 0.37 | 28.98 | 0.30 |
| PRO 1060 | 33-03-G02 CDR | 38-02-A04 CDR | NA | Morrison-H | 5.67 | 0.30 | 17.42 | 0.49 |
| PRO 1062 | 33-03-G02 CDR | 38-27-C05 CDR | NA | Morrison-H | 11.33 | 0.32 | 19.53 | 0.51 |
| | | | | | | | | |
| PRO997 | 37-20-B03 CDR | NA | NA | scFv | 0.50 | 4.16 | 6.359 | 2.34 |
| PRO1013 | 37-20-B03 CDR, VH1 | NA | NA | scFv | 0.57 | 3.67 | 4.05 | 3.68 |
| PRO830 | 33-03-G02 CDR | NA | NA | scFv | 3.40 | 0.61 | 12.87 | 1.16 |

| | | | | | | | | |
|---|---|---|---|---|---|---|---|---|
| NA: not applicable *: IC_{50,Avelumab} (ng/ml)/IC_{50,test molecule} (ng/ml) | | | | | | | | |

### Example 5: Epitope binning of 38-02-A04 and 38-27-C05 against Urelumab and Utolimumab.

### Methods:

The binding epitopes on CD137 of the proteins PRO885 (scDb containing 38-02-A04 CDR graft), PRO951 (scDb containing 38-27-C05 CDR graft) and the competitor molecules Urelumab (BMS) and Utomilumab Pfizer) were compared in a SPR epitope binning assay using a MASS-1 device (Sierra Sensors). A sandwich setup was chosen to examine if the molecules block one another's binding to CD137. Therefore, PRO885 and PRO951 were immobilized on high capacity amine sensor chips (HCA, Sierra Sensors). Then, 90 nM of the antigen CD137 was captured on the scDbs, followed immediately by an injection of 22.5 nM of the second antibody (PRO885, PRO951, Urelumab and Utolimumab).

The capture levels of CD137 on each protein and the second binder response levels were determined (response units, RU). By calculating the theoretical maximum response (Rmax), which depends on the molecular weights of the involved proteins and the capture levels, the relative binding level (%) of the proteins on the captured antigen were determined. If the molecules bind overlapping or similar epitopes on CD137, no binding of the antibody injected over the captured CD137 should be observed. Consequently, when binding of the antibody is observed the two antibody pairs bind non-overlapping epitopes.

### Results:

When PRO885 was immobilized on a sensor chip, all 3 antibodies PRO951, Urelumab and Utomilumab showed binding to CD137 captured by PRO885. As expected, no binding was observed for PRO885 that was used a control (Figure 10 and Figure 11). When PRO951 was immobilized on the sensor chip, Urelumab and PRO885 showed binding while Utomilumab and PRO951 that was used as control did not show any significant binding (Figure 10 and Figure 12). These results show that PRO885 derived from clone 38-02-A04 binds to different epitopes on CD137 than Urelumab, Utolimumab and PRO951 derived from 38-27-C05. In contrast, RO951 bind to an epitope that is overlapping with Utolimumab but not with Urelumab and PRO885.

### Example 6: Assessment of the CD137 agonistic effect of anti-PD-LlxCD137 molecules by using a cell-based assay of transgenic NFkB Jurkat reporter cell line expressing CD137.

### Introduction

In this assay, the activation of CD137 signaling in Jurkat cells was assessed. The activitiy of CD137 signaling is reported by measurement of Luciferase expression which is driven by CD137 induced NF-kB activation in a Jurkat reporter cell line. The expression of Luciferase directly correlates with the activity of CD137. Moreover, clustering of CD137 which is required for activation of the signal pathway is facilitated via then formation of an immunological synapse between the Jurkat cells and a PD-L1 expressing cell line. Therefore, PD-L1 expression is needed for clustering and activation of CD137 on the reporter cell line.

### Methods

PD-L1 expressing CHO (clone A2) and HCC827 cells unstimulated or stimulated for 24h with 10 ng/ml IFNy to increase PD-L1 expression were seeded at 25'000 cells per well on 96-well culture plates. As a negative control, CHO WT cells without PD-L1 expression were seeded at the same cell density. Then, serial dilutions of the anti-PD-L1xCD137 molecules as well as the competitor Urelumab were prepared and added to the cells. Next, Jurkat reporter cells were prepared in assay medium containing HSA at 25 mg/ml or without and added at a cell density of 40'000 cells per well. Luciferase expression was detected by addition of Luciferase reagent and was read by a luminescence reader 6 or 24h after addition of Jurkat cells. Data were analyzed by normalization the relative luminescence units (RLU) of the test samples to the RLU measured for Urelumab yielding values of the relative activation of CD137 signaling.

### Results

### I. Test of PRO885 and PRO951 using CHO-PD-L1 cells:

As shown in Figure 13, PRO885 and PRO951 activated CD137 signaling more efficient in the presence of PD-L1 expressing CHO cells than Urelumab. PRO885 showed the best potency and highest signal of activation (PRO885, EC₅₀ = 11.72 ng/ml, PRO951: EC₅₀ = 33.68 ng/ml; Urelumab: EC₅₀ = 79.11 ng/ml, Table 9). In the absence of PD-L1, neither **PRO885** nor **PRO951** could activate CD137 in reporter cells while Urelumab showed activation of CD137 signaling independently of PD-L1.

**TABLE 9 EC50 values for anti-PD-L1xCD137 molecules using CHO-PD-L1 cells.**

| | Urelumab | PRO885 | PRO951 |
|---|---|---|---|
| Bottom | -0.4628 | -8.1 | -4.066 |
| Top | 101.5 | 491.2 | 411.4 |
| EC₅₀ in ng/ml | 79.11 | 11.72 | 33.68 |
| R square | 0.995 | 0.9922 | 0.9899 |

### II. Test of STR-grafted scDbs using CHO-PD-L1 cells:

As shown in Figure 14 and Table 10, the anti-PD-L1xCD137 scDb molecules stimulated CD137 signaling more efficiently than Urelumab. In contrast to Urelumab, the stimulatory effect was only seen for the scDb when PD-L1 expressing target cells were present. All scDbs showed identical potency to stimulate Nf-kB reporter gene activation in presence of CHO cells expressing PD_L1 at high levels. Next, the same molecules were tested in the presence of cells expressing a lower amount of PD-L1.

### III. Test of STR-grafted scDb using HCC827 cells without IFNy:

As shown in Figure 15 and Table 11, the anti-PD-L1xCD137 scDb molecules stimulated CD137 signaling more efficiently than Urelumab. The scDbs with affinity improved CD137 domain (STR grafts of 38-02-A04, PRO1120 and PRO 1124) showed an improved potency in CD137 activation when compared to the CDR grafted CD137 domain (for instance, PRO885, EC₅₀ = 13.02 ng/ml, PRO1124: EC₅₀ = 5.62 ng/ml, Table 11). Of note, increased affinity to PD-L1 as it was found for the STR graft of the PD-L1 domain (Pro 1126) also resulted in increased potency when compared to the parental molecule PRO885 (PRO885, EC₅₀ = 13.02 ng/ml, PRO1126: EC₅₀ = 6.97 ng/ml, Table 11). At high concentrations, the STR grafted scDb showed a tendency of decreasing signal of activation. This was more pronounced for molecules having a STR grafted CD137 domain (PRO1120 and PRO1126). Interestingly, when the STR graft of the PD-L1 domain was combined with the CDR graft of CD137 the signal decrease at high concentrations was not observed (compare PRO885 and PRO1124 in Figure 15). Thus, potency increased slightly with increasing affinity to CD137 and PDL1. A signal decrease at high concentrations (bell-shaped curve) was more pronounced with increasing affinity to CD 137, while increased affinity to PDL1 did not contribute to this effect. Thus, rather the ratio between affinity to CD137 and PD-L1, than the absolute affinities of each domain seem critical to extend the concentration window of maximal activity.

| | | **Affinity to human CD137: SPR data** | | | | **Affinity to mouse CD137: SPR data** | | | | | **Affinity to human PD-L1: SPR data** | | | | | |
|---|---|---|---|---|---|---|---|---|---|---|---|---|---|---|---|---|
| **PRO Number** | **Protein description** | **kₐ [M⁻¹s⁻¹]** | **k_{d}[s⁻¹]** | **K_{D} [M]** | **Binding level normalized to theoretical Rmax (%)** | | **kₐ [M⁻¹s⁻¹]** | **k_{d}[s⁻¹]** | **K_{D} [M]** | **Binding level normalized to theoretical Rmax (%)** | | **kₐ [M⁻¹s⁻¹]** | **k_{d} [s⁻¹]** | **Kₒ [M]** | **Binding level normalized to theoretical Rmax (%)** | |
| PRO1118 | 38-02-A04 sc01 scDb-1/33_02_G02 sc01 scDb-o | 2.75E+05 | 723E-04 | 2.63E-09 | 79.8 | | 8.29E+04 | 2.27E-04 | 2.73E-09 | 11.5 | | 1.34E+06 | < 1.00E-05 | < 7.46E-12 | 79.2 | |
| | | | | | | | | | | | | | | | | |
| PRO1119 | 38-02-A04 sc05 IF scDb-i/33_02_G02 sC01 scDb-o | 3.81E+05 | 2.76E-04 | 7.24E-10 | 79.1 | | 1.40E+05 | 2.48E-02 | 1.77E-07 | 81.0 | | 1.50E+06 | < 1.00E-05 | < 6.67E-12 | 77.5 | |
| | | | | | | | | | | | | | | | | |
| PRO1120 | 38-02-A04 sc06 Full scDb-i/33_02_G02 sc01 scDb-o | 5.78E+05 | < 1.00E-05 | < 1.73E-11 | 74.7 | | 2.12E+05 | 3.25E-03 | 1.54E-08 | 87.5 | | 1.27E+06 | < 1.00E-05 | < 7.87E-12 | 76.8 | |

### IV. Test of STR-grafted scDb using HCC827 cells with IPNγ:

Stimulation of HCC827 with IFNy led to increased signal of activation without changing the potency of the scDb molecules. Of note, the drop of signal at high concentration of the tested scDb was less obvious in this setting suggesting a correlation with PD-L1 expresion (Figure 16 and Table 12).

### V. Test of long half-life molecules using CHO-PD-L1 cells:

As shown in Figures 17 and 18 and Tables 13 and 14, tested long half-life anti-PD-L1xCD137 molecules stimulated CD137 signaling to the same extend as Urelumab did. There was a difference when the Morrision formats (PRO1060, PRO 1062) were compared to the scDb-scFv formats (PRO1057, PRO1058). While the Morrison formats showed a higher potency, the maximum signal of activation was substantially increased when the scDb-scFv were tested. Of note, after 24h of incubation PRO1057 showed a remarkable high signal of activation. All tested long half-life molecules activated CD137 signaling only in the presence of PD-L1 expressing cells. Interestingly, despite similar affinities to both targets, PRO1057 showed a much higher maximal signal than PRO1058. And further, the monovalent scDb-scFv PRO1057 showed stronger activation than the respective bivalent Morrison format PRO1060.

### VI. Test of long half-life molecules using HCC827 cells without and with IFNy

As shown in Figure 19 and Table 15, tested long half-life anti-PD-L1xCD137 molecules stimulated CD137 signaling to the same extend as Urelumab in the presence of cells expressing lower amounts of PD-L1. The maximum activation of tested molecules was further increased when the target cells were stimulated by IFNy suggesting a direct correlation of CD 137 activation with the levels of PD-L1 expression on the target cells. As already stated above, PRO1057 showed a higher level of reporter gene activation when compared to the Morrison formats.

**TABLE 10 EC₅₀ values for anti-PD-L1xCD137 molecules using CHO-PD-L1 cells.**

| | Urelumab | PRO885 | PRO1118 | PRO1119 |
|---|---|---|---|---|
| Bottom | -0.5169 | -6.408 | -2.387 | 2.039 |
| Top | 99.99 | 249.1 | 245.3 | 238.9 |
| EC50 in ng/ml | 79.47 | 5.135 | 4.596 | 4.22 |
| R square | 0.9637 | 0.9708 | 0.9761 | 0.9726 |

| | Urelumab | PRO885 | PRO1120 | PRO1123 |
|---|---|---|---|---|
| Bottom | -0.6273 | 0.5392 | 6.497 | -1.816 |
| Top | 100 | 222.1 | 201.1 | 219.2 |
| EC50 in ng/ml | 104.2 | 4.856 | 4.689 | 5.753 |
| R square | 0.9677 | 0.9769 | 0.963 | 0.9568 |

| | Urelumab | PRO885 | PRO1124 | PRO1126 |
|---|---|---|---|---|
| Bottom | -0.499 | -11.51 | 2.552 | -2.922 |
| Top | 100 | 253.8 | 228.6 | 242.1 |
| EC50 in ng/ml | 86.51 | 6.299 | 3.681 | 5.997 |
| R square | 0.979 | 0.9714 | 0.9561 | 0.9467 |

**TABLE 11 EC₅₀ values for anti-PD-L1xCD137 molecules using HCC827 cells without IFNγ.**

| | Urelumab | PRO885 | PRO1118 | PRO1119 |
|---|---|---|---|---|
| Bottom | -2.035 | -0.5477 | -0.3666 | 0.1789 |
| Top | 100 | 96.45 | 81.98 | 74.91 |
| EC50 in ng/ml | 212.5 | 11.5 | 8.559 | 5.045 |
| R square | 0.9815 | 0.9852 | 0.995 | 0.9725 |

| | Urelumab | PRO885 | PRO1120 | PRO1123 |
|---|---|---|---|---|
| Bottom | -1.986 | -0.2298 | 0.0807 | -0.8367 |
| Top | 99.96 | 111.1 | 126.5 | 123.2 |
| EC50 in ng/ml | 109.9 | 13.06 | 6.685 | 16.12 |
| R square | 0.9895 | 0.9759 | 0.9609 | 0.96 |

| | Urelumab | PRO885 | PRO1124 | PRO1126 |
|---|---|---|---|---|
| Bottom | -1.384 | -0.3237 | -1.084 | -8.28 |
| Top | 99.98 | 118.7 | 113.2 | 135.1 |
| EC50 in ng/ml | 111.7 | 13.02 | 5.616 | 6.966 |
| R square | 0.9941 | 0.9816 | 0.9875 | 0.9577 |

**TABLE 12 EC₅₀ values for STR grafted scDb using HCC827 cells stimulated with IFNγ.**

| | Urelumab | PRO885 | PRO1118 | PRO1119 |
|---|---|---|---|---|
| Bottom | -1.208 | -1.446 | -1.564 | 0.1399 |
| Top | 100 | 167.4 | 146.5 | 139.9 |
| EC50 in ng/ml | 114.2 | 9.266 | 7.965 | 4.855 |
| R square | 0.9939 | 0.9803 | 0.996 | 0.9767 |

| | Urelumab | PRO885 | PRO1120 | RO1123 |
|---|---|---|---|---|
| Bottom | -1.009 | -2.023 | 0.1813 | -3.584 |
| Top | 99.98 | 134.2 | 117.3 | 154.4 |
| EC50 in ng/ml | 144.8 | 8.883 | 5.15 | 11.87 |
| R square | 0.9811 | 0.9795 | 0.9554 | 0.9883 |

| | Urelumab | PRO885 | PRO1124 | PRO1126 |
|---|---|---|---|---|
| Bottom | -1.28 | -0.7258 | 1.572 | -1.561 |
| Top | 100 | 165.8 | 188.5 | 207.7 |
| EC50 in ng/ml | 108.2 | 9.229 | 4.833 | 5.48 |
| R square | 0.9976 | 0.9764 | 0.9906 | 0.9825 |

**TABLE 13 EC₅₀ values for long half-life molecules using PD-L1 expressing CHO cells (6h).**

| | Urelumab | PRO885 | PRO1060 | PRO1062 |
|---|---|---|---|---|
| Bottom | -0.09762 | 6.019 | -5.304 | -1.947 |
| Top | 99.96 | 337.5 | 220.4 | 199.1 |
| EC50 in ng/ml | 56.45 | 7.843 | 56.42 | 52.16 |
| R square | 0.9948 | 0.9474 | 0.9874 | 0.993 |

| | Urelumab + HSA | PRO885 + HSA | PRO1057 + HSA | PRO1058 + HSA |
|---|---|---|---|---|
| Bottom | -0.8837 | 6.69 | -7.721 | -0.5282 |
| Top | 106.3 | 492.8 | 462.9 | 40.06 |
| EC50 in ng/ml | 77.72 | 7.916 | 111.5 | 70.93 |
| R square | 0.9957 | 0.9457 | 0.9943 | 0.6132 |

**TABLE 14 EC₅₀ values for long half-life molecules using PD-L1 expressing CHO cells (24h).**

| | Urelumab | PRO885 | PRO1060 | PRO1062 |
|---|---|---|---|---|
| Bottom | 0.2207 | -11.83 | -5.151 | -3.914 |
| Top | 88.59 | 196.1 | 141.1 | 130.5 |
| EC50 in ng/ml | 46.63 | 13.75 | 80.85 | 86.87 |
| R square | 0.9739 | 0.9869 | 0.991 | 0.9737 |

| | Urelumab + HSA | PRO885 + HSA | PRO1057 + HSA | PRO1058 + HSA |
|---|---|---|---|---|
| Bottom | -0.3454 | -5.805 | -7.71 | -0.4289 |
| Top | 90.45 | 258.5 | 744.9 | 20.53 |
| EC50 in ng/ml | 78.17 | 14.85 | 792.4 | 121.4 |
| R square | 0.9893 | 0.9812 | 0.9955 | 0.6294 |

**TABLE 15 EC₅₀ values for long half-life molecules using HCC827 cells stimulated with IFNγ.**

| | HCC827 without IFNy stimulation | | | |
|---|---|---|---|---|
| | Urelumab | PRO885 | PRO1057 (+HSA) | PRO1060 |
| Bottom | -0.7119 | -5.806 | 0.08644 | -0.6993 |
| Top | 85.16 | 136.6 | 63.18 | 43.56 |
| EC50 in ng/ml | 47.49 | 9.518 | 76.61 | 13.57 |
| R square | 0.975 | 0.9838 | 0.9478 | 0.9255 |

| | HCC827 with IFNy stimulation | | | |
|---|---|---|---|---|
| | Urelumab | PRO885 | PRO1057 (+HSA) | PRO1060 |
| Bottom | -2.649 | -3.386 | -3.323 | -1.177 |
| Top | 91.62 | 140.2 | 136.6 | 83.61 |
| EC50 in ng/ml | 52.77 | 7.515 | 115 | 17.51 |
| R square | 0.9743 | 0.9901 | 0.9911 | 0.972 |

### Example 7: Assessment of T cell stimulatory effect of concomitant PD-L1 blockade and CD137 stimulation in a cell-based assay using human PBMC and transgenic CHO cells expressing PD-L1.

### Method

CHO-A2 cells expressing PD-L1 were seeded at three different densities, ranging from 50000 to 200000 cells per well on 96-well culture plates pre-coated with an anti-human CD3 antibody. The plates were incubated overnight at 37 °C, 5% CO₂. On the next day, peripheral blood mononuclear cells (PBMC) were isolated from fresh human whole blood by means of density gradient centrifugation. 100000 PBMCs per well were added to the 96-well plate, followed by the addition of the anti-PD-L1xCD137 scDb PRO885 at concentrations of 500, 50 and 5 ng/ml. After 76 hours of incubation, cell supernatants were harvested. Human interleukin-2 (IL-2) levels in the culture supernatants were quantified using the IL-2 human ELISA MAX assay from Biolegend, according to kit instructions. IL-2 concentrations were interpolated from a IL-2 standard curve, back-calculated and plotted against PRO885 concentrations for calculation of EC50 values.

### Results

**TABLE 17 Induction of IL-2 secretion by T cells upon treatment with PRO885.**

| ug/ml of anti-CD3 antibody | 2 | | | 1 | | |
|---|---|---|---|---|---|---|
| CHO-A1 cells/well | 50000 | 100000 | 200000 | 50000 | 100000 | 200000 |
| EC50 (ng/ml) | 52.01 | 41.85 | 30.05 | 12.85 | 86.22 | 80.62 |

As shown in Figure 20, IL-2 was secreted by T cells following concomitant blockade of PD-1/PD-L1 interaction and stimulation of CD137 by the addition of the bispecific molecule PRO885. Secreted IL-2 levels increased with augmenting anti-CD3 antibody and CHO-A2 cell-densities, and increasing PRO885 concentrations. In the absence of anti-CD3 antibodies, IL-2 levels were comparable to basal IL-2 secretion. PRO885 only activated T-cells co-stimulated by an anti-CD3 antibody. This finding demonstrates that PRO885 only stimulates activated T-cells and suggests that *in vivo* PRO885 would specifically stimulate tumor specific T-cells.

**TABLE 16: NF-kB reporter gene activation by PD-L1 x CD137 multispecific constructs.**

| | | | | | **Activation of NF-kB reporter gene CHO-PD-L1** | | | | **Activation of NF-kB reporter gene HCC827 -IFNg** | | | **Activation of NF-kB reporter gene HCC827 +IFNg** | | | |
|---|---|---|---|---|---|---|---|---|---|---|---|---|---|---|---|
| PRO ID | Clone ID PD-L1 | Clone ID CD137 | Clone ID SA | Format | IC₅₀ (ng/ml) | rel. IC₅₀^{&} | max. activation (%) | HSA | IC₅₀ (ng/ml) | rel. IC₅₀^{&} | max. activation (%) | IC₅₀ (ng/ml) | rel. IC₅₀^{&} | max. activation (%) | HSA |
| PRO885 | 33-03-G02 CDR | 38-02-A04 CDR | NA | scDb | 11.72 | 6.75 | 499.42 | no | 13.06 | 8.42 | 111.10 | 8.88 | 16.30 | 134.20 | no |
| PRO951 | 33-03-G02 CDR | 38-27-C05 CDR | NA | scDb | 33.68 | 2.35 | 431.70 | no | ND | ND | ND | ND | ND | ND | ND |
| PRO1123 | 33-03-G02 CDR | 38-02-A04 IF | NA | scDb | 5.75 | 18.11 | 219.20 | no | 16.12 | 6.82 | 123.20 | 11.87 | 12.20 | 154.40 | no |
| PRO1124 | 33-03-G02 CDR | 38-02-A04 STR | NA | scDb | 3.68 | 23.50 | 228.60 | no | 5.62 | 19.89 | 113.20 | 4.83 | 22.39 | 188.50 | no |
| PRO1126 | 33-03-G02 STR | 38-02-A04 CDR | NA | scDb | 6.00 | 14.43 | 242.10 | no | 6.97 | 16.04 | 135.10 | 5.48 | 19.74 | 207.70 | no |
| | | | | | | | | | | | | | | | |
| PRO963 | 33-03-G02 CDR | 38-02-A04 CDR | 19-01-H04 STR | Triabody | ND | ND | 368.67 | yes, 24h | ND | ND | ND | ND | ND | ND | ND |
| PRO1057 | 33-03-G02 CDR | 38-02-A04 CDR | 23-13-A01 STR | Triabody | 792.40 | 0.10 | 662.79 | yes, 24h | 76.61 | 0.62 | 68.99 | 115.00 | 0.46 | 135.34 | yes, 24h |
| PRO1058 | 33-03-G02 CDR | 38-27-C05 CDR | 23-13-A01 STR | Triabody | 121.40 | 0.64 | 36.23 | yes, 24h | ND | ND | ND | ND | ND | ND | ND |
| | | | | | | | | | | | | | | | |
| PRO1059 | 33-03-G02 CDR | 38-02-A04 CDR | NA | Morrison-L | 289.10 | 0.09 | 189.09 | no, 24h | ND | ND | ND | ND | ND | ND | ND |
| PRO1060 | 33-03-G02 CDR | 38-02-A04 CDR | NA | Morrison-H | 80.85 | 0.58 | 144.13 | no, 24h | 13.57 | 3.50 | 54.94 | 17.51 | 3.01 | 97.04 | no, 24h |
| PRO1061 | 33-03-G02 CDR | 38-27-C05 CDR | NA | Morrison-L | ND | ND | ND | ND | ND | ND | ND | ND | ND | ND | ND |
| PRO1062 | 33-03-G02 CDR | 38-27-C05 CDR | NA | Morrison-H | 86.87 | 0.54 | 133.52 | no, 24h | ND | ND | ND | ND | ND | ND | ND |

| | | | | | | | | | | | | | | | |
|---|---|---|---|---|---|---|---|---|---|---|---|---|---|---|---|
| NA: not applicable ND: not determined ^{&}: IC_{50,Urelumab} (ng/ml)/IC_{50,test molecule} (ng/ml) | | | | | | | | | | | | | | | |

### Example 8: Assessment of the stimulatory effect of concomitant PD-L1 blockade and CD137 stimulation in a cell-based assay using human PBMC stimulated with superantigen SEA.

In this experiment, the synergistic effect of PD-1/ PD-L1 inhibition and CD137 agonism was assessed. The assay used peripheral blood mononuclear cells (PBMC) that were stimulated with the superantigen Staphylococcal Enterotoxin A (SEA) in order to induce expression of PD-L1 on antigen-presenting cells (APC) and T cells respectively and CD137 on T-cells. By applying anti-PD-L1xCD137 molecules two T-cell regulatory signaling pathways were targeted concomitantly: inhibition of the inhibitory PD-1/PD-L1 pathway as well as activation of the CD137 pathway via formation of an immunological synapse mediated by the bispecific anti-PD-L1xCD137 molecule (PRO885). The activation of T-cells was assessed by the secretion of Interleukin-2 (IL-2) and compared to the effect mediated by PD-L1 inhibition mediated by the benchmarking reference antibody Avelumab. In addition, the anti-PD-Ll scFv, PRO997, was tested and compared to Avelumab in the same experimental setup.

### Method

Peripheral blood mononuclear cells (PBMC) were isolated from fresh human whole blood by means of density gradient centrifugation. Then, PBMC were depleted for NK cells using anti-CD56 antibody and the MACS cell separation kit (Miltenyi Biotec). Next, 100'000 PBMCs per well were added to the 96-well plate, followed by the addition of serial dilutions of PRO885, PRO997 and Avelumab in assay buffer containing SEA at a concentration of 10 ng/ml. After 96 hours of incubation at 37°C and 5% CO₂, cell supernatants were harvested and human Interleukin-2 (IL-2) levels in the culture supernatants were quantified using the IL-2 human ELISA MAX assay from BioLegend according to kit instructions. IL-2 concentrations were interpolated from a IL-2 standard curve, back-calculated and plotted against Avelumab and PRO885 concentrations for calculation of EC50 values.

### Results

**TABLE 18: EC50 values for PRO885 and PRO997 in PBMC assay using SEA stimulation.**

| | Avelumab | PRO885 |
|---|---|---|
| Bottom | 2479 | 7463 |
| Top | 8687 | 20663 |
| EC50 in ng/ml | 69.89 | 39.92 |
| R square | 0.8589 | 0.9052 |

| | Avelumab | PRO997 |
|---|---|---|
| Bottom | 2117 | 3226 |
| Top | 8588 | 9480 |
| EC50 in ng/ml | 90.18 | 40.86 |
| R square | 0.8783 | 0.867 |

As shown in Figure 21, IL-2 was secreted by T-cells following concomitant blockade of PD-1/PD-L1 interaction and stimulation of CD137 by the addition of the bispecific molecule PRO885.When compared to Avelumab, PRO885 showed higher T cell activation and better potency (PRO885, EC₅₀ = 39.92 ng/ml; Avelumab, EC₅₀ = 69.89 ng/ml, Table 18). This finding demonstrates that the bispecific anti-PD-L1xCD137 scDb PRO885 is able to induce stronger T cell stimulaton than mere PD-L1 blockade by Avelumab. Moreover, the high-affinity anti-PD-Ll scFv PRO997 was found to be more potent in stimulation of T-cells than Avelumab (PRO997, EC₅₀ = 40.86 ng/ml; Avelumab, EC₅₀ = 90.18 ng/ml, Table 18).

### Example 9: Assessment of the anti-tumor efficacy of PD-L1 blockade and concomitant localized stimulation of CD137 in the human cell line-derived lung cancer xenograft model HCC827.

Anti-tumor activity of the multispecific antibody of the present invention will be compared to anti-PD-Ll and anti-CD137 therapy in human HCC827 NSCLC xenografts using the immunodeficient NOG mice strain from Taconic and allogeneic human peripheral blood mononuclear cells. Engrafted human T lymphocytes show xeno-reactivity against foreign major histocompatibility (MHC) class I and II and other antigens from mice cells. As a result, T lymphocytes cause an inflammatory infiltrate in different organs that leads to death of the animals after several weeks, a process known as xenograft-versus-host disease (xGVHD). Treatment with immunomodulatory antibodies such as anti-PD-Ll and anti-CD137 was shown to exacerbate xGVHD (Sanmamed MF et al. Nivolumab and Urelumab enhance antitumor activity of human T lymphocytes engrafted in Rag2-/-IL2Rgnull immunodeficient mice. Cancer Res 2015;75(17):3466-3478). Effects of PRO1057 (scDb-scFv) and PRO1060 (IgG-scFv with CD137 scFv fused to the C-terminus of the heavy chain of the IgG) on tumor volume will be compared to treatment with the IgG1 containing the same PD-L1 specific variable domain as the multispecific antibody of the present invention (e.g., PRO1137) and with the IgG4 with the same CD137 specific variable domain (PRO1138). To provide further evidence of localized antitumor immune response, frequency of tumor infiltrating lymphocytes such as CD8+, CD4+ and regulatory T cells will be analyzed by flow cytometry. To explore modulation of the immune system systemically following anti-CD137/anti-PD-L1 treatment the frequency of CD4+ and CD8+ T cells in liver and spleen will be analyzed by flow cytometry. Moreover, systemic IFNg levels will be analyzed using a quantitative ELISA method.

### Study set-up and treatment schedule

Female NOG mice will receive unilateral injections of 5x10⁶ HCC827 cells. Cells will be injected in a mixture of 50% cell suspension in PBS and 50% matrigel in a total injection volume of 100 µl. After injection of tumor cells into NOG mice and successful tumor engraftment (median group tumor volume of 80-100 mm³), mice will be substituted with 5x10⁶ human PBMCs by intravenous injection. On the day of randomization, four mice of each group will be reconstituted with PBMCs of donor A and another four mice with PBMCs of donor B. Treatment will start 1-2 hours after the injection of PBMCs and will be applied as follows.

| **group ID** | **compound** | **total daily dose[mg]** | **dosing days** | **route** | **no. of mice** |
|---|---|---|---|---|---|
| 1 | **Vehicle** | na | 0,3,7,10 | ip | 8 |
| 2 | **PRO1057 low dose** | 0.08 | 0,3,7,10 | ip | 8 |
| 3 | **PRO1057 medium dose** | 0.4 | 0,3,7,10 | ip | 8 |
| 4 | **PRO1057 high dose** | 2 | 0,3,7,10 | ip | 8 |
| 5 | **PRO1137** | 0.2 | 0,3,7,10 | ip | 8 |
| 6 | **PRO1138** | 0.2 | 0,3,7,10 | ip | 8 |
| 7 | **PRO1060** | 0.2 | 0,3,7,10 | ip | 8 |

Body weights and tumor volume by caliper measurement will be performed twice weekly. Animals will be terminated at defined time-points depending on the study results.

Option A: Four animals per group will be terminated at an earlier time-point compared to the rest of the group (e.g. day 21) while the remaining animals will be terminated one week later (e.g. day 28) aiming at the maximum possible observation time which is limited by the onset of xGvHD.

Option B: All animals will be terminated at the 'same' time-point (between day 21 and day 28) with sample collection and processing of the first half of each group being performed on the first day and of the second half of each group on the following day for capacity reasons.

For both options, animals reconstituted with PBMCs from the two different donors will be equally represented in the two sampling cohorts.

Tumors, spleens and livers from all animals will be collected at the end of the study and will be processed for flow cytometry where the following human markers will be analyzed: Live/Dead, CD4, CD8, CD25, FOXP3, TIM3 and Granzyme B. Serum samples will be analyzed for IFNg levels by ELISA using the DuoSet® ELISA Development System from R&D Systems according to the manufacturer's instructions.

### Example 10: Assessment of the anti-tumor efficacy of PD-L1 blockade and concomitant localized stimulation of CD137 in a syngeneic MC38 colon cancer model.

In addition, anti-tumor activity of the multispecific antibody of the present invention will be tested in a MC38 colon carcinoma model in syngeneic C57BL/6 mice with an intact immune system. This model has been used by others to show enhanced antitumor activity by combination treatment with CD137 agonists and PD-1/PD-L1 antagonists (Chen S et al. Combination of 4-1BB agonist and PD-1 antagonist promotes antitumor effector/memory CD8 T cells in a poorly immunogenic tumor model. Cancer Immunol Res 2014;3(2):149-160 and Rodriguez-Ruiz ME et al. Abscopal effects of radiotherapy are enhanced by combined immunostimulatory mAbs and are dependent on CD8 T cells and crosspriming. Cancer Res 2016;76(20):5994-6005).

Since both, the anti-CD137 domain and the anti-PD-Ll domain of of the multispecific antibody of the present invention are not cross-reactive to mouse PD-L1 an engineered human CD137 knock-in model established by CrownBio will be used. In this model, the extracellular and transmembrane domain of mouse CD137 was replaced by the respective sequence of human CD137 in the C57BL/6 mice background using the CRISPR/Cas9 system. In addition, a modified MC38 tumor cell line expressing human PD-L1 under control of a CMV promoter instead of mouse PD-L1 will be used. Effects of the multispecific antibody of the present invention on tumor volume will be compared to combination treatment with the humanized IgG1 containing the same PD-L1 specific variable domain as ND021 and with the humanized IgG4 with the same CD137 specific variable domain. To provide further evidence of localized antitumor immune response, frequency of tumor infiltrating lymphocytes such as CD8+, CD4+ and regulatory T cells will be analyzed by flow cytometry. To explore modulation of the immune system systemically following anti-CD137/anti-PD-L1 treatment, the frequency of CD4+ and CD8+ T cells in liver and spleen will be analyzed by flow cytometry and possibly immunohistochemistry. Moreover, systemic IFNg levels could be analyzed using a quantitative ELISA method. To further characterize the safety profile of the anti-CD 13 7/anti-PD-L1 combination therapy, clinical chemistry pathology parameters associated primarily with liver toxicity (observed for anti-CD137 therapy in the clinic), such as increased levels of alanine aminotransferase, glutamate dehydrogenase and aspartate aminotransferase could be assessed.

### Example 11: Exemplary multispecific antibodies of the present invention.

### General description scDb

Single chain Diabodies (scDb) are small bivalent antibody fragments composed of one chain, comprising two VH and two VL domains from two different antibodies (Holliger et al. PNAS, 1993 Jul 15;90(14):6444-8). In the Numab scDb format, variable domains are connected in VLA-VHB-VLB-VHA domain orientation by GS linkers. The linkers in these single-chain diabodies (scDbs) force correct assembly of the domains and improve stability without altering the antigen-binding activity. Short G4S linkers connecting VLA with VHB and VLB with VHA are substantially shorter than that required to allow assembly of adjacent domains and keep the domains in an open conformation allowing correct pairing of corresponding domains. VLA-VHB and VLB-VHA diabody arms are connected by a long (G4S)4 linker between VHB and VLB domains allowing dimerization in a head-to-tail orientation resulting in a compact bispecific molecule with a molecular mass of ∼50 kDa. The scDb can be expressed recombinantly in either E.coli or CHO-S host cells. See Figure 22A.

### PRO885

PRO885 is a scDb molecule comprising an outer (VLA/VHA) PD-L1-binding domain (33-03-G02 sc01) and an inner (VHB/VLB) CD137-specific domain (38-02-A04 sc01) connected by two short flanking G4S linkers and a long central (G4S)4 linker. Both domains consist of rabbit CDRs that were engrafted on a VH4-like human acceptor framework.

### PRO951

PRO951 is a scDb molecule comprising an outer (VLA/VHA) PD-L1-binding domain (33-03-G02 sc01) and an inner (VHB/VLB) CD137-specific domain (38-27-C05 sc02) connected by two short flanking G4S linkers and a long central (G4S)4 linker. Both domains consist of rabbit CDRs that were engrafted on human acceptor frameworks. PD-L1-specific CDR's were engrafted on a VH4 consensus-like human acceptor framework while CD137-specific CDR's were engrafted on a VH3 consensus-like human acceptor framework.

### PRO1123

PRO1123 is a scDb molecule comprising an outer (VLA/VHA) PD-L1-binding domain (33-03-G02 sc01) and an inner (VHB/VLB) CD137-specific domain (38-02-A04 sc05) connected by two short flanking G4S linkers and a long central (G4S)4 linker. Both domains consist of rabbit CDRs that were engrafted on a VH4 consensus-like human acceptor framework. In addition, CD137 domain contains few rabbit residues participating in formation of VL-VH interface.

### PRO1124

PRO1124 is a scDb molecule comprising an outer (VLA/VHA) PD-L1-binding domain (33-03-G02 sc01) and an inner (VHB/VLB) CD137-specific domain (38-02-A04 sc06) connected by two short flanking G4S linkers and a long central (G4S)4 linker. Both domains consist of rabbit CDRs that were engrafted on a VH4 consensus-like human acceptor framework. In addition, CD137 domain contains few rabbit residues participating in formation of VL-VH interface and potentially interacting with the antigen.

### PRO1125

PRO1125 is a scDb molecule comprising an outer (VLA/VHA) PD-L1-binding domain (33-03-G02 sc02) and an inner (VHB/VLB) CD137-specific domain (38-02-A04 sc01) connected by two short flanking G4S linkers and a long central (G4S)4 linker. Both domains consist of rabbit CDRs that were engrafted on a VH4 consensus-like human acceptor framework. In addition, PD-L1 domain contains few rabbit residues participating in formation of VL-VH interface.

### PRO1126

PRO1126 is a scDb molecule comprising an outer (VLA/VHA) PD-L1-binding domain (33-03-G02 sc03) and an inner (VHB/VLB) CD137-specific domain (38-02-A04 sc01) connected by two short flanking G4S linkers and a long central (G4S)4 linker. Both domains consist of rabbit CDRs that were engrafted on a VH4 consensus-like human acceptor framework. In addition, PD-L1 domain contains back mutated rabbit residues participating in formation of VL-VH interface and potentially interacting with the antigen.

### PRO1134

PRO1134 is a scDb molecule comprising an outer (VLA/VHA) PD-L1-binding domain (33-03-G02 sc07) and an inner (VHB/VLB) CD137-specific domain (38-02-A04 sc01) connected by two short flanking G4S linkers and a long central (G4S)4 linker. Both domains consist of rabbit CDRs that were engrafted on a VH4 consensus-like human acceptor framework. In addition, PD-L1 domain contains rabbit germline residues participating in formation of VL-VH interface and potentially interacting with the antigen.

### General description Triabody

Single chain Triabodies (scTb) is a format developed at Numab that adds a third single chain variable domain pair (VLC/VHC) connected via (G4S)2 linker to the highly stable single chain diabody (scDb) format as described above (VLA-VHB-VLB-VHA domain orientation with short G4S core linkers and long (G4S)4 central linker). Thus, scTb format consists of a tandem arrangement of a scDb with a scFv entity on a single protein chain and a molecular weight of ∼80 kDa. The scTb antibody fragment can be expressed recombinantly in mammalian cells. See Figure 22B.

### PRO963

PRO963 is a Triabody molecule consisting of an anti-PD-Ll (33-03-G02 sc01, VLA/VHA) and anti-CD 137 (38_02_A04 sc01, VHB/VLB) scDb core fused fused at the C-terminus with an anti-HSA scFv entity (19-01-H04-sc03, VLC/VHC). PD-L1 and CD137 specific domains are human VH4 consensus-like acceptor frameworks with engrafted rabbit CDR's, while the HSA domain consists of rabbit CDR's engrafted on a human VH3-based acceptor framework containing additional rabbit residues supporting preservation of parental rabbit IgG binding characteristics.

### PRO966 (PRO1052)

PRO966 is a Triabody molecule consisting of an anti-PD-Ll (33-03-G02 sc01, VLA/VHA) and anti-CD 137 (38_27_C05 sc01, VHB/VLB) scDb core fused fused at the C-terminus with an anti-HSA scFv entity (19-01-H04-sc03, VLC/VHC). PD-L1 and CD137 specific domains are human VH4 consensus-like acceptor frameworks with engrafted rabbit CDR's, while the HSA domain consists of rabbit CDR's engrafted on a human VH3-based acceptor framework containing additional rabbit residues supporting preservation of parental rabbit IgG binding characteristics.

### PRO1057

PRO1057 is a Triabody molecule consisting of an anti-PD-Ll (33-03-G02 sc01, VLA/VHA) and anti-CD137 (38_02_A04 sc01, VHB/VLB) scDb core fused fused at the C-terminus with an anti-HSA scFv entity (23-13-A01-sc03, VLC/VHC). PD-L1 and CD137 specific domains are human VH4 consensus-like acceptor frameworks with engrafted rabbit CDR's, while the HSA domain consists of rabbit CDR's engrafted on a human VH3-based acceptor framework containing additional rabbit residues supporting preservation of parental rabbit IgG binding characteristics including cross-reactivity to murine serum albumin.

### PRO1058

PRO1058 is a Triabody molecule consisting of an anti-PD-Ll (33-03-G02 sc01, VLA/VHA) and anti-CD 137 (38_27-C05 sc01, VHB/VLB) scDb core fused at the C-terminus with an anti-HSA scFv entity (23-13-A01-sc03, VLC/VHC). PD-L1 and CD137 specific domains are human VH4 consensus-like acceptor frameworks with engrafted rabbit CDR's, while the HSA domain consists of rabbit CDR's engrafted on a human VH3-based acceptor framework containing additional rabbit residues supporting preservation of parental rabbit IgG binding characteristics including cross-reactivity to murine serum albumin.

### General description IgG-scFv

IgG-scFv molecules are bispecific antibodies consisting of scFv moieties fused to a monospecific IgG (Coloma M.J., Morrison S.L. Nat. Biotechnol. 1997;15:159-163.). Either the amino or the carboxy terminus of each light or heavy chain can be appended with scFv domains of even different specificities, which leads to a diverse repertoire of IgG-scFv bispecific antibody types. The molecules produced at Numab are of the IgG(H)-scFv or IgG(L)-scFv type, two scFvs with same specificity linked to the C terminus of the full-length IgG heavy chain (HC) or light chain (LC), containing a silenced Fc-portion and (G4S)2 linker connecting IgG portion with scFv portion. The total molecular weight of both types is ∼200 kDa and the molecules can be expressed recombinantly in mammalian cells. See Figures 22C and 22D.

### PRO1059 (IgG(L)-scFv)

Silent anti-PD-Ll (33-03-G02-sc01) hIgG1 with an anti-CD137 scFv domain (38_02_A04 sc01) fused to the C-terminus of the LC. A (G4S)2 linker is connecting IgG portion with scFv portion, which itself consists of a VL domain connected via (G4S)4 linker to the corresponding VH domain.

### PRO1060 (IgG(H)-scFv)

Silent anti-PD-Ll (33-03-G02-sc01) hIgG1 with an anti-CD137 (38_02_A04 sc01) scFv domain fused to the C-terminus of the HC. A (G4S)2 linker is connecting IgG portion with scFv portion, which itself consists of a VL domain connected via (G4S)4 linker to the corresponding VH domain.

### PRO1061 (IgG(L)-scFv)

Silenced anti-PD-Ll (33-03-G02-sc01) hIgG1 with an anti-CD137 scFv domain (38_27_C05 sc01) fused to the C-terminus of the LC. A (G4S)2 linker is connecting IgG portion with scFv portion, which itself consists of a VL domain connected via (G4S)4 linker to the corresponding VH domain.

### PRO1062 (IgG(H)-scFv)

Silent anti-PD-Ll (33-03-G02-sc01) hIgG1 with an anti-CD137 (38_27_C05 sc01) scFv domain fused to the C-terminus of the HC. A (G4S)2 linker is connecting IgG portion with scFv portion, which itself consists of a VL domain connected via (G4S)4 linker to the corresponding VH domain.

## Claims

1. A multispecific antibody comprising:
a) at least one CD137 binding domain (CD137-BD); and
b) at least one PDL1 binding domain (PDL1-BD),
wherein the affinity of said CD137-BD relative to the affinity of said PDL1-BD is at least 0.2 times, at least 10 times, preferably at least 50, more preferably at least 500 times weaker (lower).

2. The multispecific antibody of claim 1, wherein said antibody is monovalent, bivalent or multivalent for CD137 specificity, preferably monovalent, and, optionally, wherein said antibody is monovalent, bivalent or multivalent for PDL1 specificity, preferably monovalent.

3. The multispecific antibody of any one of claims 1 to 2, wherein said antibody further comprises at least one human serum albumin binding domain, preferably one human serum albumin binding domain.

4. The multispecific antibody of any of the preceding claims, wherein said binding domains are capable of binding to their respective antigen or receptor simultaneously.

5. The multispecific antibody of claim of any of the preceding claims, wherein said CD137-BD is an agonist of CD137.

6. The multispecific antibody of claim 5, wherein said CD137-BD:
a) binds to human CD137 with a dissociation constant (KD) of less than 50nM, particularly less than10 nM, particularly less than 5 nM, particularly less than 1nM, particularly less than 500 pM, more particularly less than 100 pM, more particularly less than 50 pM;
b) binds to human CD137 with a K_{off} rate of 10⁻³ s⁻¹ or less, or 10⁻⁴ s⁻¹ or less, or 10⁻⁵ s⁻¹ or less as measured by SPR;
c) binds to human CD137 with a Kₒₙ rate of at least 10⁴ M⁻¹s⁻¹ or greater, at least 10⁵ M⁻¹s⁻¹ or greater, at least 10⁶ M⁻¹s⁻¹ or greater, as measured by SPR;
d) does not cross-compete with urelumab and utolimumab; and/or
e) is cross-reactive with Macaca fascicularis (Cynomolgus) CD137.

7. The multispecific antibody of claim 5 or claim 6, wherein said CD137-BD comprises
(a) a VH sequence of SEQ ID NO: 11 and a VL sequence of SEQ ID NO: 23; (b) a VH sequence of SEQ ID NO: 12 and a VL sequence of SEQ ID NO: 24; or (c) a VH sequence of SEQ ID NO: 13 and a VL sequence of SEQ ID NO: 25.

8. The multispecific antibody of claim of any of the preceding claims, wherein said PDL1-BD is a blocker of PDL1.

9. The multispecific antibody of claim 8, wherein said PDL1-BD:
a) binds to human PDL1 with a dissociation constant (KD) of less than 50nM, particularly less than 10nM, particularly less than 5nM, particularly less than 1nM, particularly less than 500pM, more particularly less than 100pM, preferably less than 10pM, more preferably 5pM;
b) binds to human PDL1 with a K_{off} rate of 10⁻³ s⁻¹ or less, or 10⁻⁴ s⁻¹ or less, or 10⁻⁵ s⁻¹ or less as measured by SPR;
c) binds to human PDL1 with a Kₒₙ rate of at least 10³ M⁻¹s⁻¹ or greater, at least 10⁴ M⁻¹s⁻¹ or greater, at least 10⁵ M⁻¹s⁻¹ or greater, at least 10⁶ M⁻¹s⁻¹ or greater as measured by SPR;
d) is cross-reactive with Macaca fascicularis (Cynomolgus) PDL1; and/or
e) is non-cross reactive to Mus musculus PDL1.

10. The multispecific antibody of claim 8 or claim 9, wherein said PDL1-BD comprises:
(a) a VH sequence of SEQ ID NO: 62 and a VL sequence of SEQ ID NO: 73; (b) a VH sequence of SEQ ID NO: 63 and a VL sequence of SEQ ID NO: 73; (c) a VH sequence of SEQ ID NO: 87 and a VL sequence of SEQ ID NO: 98; or (d) a VH sequence of SEQ ID NO: 88 and a VL sequence of SEQ ID NO: 99.

11. The multispecific antibody of any one of claim 3 to 10, wherein said HSA-BD comprises: (a) a VH sequence of SEQ ID NO: 112 and a VL sequence of SEQ ID NO: 122; or (b) a VH sequence of SEQ ID NO: 134 and a VL sequence of SEQ ID NO: 144.

12. The multispecific antibody of any of claim 1, wherein said antibody is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab, , Fab-Fv2, Morrison (IgG CH₃-scFv fusion (Morrison L) or IgG CL-scFv fusion (Morrison H)), triabody (scDb-scFv), bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH and DuoBodies.

13. A pharmaceutical composition comprising the multispecific antibody of anyone of the preceding claims and a pharmaceutically acceptable carrier.

14. The multispecific antibody of anyone of claims 1 to 12or the pharmaceutical composition of claim 13 for use as a medicament.

15. A method of producing the multispecific antibody according to anyone of claims 1 to 12 or a binding domain thereof or a fragment thereof, the method comprising the step of culturing a host cell expressing a nucleic acid encoding the multispecific antibody according to claims 1 to 12 or a binding domain thereof or a fragment thereof.
